# EUROPEAN PATENT SPECIFICATION

(11) **EP 0 907 643 B1**
(45) Date of publication and mention of the grant of the patent: **15.01.2003**
(21) Application number: 97918075.9
(22) Date of filing: 24.04.1997
(51) Int. Cl.: C07D 241/08, C07D 241/04, C07D 243/08, C07D 401/12, C07D 409/06, A61K 31/495

(54) **COMPOUNDS WITH GROWTH HORMONE RELEASING PROPERTIES**
VERBINDUNGEN MIT WACHTUMSHORMON FREISETZENDEN EIGENSCHAFTEN
COMPOSES CAPABLES DE LIBERER UNE HORMONE DE CROISSANCE

(30) Priority: 24.04.1996 DK 48996; 26.11.1996 DK 134496
(43) Date of publication of application: 14.04.1999
(73) Proprietor: NOVO NORDISK A/S, 2880 Bagsvaerd (DK)
(72) Inventor: HANSEN, Thomas, Kruse, DK-2730 Herlev (DK); PESCHKE, Bernd, DK-2760 Maaloev (DK); ANDERSEN, Knud, Erik, DK-2765 Smorum (DK)
(86) International application number: DK9700186
(87) International publication number: WO97040023

(56) References cited:
- WO-A-95/17423
- CHEM. PHARM. BULL., Volume 44, No. 4, 1996, TETSUSHI YAMASHITA et al., "Structure-Activity Relationships of Dermorphin Analogues Containing Chiral Piperazin-2-one and Piperazine Derivatives", pages 856-859.

## Description

### FIELD OF INVENTION

The present invention relates to novel compounds, compositions containing them, and their use for treating medical disorders resulting from a deficiency in growth hormone.

### BACKGROUND OF THE INVENTION

Growth hormone is a hormone which stimulates growth of all tissues capable of growing. In addition, growth hormone is known to have a number of effects on metabolic processes, e.g., stimulation of protein synthesis and free fatty acid mobilization and to cause a switch in energy metabolism from carbohydrate to fatty acid metabolism. Deficiency in growth hormone can result in a number of severe medical disorders, e.g., dwarfism.

Growth hormone is released from the pituitary. The release is under tight control of a number of hormones and neurotransmitters either directly or indirectly. Growth hormone release can be stimulated by growth hormone releasing hormone (GHRH) and inhibited by somatostatin. In both cases the hormones are released from the hypothalamus but their action is mediated primarily via specific receptors located in the pituitary. Other compounds which stimulate the release of growth hormone from the pituitary have also been described. For example arginine, L-3,4-dihydroxyphenylalanine (L-Dopa), glucagon, vasopressin, PACAP (pituitary adenylyl cyclase activating peptide), muscarinic receptor agonists and a synthethic hexapeptide, GHRP (growth hormone releasing peptide) release endogenous growth hormone either by a direct effect on the pituitary or by affecting the release of GHRH and/or somatostatin from the hypothalamus.

In disorders or conditions where increased levels of growth hormone is desired, the protein nature of growth hormone makes anything but parenteral administration non-viable. Furthermore, other directly acting natural secretagogues, e.g., GHRH and PACAP. are longer polypeptides for which reason oral administration of them is not viable.

The use of certain compounds for increasing the levels of growth hormone in mammals has previously been proposed, e.g. in EP 18 072, EP 83 864, WO 89/07110, WO 89/01711, WO 89/10933, WO 88/9780, WO 83/02272, WO 91/18016, WO 92/01711, WO 93/04081, WO 95/17422, WO 95/17423 and WO 95/14666.

The composition of growth hormone releasing compounds is important for their growth hormone releasing potency as well as their bioavailability. It is therefore an object of the present invention to provide novel compounds with growth hormone releasing properties which have improved properties relative to known compounds of this type.

### SUMMARY OF THE INVENTION

In accordance with the present invention there is provided compounds which act directly on the pituitary cells under normal experimental conditions in vitro to release growth hormone therefrom.

These growth hormone releasing compounds can be utilized in vitro as unique research tools for understanding, inter alia, how growth hormone secretion is regulated at the pituitary level.

Moreover, the growth hormone releasing compounds of the present invention can also be administered in vivo to increase growth hormone release.

Accordingly, the present invention relates to a compound of general formula I 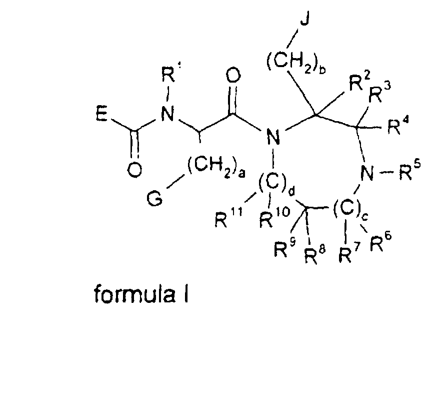 wherein
R¹ is hydrogen, or C₁₋₆-alkyl optionally substituted with aryl;
a and b are independently 1 or 2;
c and d are independently 0, 1, or 2;
c + d is 0, 1, or 2;
R², R³, R⁴, R⁶, R⁷, R⁸, R⁹, R¹⁰, and R¹¹ are independently hydrogen, aryl optionally substituted with halogen, amino, C₃₋₆-cycloalkyl, hydroxy, aryl, -COOR²², or -CONR²³R²⁴, or C₁₋₆-alkyl optionally substituted with halogen, amino C₃₋₆-cycloalkyl, hydroxy, aryl, -COOR²², or -CONR²³R²⁴;
R³ and R⁴ can be taken together to form =O or =S;
R⁸ and R⁹ can be taken together to form =O or =S;
R⁵ is hydrogen, 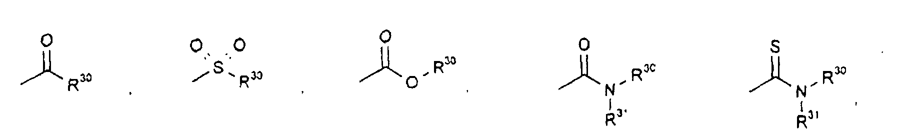 or C₁₋₆-alkyl optionally substituted with aryl, hydroxy, C₃₋₆-cycloalkyl, amino, -COOR²⁵, -CONR²⁶R²⁷, -NR'R", 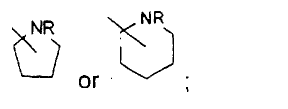 R, R' and R" are independently hydrogen or C₁₋₆-alkyl;
R³⁰ and R³¹ are independently C₁₋₆-alkyl optionally substituted with aryl, hydroxy, C₃₋₆-cycloalkyl, or amino;
R²², R²³, R²⁴, R²⁵, R²⁶, and R²⁷ are independently hydrogen or C₁₋₆-alkyl;
when R³ and R⁴ are taken together to form =O or =S, E is 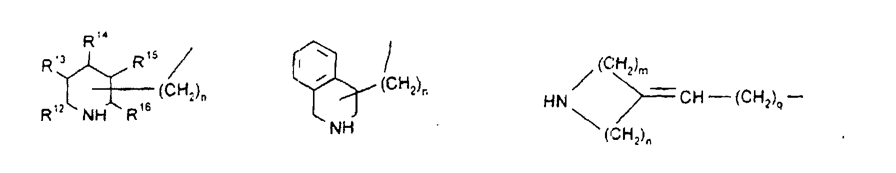 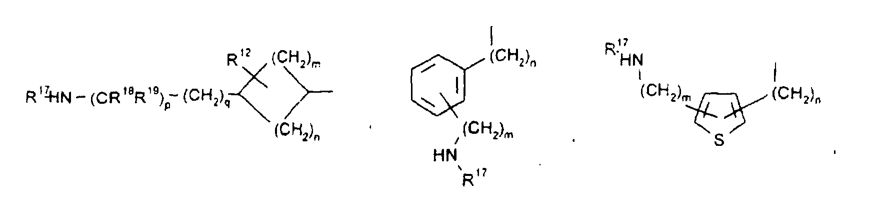 or

R¹⁷·NH(CR¹⁸R¹⁹)ₚ(CH₂)ₘ-M―(CHR²⁰)ₒ―(CH₂)ₙ― ,

when R³ or R⁴ is hydrogen, aryl optionally substituted with halogen, amino, C₃₋₆-cycloalkyl, hydroxy, aryl, -COOR²², or -CONR²³R²⁴, or C₁₋₆-alkyl, optionally substituted with halogen, amino, C₃₋₆-cycloalkyl, hydroxy, aryl, -COOR²², or -CONR²³R²⁴, E is 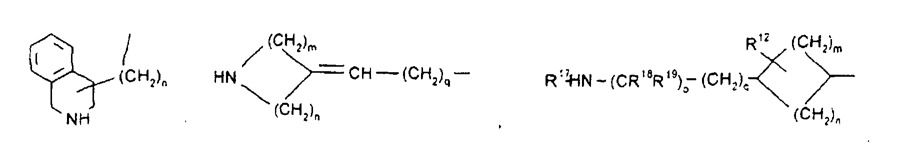 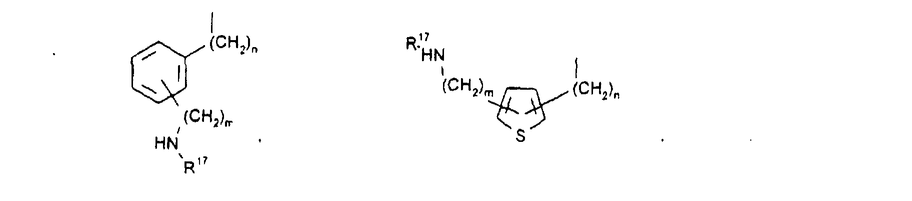 or

R¹⁷ NH―(CR¹⁸R¹⁹)ₚ―(CH₂)ₘ-Q―(CHR²⁰)ₒ―(CH₂)ₙ― ;

R¹², R¹³, R¹⁴, R¹⁵, R¹⁶, R¹⁷, R¹⁸, R¹⁹, and R²⁰ are independently hydrogen or C₁₋₆-alkyl optionally substituted with halogen, amino, hydroxyl or aryl;
any two of R¹⁷, R¹⁸, and R¹⁹ can independently be joined together to form an C₁₋₆-alkylene bridge;
n, m and q are independently 0, 1, 2, or 3;
o and p are independently 0 or 1;
M is -CH=CR²¹-, -O-, -S-, or a valence bond ;
Q is -CH=CR²¹-, -O-, or -S-;
R²¹ is hydrogen or C₁₋₆-alkyl optionally substituted with halogen, amino, hydroxyl or aryl;
G is hydrogen, 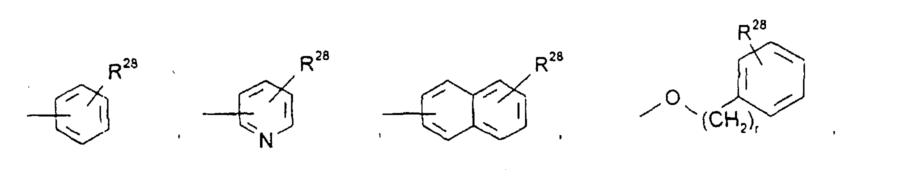 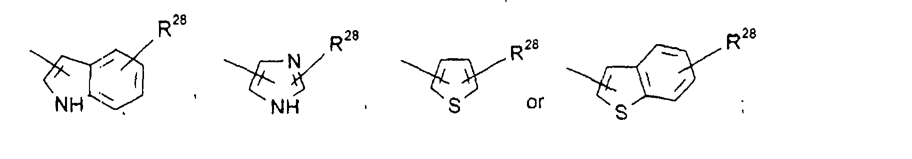 wherein R²⁸ is hydrogen, halogen, C₁₋₆alkyl, C₁₋₆-alkoxy or aryl;
r is 0, 1, or 2;
J is hydrogen, 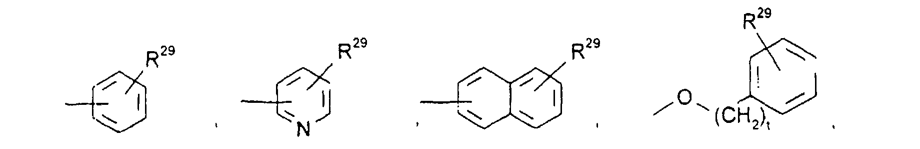 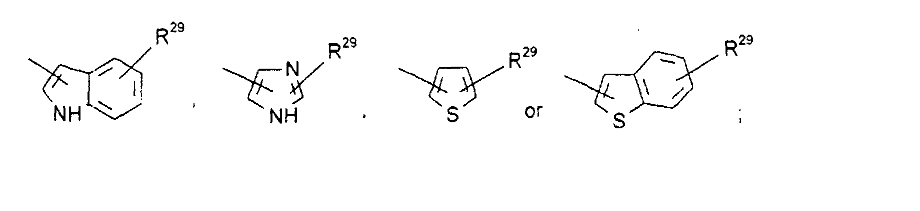 wherein R²⁹ is hydrogen, halogen, C₁₋₆-alkyl, C₁₋₆-alkoxy or aryl;
t is 0, 1, or 2;
or a pharmaceutically acceptable salt thereof.

The compounds of formula I comprise any optical isomers thereof, in the form of separated, pure or partially purified optical isomers or racemic mixtures thereof.

The compounds of formula II-V are independently preferred embodiments of the compound of formula I, thus any reference in the present description to formula I also means reference to formula II-V.

In one embodiment of the compound of the above formula I R¹ is preferably C₁₋₆-alkyl, more preferred C₁₋₄-alkyl, such as methyl.

In a second embodiment of the compound of the above formula I a is preferably 1.

In a further embodiment of the compound of the above formula I b is preferably 1.

In a still further embodiment of the compound of the above formula I c is preferably 0 or 1.

In a further embodiment of the compound of the above formula I d is preferably 0 or 1.

In a still further embodiment of the compound of the above formula I c+d is preferably 1.

In a further embodiment of the compound of the above formula I R² is preferably hydrogen or C₁₋₄-alkyl, more preferred hydrogen.

In a still further embodiment of the compound of the above formula I R³ and R⁴ are preferably taken together to form =O or =S, more preferred =O.

In another embodiment of the compound of the above formula I R³ and R⁴ are independently of each other hydrogen.

In a further embodiment of the compound of the above formula I R⁵ is preferably hydrogen, C₁₋₆-alkylsulfonyl, such as methylsulfonyl, or C₁₋₆-alkyl, more preferred C₁₋₄-alkyl, such as methyl.

In a still further embodiment of the compound of the above formula I R⁶, R⁷, R⁸ and R⁹ are independently of each other preferably hydrogen or C₁₋₆alkyl, more preferred C₁₋₄-alkyl, such as methyl.

In a further embodiment of the compound of the above formula I R¹⁰ and R¹¹ are independently of each other preferably hydrogen or C₁₋₄-alkyl, more preferred hydrogen.

In a still further embodiment of the compound of the above formula I E is preferably

R¹⁷ NH―(CR¹⁸R¹⁹)ₚ―(CH₂)ₘ-M―(CHR²⁰)ₒ―(CH₂)ₙ― ,

wherein R¹⁷ is hydrogen or C₁₋₄ alkyl, preferably methyl,
R¹⁸ is hydrogen or C₁₋₄ alkyl, preferably methyl, and
R¹⁹ is hydrogen or C₁₋₄ alkyl, preferably methyl, or
R¹⁸ and R¹⁹ are joined together to form a C₁₋₆-alkylene bridge, preferably a C₁₋₄-alkylene bridge, such as a trimethylene-bridge, and
n is 0 or 1, preferably 0,
p is 1, m is 1 and M is -CH=CR²¹, wherein R²¹ is hydrogen or C₁₋₄ alkyl, preferably methyl,
more preferred E is -CH=CH-CH₂-C(CH₃)₂NH₂, -CH=C(CH₃)-CH₂-C(CH₃)₂NH₂, -CH=CH-CH₂-C(CH₃)₂-NH(CH₃) or

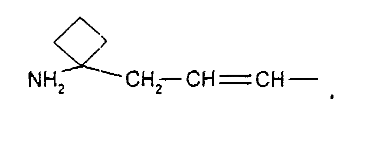

In a further embodiment of the compound of the above formula I G is preferably 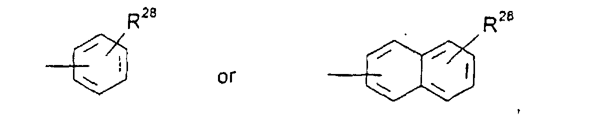 wherein R²⁸ is hydrogen or aryl, preferably phenyl,
more preferred G is 2-naphthyl or biphenyl-4-yl.

In a still further embodiment of the compound of the above formula I J is preferably 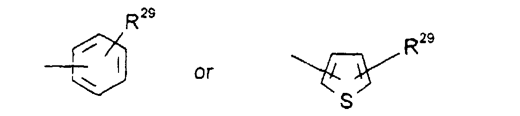 wherein R²⁹ is hydrogen or C₁₋₆-alkyl, preferably hydrogen,
more preferred J is phenyl or 2-thienyl.

Preferred compounds of the invention are:
(2E)-5-Amino-5-methylhex-2-enoic acid N-((1R)-1-((2R)-2-benzyl-3-oxopiperazine-1-carbonyl)-2-(2-naphthyl)ethyl)-N-methylamide: 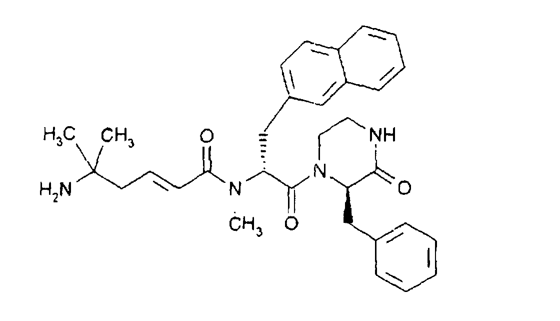
(2E)-5-Amino-5-methylhex-2-enoic acid N-((1R)-1-((2R)-2-benzyl-4-(cyclopropylmethyl)-3-oxopiperazine-1-carbonyl)-2-(2-naphthyl)ethyl)-N-methylamide: 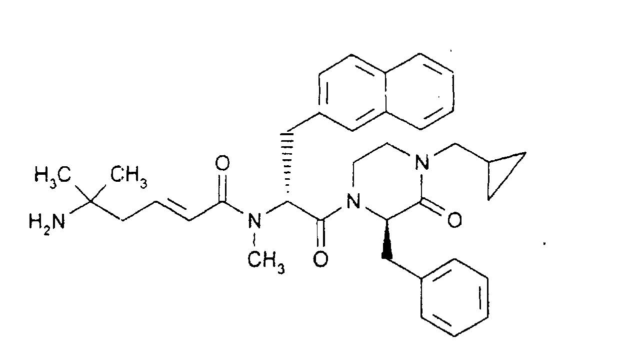
(2E)-5-Amino-5-methylhex-2-enoic acid N-((1R)-1-((2R,5R)-2-benzyl-5-(hydroxymethyl)-3-oxopiperazine-1-carbonyl)-2-(2-naphthyl)ethyl)-N-methylamide: 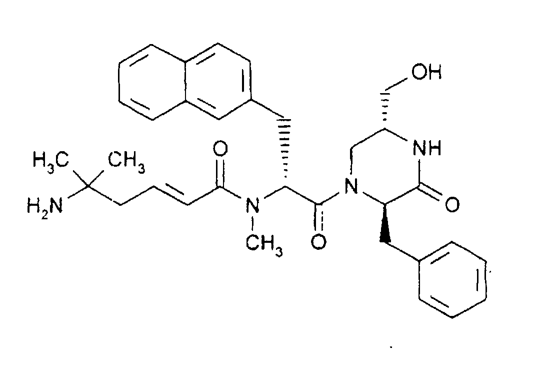
(2E)-5-Amino-5-methylhex-2-enoic acid N-((1R)-1-((2R)-2-benzyl-3-oxo-1,4-diazepane-1-carbonyl)-2-(2-naphthyl)ethyl)-N-methyl amide: 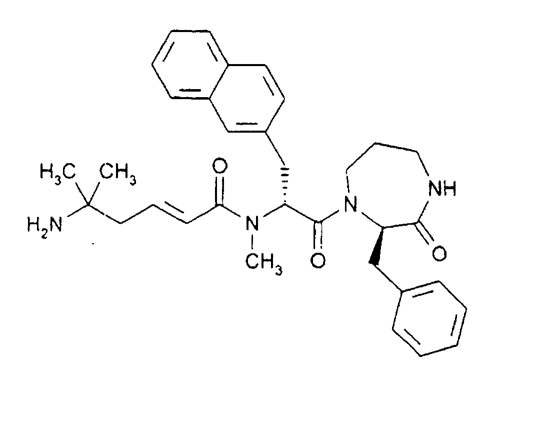
Piperidine-4-carboxylic acid N-methyl-N-((1R)-2-(2-naphthyl)-1-((2R)-2-((2-napthyl)methyl)-3-oxopiperazine-1-carbonyl)ethyl) amide: 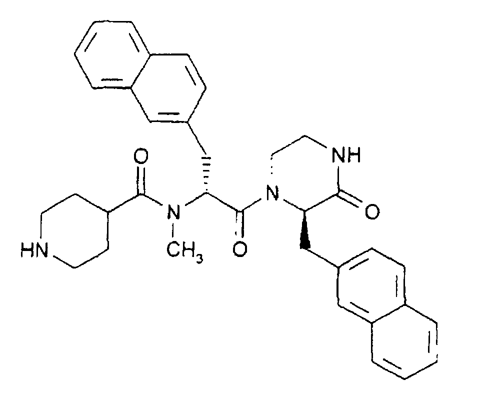
(2E)-5-Amino-5-methylhex-2-enoic acid N-((1R)-1-((2R)-2-benzyl-4-(dimethylcarbamoylmethyl)-3-oxopiperazine-1-carbonyl)-2-(2-naphthyl)ethyl)-N-methyl amide: 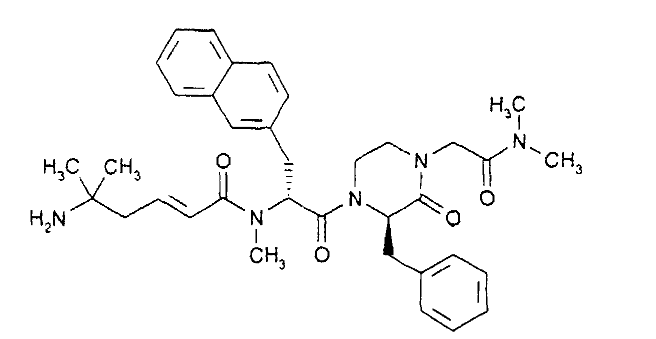
(2E)-5-Methyl-5-(methylamino)hex-2-enoic acid N-((1R)-1-((2R)-2-benzyl-3-oxopiperazine-1-carbonyl)-2-(2-naphthyl)ethyl)-N-methylamide: 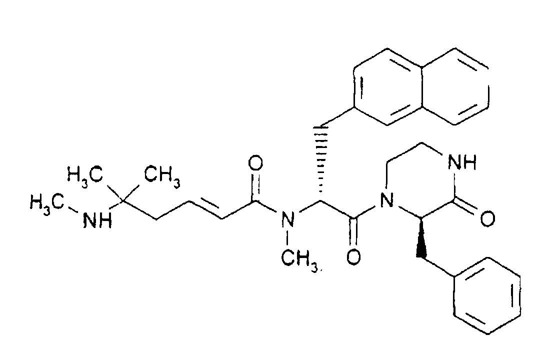
(2E)-5-Amino-5-methylhex-2-enoic acid N-((1R)-1-((2R,6S)-2-benzyl-6-methyl-3-oxopiperazine-1-carbonyl)-2-(2-naphthyl)ethyl)-N-methylamide: 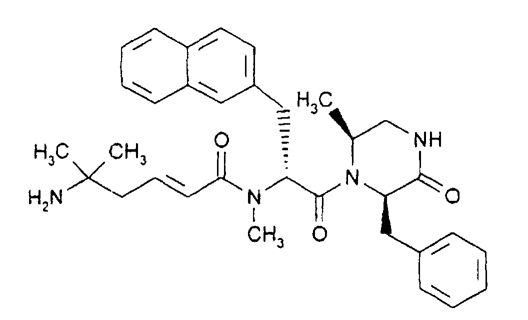
(2E)-5-Amino-5-methylhex-2-enoic acid N-((1R)-1-((2R)-2-benzyl-3-oxopiperazine-1-carbonyl)-2-(biphenyl-4-yl)ethyl)-N-methylamide: 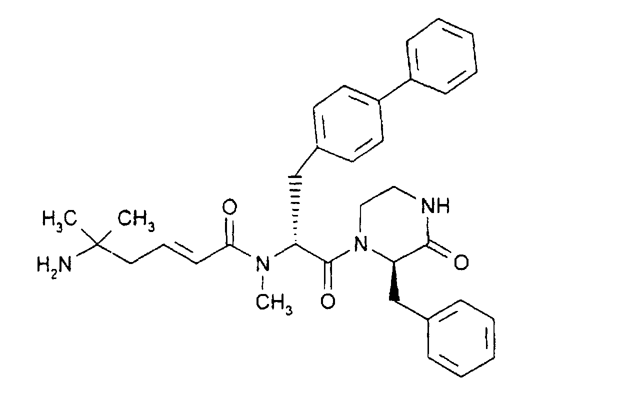
(2E)-5-Amino-5-methylhex-2-enoic acid N-((1R)-1-((2R)-2-benzyl-4-methanesulfonylpiperazine-1-carbonyl)-2-(2-naphthyl)ethyl)-N-methylamide: 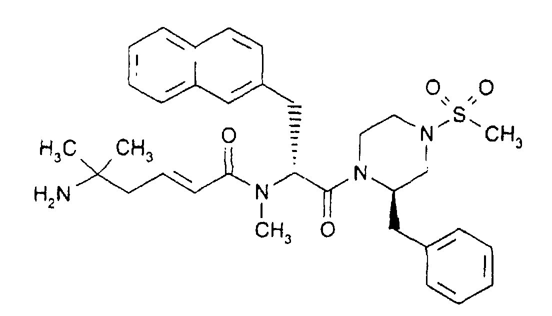
(2E)-5-Amino-5-methylhex-2-enoic acid N-((1R)-1-((2R)-2-benzyl-4-methanesulfonylpiperazine-1-carbonyl)-2-benzyloxyethyl)-N-methylamide: 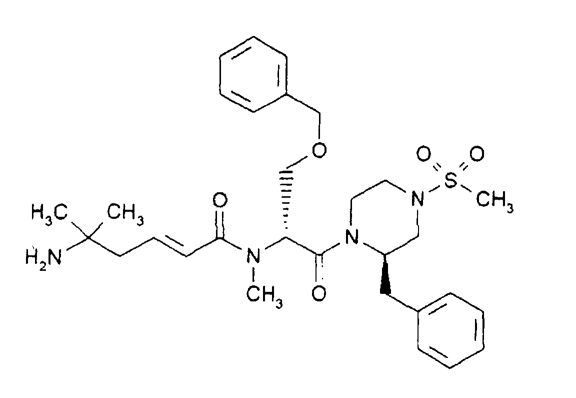
(2E)-5-Amino-3,5-dimethylhex-2-enoic acid N-((1R)-1-((2R)-2-benzyl-3-oxopiperazine-1-carbonyl)-2-(2-naphthyl)ethyl)-N-methylamide. 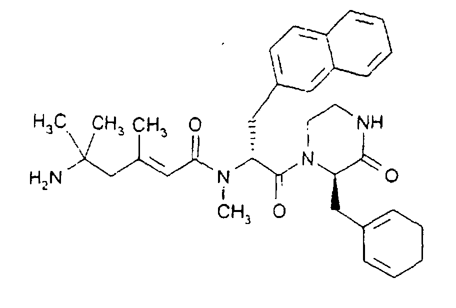
(2E)-5-Methyl-5-methylaminohex-2-enoic acid N-((1R)-1-((2R,5R)-2-benzyl-5-methyl-3-oxopiperazine-1-carbonyl)-2-(2-naphthyl)ethyl)-N-methylamide. 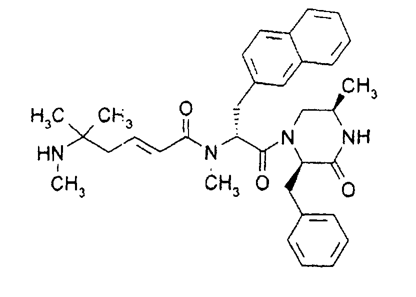
(2E)-5-Amino-5-methylhex-2-enoic acid N-((1R)-2-((2R)-2-benzyl-4-methyl-3-oxopiperazin-1-yl)-1-((2-naphthyl)methyl)-2-oxoethyl)-N-methylamide 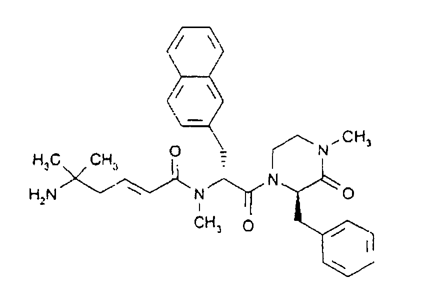
(2E)-5-Methyl-5-aminohex-2-enoic acid N-((1R)-1-((2R,5R)-2-benzyl-5-methyl-3-oxo piperazine-1-carbonyl)-2-(2-naphthyl)ethyl)-N-methylamide. 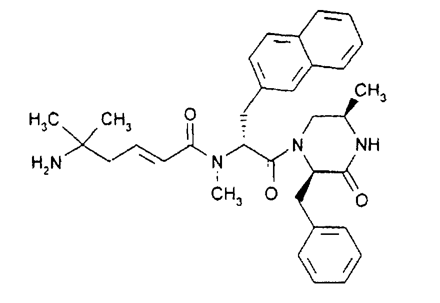
(2E)-4-(1-Aminocyclobutyl)but-2-enoic acid N-methyl-N-((1R)-2-(2-naphthyl)-1-((2R)-3-oxo-2-((2-thienyl)methyl)piperazine-1-carbonyl)ethyl)amide 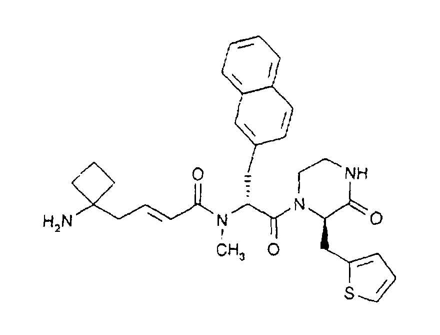
(2E)-4-(1-Aminocyclobutyl)but-2-enoic acid N-((1R)-2-((2R,5R)-2-benzyl-5-methyl-3-oxopiperazin-1-yl)-1-((2-naphthyl)methyl)-2-oxoethyl)-N-methylamide 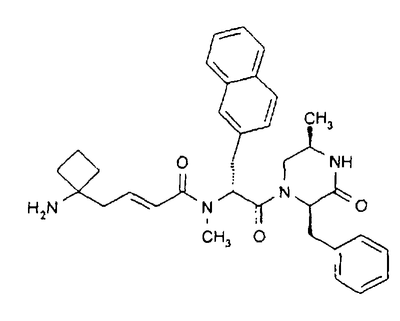
(2E) 5-Methyl-5-methylamino-hex-2-enoic acid N-methyl-N-((1R)-1-(2-naphthyl)methyl-2-oxo-2-((2R)-3-oxo-2-((2-thienyl)methyl)piperazin-1-yl)ethyl)amide. 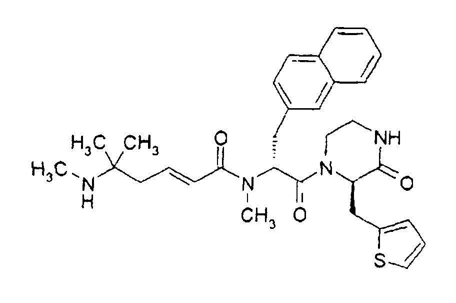
(2E)-5-Amino-5-methyl-hex-2-enoic acid (2-((2R)-2-benzyl-4-methanesulfonylpiperazin-1-yl)-(1R)-1-(napht-2-ylmethyl)-2-oxo-ethyl)-methyl-amide.

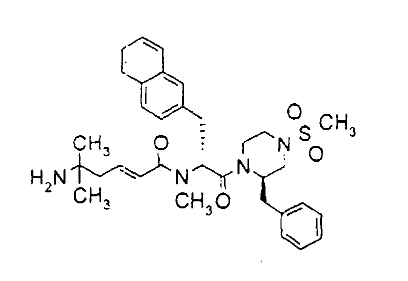

It is believed that compounds of formula 1 exhibit an improved resistance to proteolytic degradation by enzymes compared to that of the peptides suggested in the prior literature, due to the lack of natural peptide bonds. The increased resistance to proteolytic degradation combined with the reduced size of the compounds of the invention in comparison with known growth hormone releasing peptides is expected to improve their bioavailability compared to that of the peptides suggested in the prior literature.

In the above structural formulas and throughout the present specification, the following terms have the indicated meanings:
The C₁₋₆-alkyl, or C₁₋₄-alkyl groups specified above are intended to include those alkyl groups of the designated length in either a linear or branched or cyclic configuration. Examples of linear alkyl are methyl, ethyl, propyl, butyl, pentyl, and hexyl. Examples of branched alkyl are isopropyl, sec-butyl, tert-butyl, isopentyl, and isohexyl. Examples of cyclic alkyl are C₃₋₆-cycloalkyl such as cyclopropyl, cyclobutyl, cyclopentyl and cyclohexyl.

The C₁₋₆-alkoxy groups specified above are intended to include those alkoxy groups of the designated length in either a linear or branched or cyclic configuration. Examples of linear alkyloxy are methoxy, ethoxy, propoxy, butoxy, pentoxy, and hexoxy. Examples of branched alkoxy are isopropoxy, sec-butoxy, tert-butoxy, isopentoxy, and isohexoxy. Examples of cyclic alkoxy are cyclopropyloxy, cyclobutyloxy, cyclopentyloxy and cyclohexyloxy.

In the present context, the term "aryl" is intended to include aromatic rings, such as carbocyclic and heterocyclic aromatic rings selected from the group consisting of phenyl, naphthyl, pyridyl, 1-H-tetrazol-5-yl, thiazolyl, imidazolyl, indolyl, pyrimidinyl, thiadiazolyl, pyrazolyl, oxazolyl, isoxazolyl, oxadiazolyl, thiopheneyl, quinolinyl, pyrazinyl, or isothiazolyl, optionally substituted by one or more C₁₋₆-alkyl, C₁₋₆-alkoxy, halogen, amino or aryl. Aryl is preferably phenyl, thienyl, imidazolyl, oxadiazolyl, pyridyl, indolyl, quinolinyl or naphthyl optionally substituted with halogen, amino, hydroxy, C₁₋₆-alkyl or C₁₋₆-alkoxy.

The term "halogen" is intended to include chlorine (Cl), fluorine (F), bromine (Br) and iodine (I).

The compounds of the present invention may have one or more asymmetric centres and it is intended that stereoisomers, as separated, pure or partially purified stereoisomers or racemic mixtures thereof are included in the scope of the invention.

The compounds of the present invention may optionally be on a pharmaceutically acceptable salt form such as the pharmaceutically acceptable acid addition salts of compounds of formula I which include those prepared by reacting the compound of formula I with an inorganic or organic acid such as hydrochloric. hydrobromic, sulfuric, acetic, phosphoric, lactic, maleic, phthalic, citric, glutaric, gluconic, methanesulfonic, salicylic, succinic, tartaric, toluenesulfonic, trifluoracetic, sulfamic or fumaric acid.

The compounds of formula I may be administered in pharmaceutically acceptable acid addition salt form or, where appropriate, as a alkali metal or alkaline earth metal or lower alkylammonium salt. Such salt forms are believed to exhibit approximately the same order of activity as the free base forms.

In another aspect, the present invention relates to a pharmaceutical composition comprising, as an active ingredient, a compound of the general formula I or a pharmaceutically acceptable salt thereof together with a pharmaceutically acceptable carrier or diluent.

Pharmaceutical compositions containing a compound of the present invention may be prepared by conventional techniques, e.g. as described in Remington's Pharmaceutical Sciences, 1985 or in Remington: The Science and Practice of Pharmacy, 19th Edition (1995). The compositions may appear in conventional forms, for example capsules, tablets, aerosols, solutions, suspensions or topical applications.

The pharmaceutical carrier or diluent employed may be a conventional solid or liquid carrier. Examples of solid carriers are lactose, terra alba, sucrose, cyclodextrin, talc, gelatin, agar, pectin, acacia, magnesium stearate, stearic acid or lower alkyl ethers of cellulose. Examples of liquid carriers are syrup, peanut oil, olive oil, phospholipids, fatty acids, fatty acid amines, polyoxyethylene or water.

Similarly, the carrier or diluent may include any sustained release material known in the art, such as glyceryl monostearate or glyceryl distearate, alone or mixed with a wax.

If a solid carrier is used for oral administration, the preparation may be tabletted, placed in a hard gelatin capsule in powder or pellet form or it can be in the form of a troche or lozenge. The amount of solid carrier will vary widely but will usually be from about 25 mg to about 1 g. If a liquid carrier is used, the preparation may be in the form of a syrup, emulsion, soft gelatin capsule or sterile injectable liquid such as an aqueous or non-aqueous liquid suspension or solution.

A typical tablet which may be prepared by conventional tabletting techniques may contain:

| Core: | |
|---|---|
| Active compound (as free compound or salt thereof) | 100mg |
| Colloidal silicon dioxide (Aerosil) | 1.5mg |
| Cellulose, microcryst. (Avicel) | 70mg |
| Modified cellulose gum (Ac-Di-Sol) | 7.5mg |
| Magnesium stearate | |
| | |

| Coating: | |
|---|---|
| HPMC approx. | 9mg |
| *Mywacett 9-40 T approx. | 0.9mg |

| | |
|---|---|
| *Acylated monoglyceride used as plasticizer for film coating. | |

For nasal administration, the preparation may contain a compound of formula I dissolved or suspended in a liquid carrier, in particular an aqueous carrier, for aerosol application. The carrier may contain additives such as solubilizing agents, e.g. propylene glycol, surfactants, absorption enhancers such as lecithin (phosphatidylcholine) or cyclodextrin, or preservatives such as parabenes.

Generally, the compounds of the present invention are dispensed in unit dosage form comprising 50-200 mg of active ingredient together with a pharmaceutically acceptable carrier per unit dosage.

The dosage of the compounds according to this invention is suitably 0.1-500 mg/day, e.g. from about 5 to about 50 mg, such as about 10 mg per dose, when administered to patients, e.g. humans, as a drug.

It has been demonstrated that compounds of the general formula I possess the ability to release endogenous growth hormone *in vivo.* The compounds may therefore be used in the treatment of conditions which require increased plasma growth hormone levels such as in growth hormone deficient humans or in elderly patients or livestock.

Thus, in a particular aspect, the present invention relates to a pharmaceutical composition for stimulating the release of growth hormone from the pituitary, the composition comprising, as an active ingredient, a compound of the general formula I or a pharmaceutically acceptable salt thereof together with a pharmaceutically acceptable carrier or diluent.

In a still further aspect, the present invention relates to the use of a compound of the general formula I or a pharmaceutically acceptable salt thereof for the preparation of a medicament for stimulating the release of growth hormone from the pituitary.
To those skilled in the art, it is well known that the current and potential uses of growth hormone in humans are varied and multitudinous. Thus, compounds of formula I can be administered for purposes stimulating release of growth hormone from the pituitary and would then have similar effects or uses as growth hormone itself. The uses of growth hormone may be summarized as follows: stimulation of growth hormone release in the elderly; prevention of catabolic side effects of glucocorticoids, prevention and treatment of osteoporosis, stimulation of the immune system, acceleration of wound healing, accelerating bone fracture repair, accelerating complicated fractures, e.g. disctraction osteogenesis, treatment of wasting secondary to fractures, treatment of growth retardation, treating renal failure or insufficiency resulting from growth retardation, treatment of cardiomyopathy, treatment of chronic liver disease, treatment of thrombocytopenia, treatment of Crohn's disease, treatment of short bowel syndrome, treatment of chronic obstructive pulmonary disease (COPD), treatment of complications associated with transplantation, treatment of physiological short stature including growth hormone deficient children and short stature associated with chronic illness, treatment of obesity and growth retardation associated with obesity, treating growth retardation associated with the Prader-Willi syndrome and Turner's syndrome; accelerating the recovery and reducing hospitalization of burn patients; treatment of intrauterine growth retardation, skeletal dysplasia, hypercortisolism and Cushing's syndrome; induction of pulsatile growth hormone release; replacement of growth hormone in stressed patients, treatment of osteochondrodysplasias, Noonan's syndrome, schizophrenia, depressions, Alzheimer's disease, delayed wound healing and psychosocial deprivation, treatment of pulmonary dysfunction and ventilator dependency, attenuation of protein catabolic responses after major surgery, reducing cachexia and protein loss due to chronic illness such as cancer or AIDS; treatment of hyperinsulinemia including nesidioblastosis, adjuvant treatment for ovulation induction; to stimulate thymic development and prevent the age-related decline of thymic function, treatment of immunosuppressed patients, improvement in muscle strength, mobility, maintenance of skin thickness, metabolic homeostasis, renal homeostasis in the frail elderly, stimulation of osteoblasts, bone remodelling and cartilage growth, stimulation of the immune system in companion animals and treatment of disorder of aging in companion animals, growth promoter in livestock and stimulation of wool growth in sheep.

For the above indications the dosage will vary depending on the compound of formula I employed, on the mode of administration and on the therapy desired. However, generally dosage levels between 0.0001 and 100 mg/kg body weight daily are administered to patients and animals to obtain effective release of endogenous growth hormone. Usually, dosage forms suitable for oral, nasal, pulmonal or transdermal administration comprise from about 0.0001 mg to about 100 mg, preferably from about 0.001 mg to about 50 mg of the compounds of formula I admixed with a pharmaceutically acceptable carrier or diluent.

Optionally, the pharmaceutical composition of the invention may comprise a compound of formula I combined with one or more compounds exhibiting a different activity, e.g., an antibiotic or other pharmacologically active material.

The route of administration may be any route which effectively transports the active compound to the appropriate or desired site of action, such as oral, nasal, pulmonary, transdermal or parenteral, the oral route being preferred.

Apart from the pharmaceutical use of the compounds of formula I, they may be useful in vitro tools for investigating the regulation of growth hormone release.

Compounds of formula I may also be useful in vivo tools for evaluating the growth hormone releasing capability of the pituitary. For example, serum samples taken before and after administration of these compounds to humans can be assayed for growth hormone. Comparison of the growth hormone in each serum sample would directly determine the ability of the patients pituitary to release growth hormone.

Compounds of formula I may be administered to commercially important animals to increase their rate and extent of growth, and to increase milk production.

A further use of growth hormone secretagogue compounds of formula I is in combination with other secretagogues such as GHRP (2 or 6), GHRH and its analogues, growth hormone and its analogues or somatomedins including IGF-1 and IGF-2.

### Pharmacological Methods

Compounds of formula I may be evaluated in vitro for their efficacy and potency to release growth hormone in rat pituitary primary cultures.

The isolation of rat pituitary cells is a modification of O. Sartor et al., Endocrinology 116, 1985, pp. 952-957. Male albino Sprague-Dawley rats (250 +/-25 grams) were purchased from Møllegaard, Lille Skensved, Denmark. The rats were housed in group cages (four animals/cage) and placed in rooms with 12 hour light cycle. The room temperature varied from 19-24°C and the humidity from 30 - 60%,

The rats were decapitated and the pituitaries dissected. The neurointermediate lobes were removed and the remaining tissue was immediately placed in icecold isolation buffer (Gey's medium (Gibco 041-04030) supplemented with 0.25% D-glucose, 2% non-essential amino acids (Gibco 043-01140) and 1% bovine serum albumine (BSA) (Sigma A-4503)). The tissue was cut into small pieces and transferred to isolation buffer supplemented with 3.8 mg/ml of trypsin (Worthington #3707 TRL-3) and 330 µg/ml of DNase (Sigma D-4527). This mixture was incubated at 70 rotations/min for 35 min at 37°C in a 95/5% atmosphere of O₂/CO₂. The tissue was then washed three times in the above buffer. Using a standard pasteur pipette, the tissue was then aspirated into single cells. After dispersion, cells were filtered through a nylon filter (160 µm) to remove undigested tissue. The cell suspension was washed 3 times with isolation buffer supplemented with trypsin inhibitor (0.75 mg/ml, Worthington #2829) and finally resuspended in culture medium; DMEM (Gibco 041-01965) supplemented with 25 mM HEPES (Sigma H-3375), 4 mM glutamine (Gibco 043-05030H), 0.075% sodium bicarbonate (Sigma S-8875), 0.1% non-essential amino acid, 2.5% fetal calf serum (FCS, Gibco 011-06290), 3% horse serum (Gibco 034-06050), 10% fresh rat serum, 1 nM T₃ (Sigma T-2752) and 40 µg/l dexamethasone (Sigma D-4902) pH 7.3, to a density of 2 x 10⁵ cells/ml. The cells were seeded into microtiter plates (Nunc, Denmark), 200 µl/well, and cultured for 3 days at 37°C and 8% CO₂.

### Compound testing

After culturing, the cells were washed twice with stimulation buffer (Hanks Balanced Salt Solution (Gibco 041-04020) supplemented with 1% BSA (Sigma A-4503), 0.25% D-glucose (Sigma G-5250) and 25 mM HEPES (Sigma H-3375) pH 7.3) and preincubated for 1 hour at 37°C. The buffer was exchanged with 90 µl stimulation buffer (37°C). Ten µl test compound solution was added and the plates were incubated for 15 min at 37°C and 5% CO₂. The medium was decanted and analyzed for GH content in an rGH SPA test system.

All compounds were tested in doses ranging from 10 pM to 100 µM. A dose-response relation was constructed using the Hill equation (Fig P, Biosoft). The efficacy (maximal GH released, Eₘₐₓ) was expressed in % of the Eₘₐₓ of GHRP-6. The potency (EC₅₀) was determined as the concentration inducing half maximal stimulation of the GH release.

Compounds of formula I may be evaluated for their metabolic stability.

Compounds were dissolved at a concentration of 1 µg/µl in water. 25 µl of this solution is added to 175 µl of the respective enzyme-solution (resulting in an enzyme:substrate ratio (w/w) of approximately 1:5). The solution is left at 37°C overnight. 10 µl of the various degradation solutions is analyzed against a corresponding zero-sample using flow injection electrospray mass spectrometry (ESMS) with selected ion monitoring of the molecular ion. If the signal has decreased more than 20% compared to the zero-sample, the remainder of the solution is analyzed by HPLC and mass spectrometry in order to identify the extent and site(s) of degradation precisely.

Several standard peptides (ACTH 4-10, Angiotensin 1-14 and Glucagon) have been included in the stability tests in order to verify the ability of the various solutions to degrade peptides.

Standard peptides (angiotensin 1-14, ACTH 4-10 and glucagon) were purchased from Sigma, MO, USA)

Enzymes (trypsin, chymotrypsin, elastase aminopeptidase M and carboxypeptidase Y and B) were all purchased from Boehringer Mannheim GmbH (Mannheim, Germany)

Pancreatic enzyme mix: trypsin, chymotrypsin and elastase in 100 mM ammoniumbicarbonate pH 8.0 (all concentrations 0.025 µg/µl).

Carboxypeptidase mix: carboxypeptidase Y and B in 50 mM ammoniumacetate pH 4.5 (all concentrations 0.025 µg/µl).

Aminopeptidase M solution: aminopeptidase M (0.025 µg/µl) in 100 mM ammoniumbicarbonate pH 8.0

Mass spectrometric analysis was performed using two different mass spectrometers. A Sciex API III triple quadrupole LC-MS instrument (Sciex instruments, Thornhill, Ontario) equipped with an electrospray ion-source and a Bio-lon 20 time-of-flight Plasma Desorption instrument (Bio-lon Nordic AB, Uppsala, Sweden).

Quantification of the compounds (before and after degradation) was done on the API III instrument using single ion monitoring of the molecular ion in question with flow injection of the analyte. The liquid flow (MeOH:water 1:1) of 100 µl/min was controlled by an ABI 140B HPLC unit (Perkin-Elmer Applied Biosystems Divisions, Foster City, CA). The instrument parameters were set to standard operation conditions, and SIM monitoring was performed using the most intense molecular ion (in most cases this corresponded to the doubly charged molecular ion).

Identification of degradation products furthermore involved the use of plasma desorption mass spectrometry (PDMS) with sample application on nitrocellulose coated targets and standard instrumental settings. The accuracy of the hereby determined masses is generally better than 0.1%.

Separation and isolation of degradation products was done using a HY-TACH C-18 reverse phase 4.6x105 mm HPLC column (Hewlett-Packard Company, Palo Alto, CA) with a standard acetonitril: TFA separation gradient. The HPLC system used was HP1090M (Hewlett-Packard Company, Palo Alto, CA).

| Peptide derivative | MW/SIM ion (amu) | Carboxypeptidase mix | Pan. enzyme mix |
|---|---|---|---|
| Standards | | | |
| ACTH 4-10 | 1124.5/562.8 | + | - |
| Glucagon | 3483/871.8 | - | - |
| Insulin (B23-29) | 859.1/430.6 | | |
| Angiotensin 1-14 | 1760.1/881.0 | - | - |
| GHRP-2 | 817.4/409.6 | - | - |
| GHRP-6 | 872.6/437.4 | - | - |
| +: Stable (less than 20% decrease in SIM signal after 24 h in degradation solution) -. Unstable (more than 20% decrease in SIM signal after 24 h in degradation solution) | | | |

### Chemical Methods

Some compounds of formula I or intermediates may be synthesized by one of the reactions sequences shown. Other examples may require different synthetic approaches, depending of the type of compound. The specific synthesis of each example is therefore given in the description of each example.

### Method A

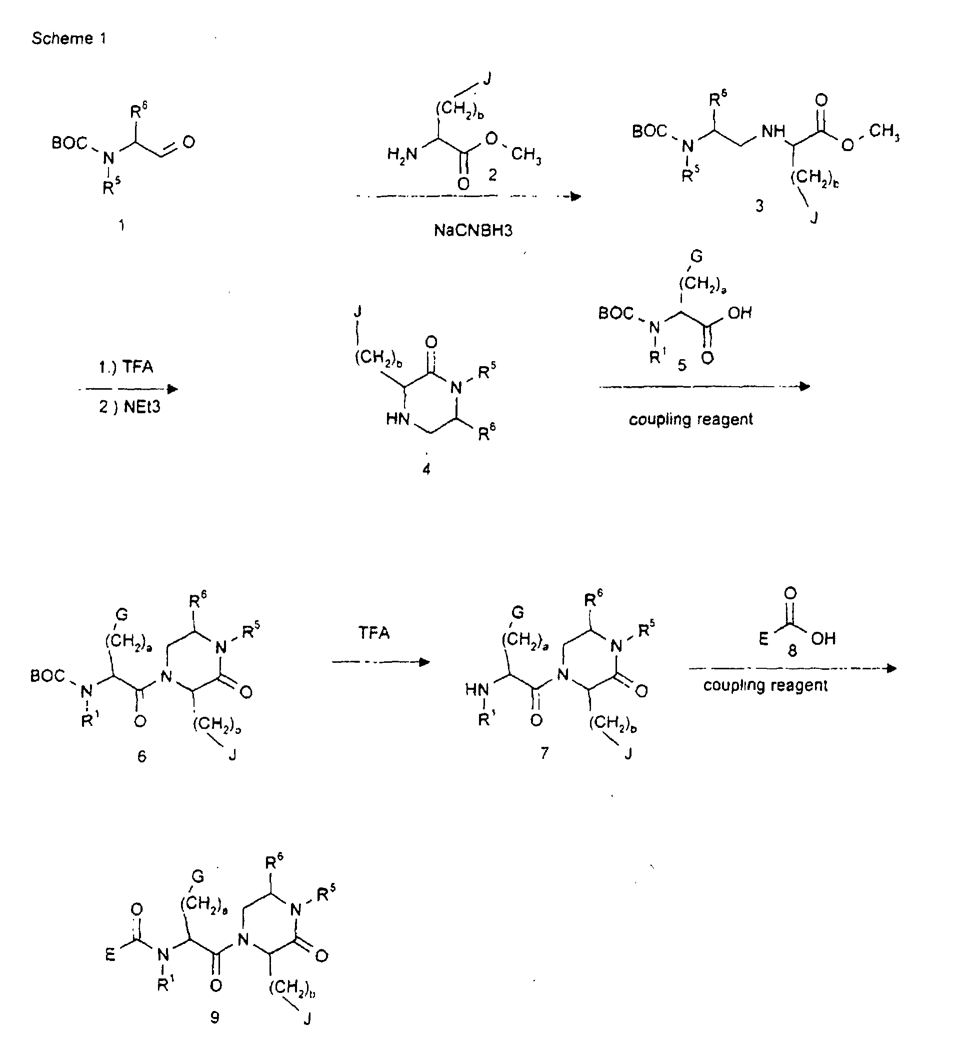

An aldehyde **1** may be reacted with an ester **2** under reductive conditions such as e.g. sodium cyano borohydride to give an ester **3.** After deprotection of the amino group a ring closure may take place to give a piperazinone **4.** A reaction with a suitable protected amino acid **5** with a coupling reagent known in the art such as e.g. 1-ethyl-3-(3-dimethylaminopropyl)carbodiimide hydrochloride or a combination of 1-ethyl-3-(3-dimethylaminopropyl)carbodiimide hydrochloride and 1-hydroxybenzotriazole or 1-hydroxy-7-azabenzotriazole in an appropriate solvent such as e.g. N.N-dimethylformamide or dichloromethane may produce the piperazinone **6**. After deprotection carried out with a method known in the art and described by e.g. T. W. Greene (Protective Groups in Organic Synthesis, 2. ed., John Wiley and Sons, New York 1991) e.g. trifluoroacetic acid or hydrogen chloride in ethyl acetate, the resulting amine **7** may be coupled with a suitable protected amino acid **8** with a coupling reagent known in the art such as e.g. 1-ethyl-3-(3-dimethylaminopropyl)carbodiimide hydrochloride or a combination of 1-ethyl-3-(3-dimethylaminopropyl)carbodiimide hydrochloride and 1-hydroxybenzotriazole or 1-hydroxy-7-azabenzotriazole in an appropriate solvent such as e.g. N,N-dimethylformamide or dichloromethane and successive deprotection with a method known in the art and described by e.g. T. W. Greene (Protective Groups in Organic Synthesis, 2. ed., John Wiley and Sons, New York 1991) e.g. trifluoroacetic acid or hydrogen chloride in ethyl acetate to give **9**, which is a compound of formula I.

### Method B

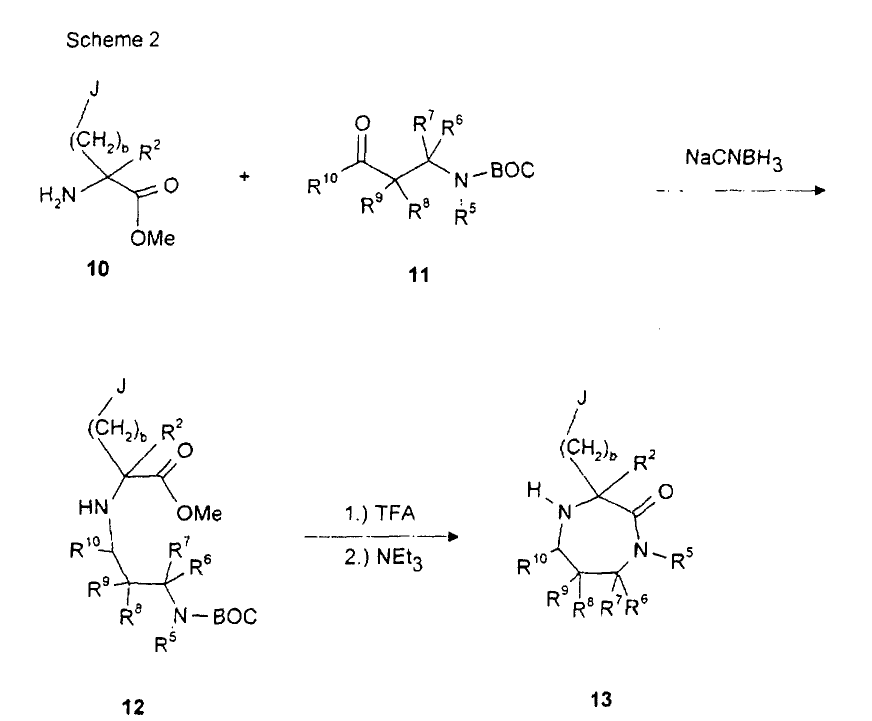

A diazepinone may be synthesized as described in scheme 2 and incorporated into the peptide in a similar fashion as in scheme 1 by reductive alkylation of a carbonyl-compound **11** onto an amino acid ester **10** under dehydrating conditions such as e.g. molecular sieves and sodium cyanoborohydride in a solvent such as e.g. methanol and subsequent removal of a protecting group from **12**. This may result in cyclization of the intermediate to form **13**, **13** may be used as an intermediate, which may be reacted in the same manner as described in scheme 1 for the intermediate **4** to give a compound of formula I.

### EXAMPLES:

The process for preparing compounds of formula I and preparations containing them is further illustrated in the following examples, which however, are not to be construed as limiting.

The structures of the compounds are confirmed by either elemental analysis (MA) nuclear magnetic resonance (NMR) or mass spectrometry (MS). NMR shifts (δ) are given in parts per million (ppm) and only selected peaks are given. mp is melting point and is given in °C. Column chromatography was carried out using the technique described by W.C. Still et al, J. Org. Chem. 1978, 43, 2923-2925 on Merck silica gel 60 (Art 9385). Compounds used as starting materials are either known compounds or compounds which can readily be prepared by methods known per se.

### Abbrevations:

- TLC:: thin layer chromatography
- DMSO:: dimethylsulfoxide
- min:: minutes
- h:: hours

### HPLC-Analysis:

### Method A1.

The RP-analysis was performed using UV detections at 214, 254, 276, and 301 nm on a 218TP54 4.6 mm x 250 mm 5µ C-18 silica column (The Seperations Group Hesperia), which was eluted at 1 mL/min at 42°C. The column was equilibrated with 5% acetonitrile in a buffer consisting of 0.1 M ammonium sulfate, which was adjusted to pH 2.5 with 4M sulfuric acid. after injection the sample was eluted by a gradient of 5% to 60% acetonitrile in the same buffer during 50 min.

### Example 1

(2E)-5-Amino-5-methylhex-2-enoic acid N-((1R)-1-((2R)-2-benzyl-3-oxopiperazin-1-carbonyl)-2-(2-naphthyl)ethyl)-N-methylamide.

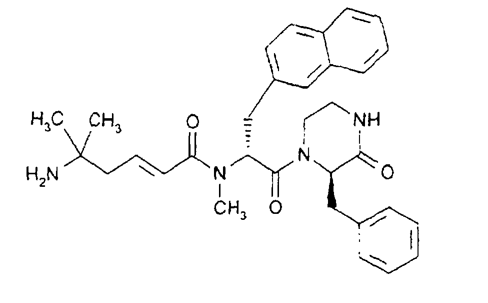

(2R)-2-(2-(tert-Butoxycarbonylamino)ethylamino)-3-phenylpropionic acid methyl ester.

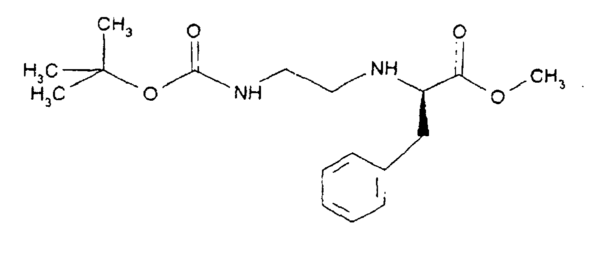

(2R)-2-Amino-3-phenylpropionic acid methyl ester (3.2 g; 15.0 mmol) and (2-oxoethyl)carbamic acid tert-butyl ester (3.2 g; 20.0 mmol) (prepared as in Dueholm et al, Org. Prep. Proced. Int. 25(4), 457-61 (1993)) were dissolved in a mixture of methanol (100 ml) and acetic acid (5 ml). Molecular sieves (3 Å; 100 g) were added and the reaction mixture was cooled to 0°C. Sodium cyanoborohydride (1.38 g; 22.0 mmol) was added portionwise and the reaction mixture was left without stirring for 12 h. The reaction mixture was filtered and water (100 ml), aqueous sodium hydrogencarbonate/sodium carbonate (10%; 100 ml; pH 9) and methylene chloride (50 ml) were added to the filtrate. The aqueous phase was extracted with methylene chloride (2 x 50 ml) and the combined organic phases were dried over magnesium sulfate. The organic phase was evaporated in vacuo and the residue was chromatographed on silica (4 x 40 cm) using ethyl acetate/heptane (3:2) as eluent to afford 2.07 g of (2R)-2-(2-(tert-butoxycarbonylamino)ethylamino)-3-phenylpropionic acid methyl ester.
¹H-NMR (CDCl₃): δ 1.44 (s, 9H); 2.55 (m, 1H); 2.72 (m, 1H); 2.90 (dd, 1H); 2.97 (dd, 1H); 3.11 (m, 2H); 3.47 (t, 1H); 3.68 (s, 3H); 7.15-7.32 (arom., 5H);

(2R)-2-(2-Aminoethylamino)-3-phenylpropionic acid methyl ester.

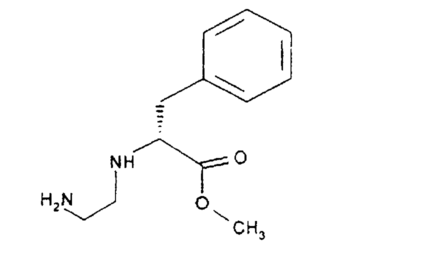

(2R)-2-(2-(tert-Butoxycarbonylamino)ethylamino)-3-phenylpropionic acid methyl ester (1.32 g; 4.094 mmol) was dissolved in methylene chloride (10 ml) and trifluoroacetic acid (10 ml) was added. The reaction mixture was stirred for 1 hour at room temperature. The solvent was evaporated in vacuo and the residue was dissolved in methylene chloride (5 ml) and evaporated in vacuo. Methylene chloride (5 ml) was added and evaporated in vacuo to afford 1.14 g of (2R)-2-(2-aminoethylamino)-3-phenylpropionic acid methyl ester as a trifluoroacetate salt.
¹H-NMR (DMSO-d₆): δ 3.03-3.39 (m, 6H); 3.66 (s, 3H); 4.45 (dd, 1H); 7.21-7.39 (arom., 5H)

(3R)-3-Benzylpiperazin-2-one.

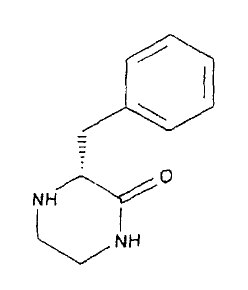

(2R)-2-(2-Aminoethylamino)-3-phenylpropionic acid methyl ester (0.67 g; 1.49 mmol) and triethylamine (1 ml) were dissolved in methylene chloride (10 ml) and stirred for 48 hours at room temperature. The reaction mixture was evaporated and the residue was chromatographed on silica (2.5 x 30 cm) using a 7% solution of ammonia in ethanol/methylene chloride (1:9) as eluent to afford 0.18 g of (3R)-3-benzylpiperazin-2-one.
¹H-NMR (CDCl₃): δ 1.72 (s(br), 1H); 2.81-3.48 (m, 6H); 3.62 (dd, 1H); 6.92 (s(br), 1H); 7.19-7.36 (arom., 5H).

N-((1R)-2-((2R)-2-Benzyl-3-oxopiperazin-1-yl)-1-((2-naphthyl)methyl)-2-oxoethyl)-N-methylcarbamic acid tert-butyl ester.

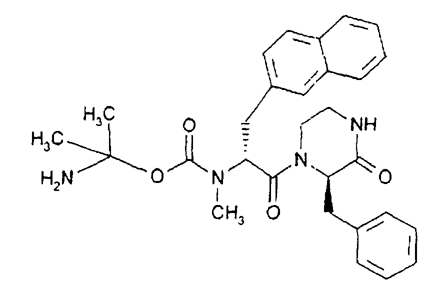

(2R)-2-(tert-Butoxycarbonylmethylamino)-3-(2-naphthyl)propionic acid (prepared as in J.R. Coggins, N.L. Benoitory, Can.J.Chem 49, 1968 (1971)) (0.327 g; 0.995 mmol) was dissolved in methylene chloride (10 ml), 1-Hydroxy-7-azabenzotriazole (0.135 g; 0.995 mmol) and N-(3-dimethylaminopropyl)-N'-ethylcarbodiimide hydrochloride (0.199 g; 1.042 mmol) were added and the reaction mixture was stirred for 10 min at room temperature. (3R)-3-Benzylpiperazin-2-one (0.180 g; 0.947 mmol) and diisopropylethylamine (0.134 g; 1.042 mmol) were added and the reaction mixture was stirred for 12 hours at room temperature. The reaction mixture was washed with 10% sodium hydrogensulfate (20 ml), saturated sodium hydrogencarbonate (20 ml), dried over magnesium sulfate and evaporated in vacuo. The residue was chromatographed on silica (2.5 x 25 cm) using ethyl acetate as eluent to afford 0.405 g of N-((1R)-2-((2R)-2-benzyl-3-oxopiperazin-1-yl)-1-((2-naphthyl)methyl)-2-oxoethyl)-N-methylcarbamic acid tert-butyl ester.
¹H-NMR (CDCl₃): δ 1.12, 1.25, 1.38 (three s, 9H); 2.54, 2,55, 2.78 (three s, 3H); 4.07, 4.42, 4.65, 4.79, 5.01, 5.30 (six m; 2H); 7.10-7.78 (arom., 12 H) (mixture of rotamers, selected peaks).

(3R)-3-Benzyl-4-((2R)-2-methylamino-3-(2-naphthyl)propionyl)piperazin-2-one.

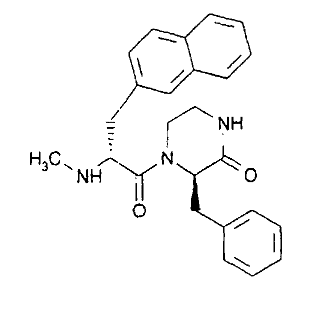

N-((1R)-2-((2R)-2-Benzyl-3-oxopiperazin-1-yl)-1-((2-naphthyl)methyl)-2-oxoethyl)-N-methylcarbamic acid tert-butyl ester (0.405 g; 0.808 mmol) was dissolved in methylene chloride (5 ml) and trifluoroacetic acid (5 ml) and stirred 10 min at room temperature. Water (10 ml) and sodium hydrogencarbonate were added to pH 8. The reaction mixture was extracted with methylene chloride (2 x 30 ml), dried over magnesium sulfate and evaporated in vacuo to afford 0.302 g of (3R)-3-benzyl-4-((2R)-2-methylamino-3-(2-naphthyl)propionyl)piperazin-2-one.
¹H-NMR (CDCl₃): δ 2.19, 2.28 (two s, 3H); 3.72, 4.66 (two dd, 1H); 4.96, 5.32 (two dd, 1H) (mixture of rotamers, selected peaks).

3-Hydroxy-1,1-dimethylpropylcarbamic acid tert-butyl ester.

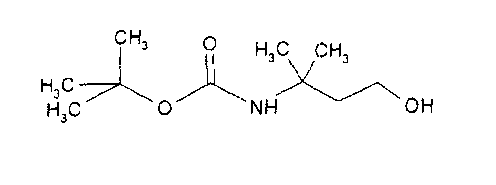

At 0°C, ethyl chloroformate (1.10 ml, 11.5 mmol) was given dropwise to a solution of 3-tert-butoxycarbonylamino-3-methylbutanoic acid (2.50 g, 11.5 mmol) (cf. Schoen et al. J. Med. Chem., 1994, vol. 37, p. 897-906, and references cited herein) and triethylamine (1.92 ml, 13.8 mmol) in tetrahydrofuran (10 ml). The solution was stirred for 40 min at 0°C. The formed precipitate was filtered off and washed with tetrahydrofuran (20 ml). The liquid was immediately cooled to 0°C. A 2M solution of lithium borohydride in tetrahydrofuran (14.4 ml, 28.8 mmol) was added dropwise. The solution was stirred at 0°C for 2 h, and then warmed to room temperature over a period of 4 h. It was cooled to 0°C and methanol (5 ml) was added. 1N Hydrochloric acid (100 ml) was added and the solution was extracted with ethyl acetate (2 x 100 ml, 3 x 50 ml). The combined organic extracts were washed with saturated sodium hydrogencarbonate solution (100 ml) and dried over magnesium sulfate. The solvent was removed in vacuo. The crude product was chromatographed on silica (110 g) with ethyl aceteate/heptane (1:2) to give 1.84 g of 3-hydroxy-1,1-dimethylpropylcarbamic acid tert-butyl ester.
¹H-NMR (CDCl₃): δ 1.33 (s, 6 H); 1.44 (s, 9 H); 1.88 (t, 2 H); 1.94 (br, 1 H); 3.75 (q, 2 H); 4.98 (br, 1 H).

3-(tert-Butoxycarbonylaminomethyl)-3-methylbutanal.

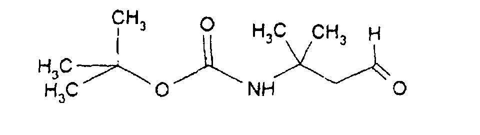

At -78°C dimethylsulfoxide (1.22 ml, 17.2 mmol) was added to a solution of oxalyl chloride (1.1 ml, 12.9 mmol) in dichloromethane (15 ml). The mixture was stirred for 15 min at -78°C. A solution of 3-hydroxy-1,1-dimethylpropylcarbamic acid tert-butyl ester (1.75 g, 8.6 mmol) in dichloromethane (10 ml) was added dropwise over a period of 15 min. The solution was stirred at -78°C for another 15 min. Triethylamine (6.0 ml, 43 mmol) was added. The solution was stirred at -78°C for 5 min and then warmed to room temperature. The solution was diluted with dichloromethane (100 ml) and extracted with 1N hydrochloric acid (100 ml). The aqueous phase was extracted with dichloromethane (50 ml). The combined organic layers were washed with saturated sodium hydrogencarbonate solution (100 ml) and dried over magnesium sulfate. The solvent was removed in vacuo. The crude product was purified by column chromatography on silica (140 g) with ethyl acetate/heptane (1:3) to give 1.10 g of 3-(tert-butoxycarbonylaminomethyl)-3-methylbutanal.

¹H-NMR (CDCl₃): δ 1.39 (s, 6 H); 1.45 (s, 9 H); 2.85 (d, 2 H); 4.73 (br. 1 H); 9.80 (t, 1 H).

Ethyl (2E)-5-(tert-butoxycarbonylamino)-5-methylhex-2-enoate.

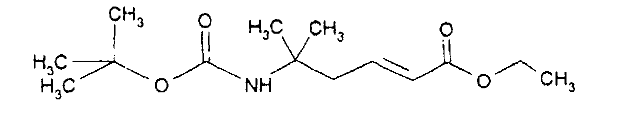

Triethylphosphonoacetate (1.96 ml, 9.8 mmol) was dissolved in tetrahydrofuran (30 ml). Potasium tert-butoxide (1.10 g, 9.8 mmol) was added. The solution was stirred for 40 min. at room temperature. A solution of 3-(tert-butoxycarbonylaminomethyl)-3-methylbutanal (1.10 g, 5.5 mmol) in tetrahydrofuran (6 ml) was added slowly. The solution was stirred at room temperature for 75 min. It was diluted with ethyl acetate (100 ml) and 1N hydrochloric acid (100 ml). The phases were separated. The aqueous phase was extracted with ethyl acetate (2 x 50 ml). The combined organic layers were washed with saturated sodium hydrogencarbonate solution (60 ml) and dried over magnesium sulfate. The solvent was removed in vacuo. The crude product was purified by column chromatography on silica (90 g) with ethyl acetate/heptane (1:4) to give 1.27 g of ethyl (2E)-5-(tert-butoxycarbonylamino)-5-methylhex-2-enoate.
¹H-NMR (CDCl₃): δ 1.30 (s, 6 H); 1.30 (t, 3 H); 1.46 (s, 9 H); 2.62 (d, 2 H); 4.27 (q, 2 H); 4.42 (br, 1 H); 5.88 (d, 1 H); 6.94 (td, 1 H).

(2E)-5-(tert-Butyloxycarbonylamino)-5-methylhex-2-enoic acid.

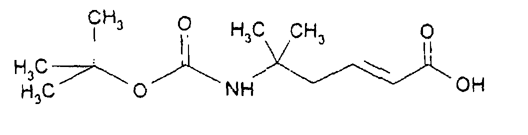

Ethyl (2E)-5-(tert-butoxycarbonylamino)-5-methylhex-2-enoate (1.23 g, 4.54 mmol) was dissolved in dioxane (20 ml). Lithium hydroxide (0.12 g, 5.00 mmol) was added as a solid. Water (10 ml) was added, until a clear solution was obtained. The solution was stirred for 16 h at room temperature. The solution was diluted with water (70 ml) and was extracted with tert-butyl methyl ether (2 x 100 ml). The aqueous phase was acidified with 1N sodium hydrogensulfate solution (pH 1) and extracted with tert-butyl methyl ether (3 x 70 ml). These organic layers were combined and dried over magnesium sulfate. The solvent was removed in vacuo to give 1.05 g of (2E)-5-(tert-butyloxycarbonylamino)-5-methylhex-2-enoic acid. The crude product was used for further syntheses.
¹H-NMR (DMSO-d₆): δ 1.15 (s, 6 H); 1.35 (s, 9 H); 2.53 (d, 2 H); 5.75 (d, 1 H); 6.57 (br, 1 H); 6.75 (td, 1 H); 12.15 (s, 1 H).

((3E)-4-(N-((1R)-2-((2R)-2-Benzyl-3-oxopiperazin-1-yl)-1-((2-naphthyl)methyl)-2-oxoethyl)-N-methylcarbamoyl)-1,1-dimethylbut-3-enyl)carbamic acid tert-butyl ester.

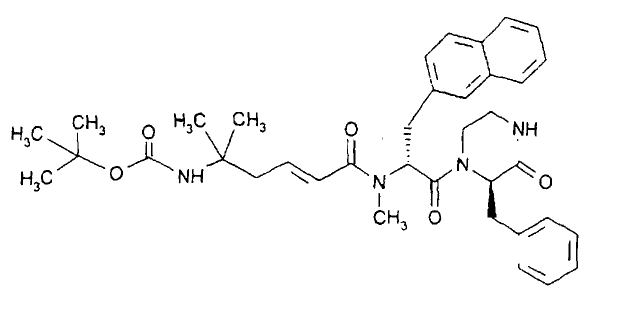

(2E)-5-(tert-Butyloxycarbonylamino)-5-methylhex-2-enoic acid (0.145 g; 0.599 mmol) was dissolved in methylene chloride. 1-Hydroxy-7-azabenzotriazole (0.081 g; 0.599 mmol) and N-(3-dimethylaminopropyl)-N'-ethylcarbodiimide hydrochloride (0.126 g; 0.658 mmol) were added and the reaction mixture was stirred for 15 min. (3R)-3-Benzyl-4-((2R)-2-methylamino-3-(2-naphthyl)propionylpiperazin-2-one (0.24 g; 0.599 mmol) and diisopropylethylamine (0.077 g; 0.599 mmol) were added and the reaction mixture was stirred for 12 hours at room temperature. The reaction mixture was washed with sodium hydrogensulfate (100 ml) and sodium hydrogencarbonate (100 ml). The organic phase was evaporated in vacuo and the residue was chromatographed on silica (2.5 x 20 cm) using ethyl acetate as eluent to afford 0,332 g of ((3E)-4-(N-((1R)-2-((2R)-2-benzyl-3-oxopiperazin-1-yl)-1-((2-naphthyl)-methyl)-2-oxoethyl)-N-methylcarbamoyl)--1,1-dimethylbut-3-enyl)carbamic acid tert-butyl ester.
¹H-NMR (CDCl₃): ) (mixture of rotamers, selected peaks for major rotamer) δ 1.24 (s, 3H); 1.27 (s, 3H); 1.42 (s, 9H); 2.72 (s, 3H); 5.30 (dd, 1H); 5.81 (dd, 1H); 6.18 (d, 1H); 6.82 (m, 1H).

((3E)-4-(N-((2R)-2-((2R)-2-benzyl-3-oxopiperazin-1-yl)-1-((2-naphthyl)methyl)-2-oxo-ethyl)-N-methylcarbamoyl)-1,1-dimethylbut-3-enyl)carbamic acid tert-butyl ester (0.32 g; 0.511 mmol) was dissolved in methylene chloride (4 ml) and trifluoroacetic acid (4 ml) was added. The reaction mixture was stirred 12 min at room temperature. Methylene chloride (50 ml) and water (50 ml) were added. Sodium hydrogencarbonate was added until pH 8. The organic phase was dried over magnesium sulfate and evaporated in vacuo to afford 0.232 g of the title compound.
¹H-NMR (CDCl₃): δ 1.14, 1.15, 1.22, 1.23 (four s, 6H); 4.05, 4.60, 4.76, 5.12, 5.38, 5.81 (six dd, 2H); 6.18, 6.22 (d, 1H); 6.70, 6.81 (two m, 1H) (mixture of rotamers, selected peaks).
ESMS: m/z 527 (M+H)⁺
HPLC (A1): rₜ: 31.03 min.

### Example 2

(2E)-5-Amino-3,5-dimethylhex-2-enoic acid N-((1R)-1-((2R)-2-benzyl-3-oxopiperazine-1-carbonyl)-2-(2-naphthyl)ethyl)-N-methylamide.

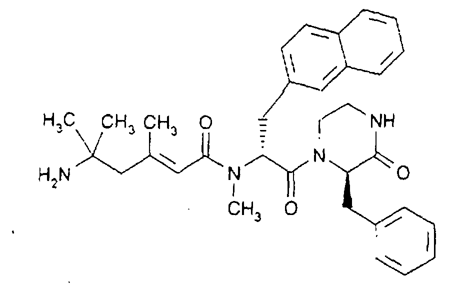

(1,1-Dimethyl-3-oxobutyl)carbamic acid tert-butyl ester:

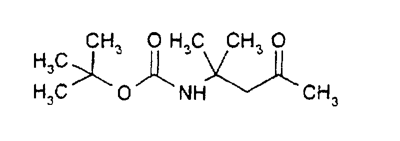

Diacetonamine hydrogen oxalate (30.0 g; 146 mmol) was suspended in tetrahydrofuran (400 ml). An aqueous solution of sodium hydroxide (1 N; 146 ml) was added. Di-tert-butyl dicarbonate (38.3 g; 175 mmol) was dissolved in tetrahydrofuran (100 ml) and added dropwise to the reaction mixture. The reaction mixture was stirred for 2 hours at room temperature. Sodium hydroxide (1 N; 146 ml) was added and the reaction mixture was stirred for 12 h at room temperature. Water (200 ml) and ethyl acetate (200 ml) were added. The aqueous phase was extracted with ethyl acetate (4 x 200 ml). The combined organic phases were dried over magnesium sulfate and the solvent was removed in vacuo. The residue was chromatographed on silica (6 x 40 cm) using ethyl acetate/heptane (1:3) as eluent to afford 28.4 g of (1,1-dimethyl-3-oxobutyl)carbamic acid tert-butyl ester.
¹H-NMR (CDCl₃) δ 1.34 (s, 6H); 1.42 (s, 9H); 2.14 (s, 3H); 2.86 (s, 2H); 4.85 (s, 1H).

(E)-5-tert-Butoxycarbonylamino-3,5-dimethylhex-2-enoic acid ethyl ester:

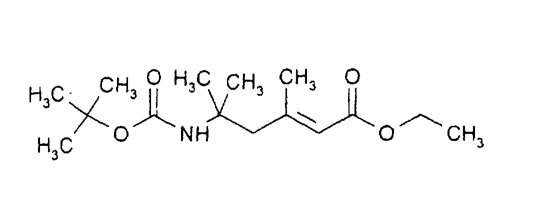

Triethyl phosphonoacetate (4.7 g; 20.9 mmol) was dissolved in tetrahydrofuran (36 ml). Potassium tert-butoxide (2.3 g; 20.9 mmol) was added and the reaction mixture was stirred for 40 min at room temperature. (1,1-Dimethyl-3-oxobutyl)carbamic acid tert-butyl ester (2.5 g; 11.6 mmol) was dissolved in tetrahydrofuran (15 ml) and added dropwise to the reaction mixture which was heated to reflux for 12 h. Ethyl acetate (100 ml) and hydrochloric acid (1 N; 100 ml) were added and the phases were separated. The aqueous phase was extracted with ethyl acetate (3 x 50 ml). The combined organic phases were washed with an aqueous solution of sodium hydrogen carbonate (saturated; 100 ml), dried over magnesium sulfate and evaporated in vacuo. The residue was chromatographed on silica (3 x 40 cm) using ethyl acetate/heptane (1:2) as eluent to afford 2.0 g of (E)-5-tert-butoxycarbonylamino-3,5-dimethylhex-2-enoic acid ethyl ester.
¹H-NMR (CDCl₃): δ 1.25 (t, 3H); 1.30 (s, 6H); 1.44 (s, 9H); 2.21 (s, 3H); 2.58 (s, 2H); 4.14 (q, 2H); 4.48 (s, 1H); 5.65 (s, 1H).

(2E)-5-tert-Butoxycarbonylamino-3,5-dimethylhex-2-enoic acid:

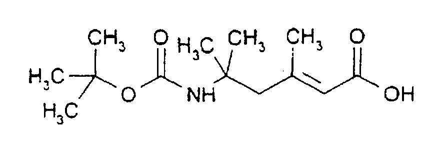

(E)-5-tert-Butoxycarbonylamino-3,5-dimethylhex-2-enoic acid ethyl ester (1.95 g; 6.83 mmol) was dissolved in 1,4-dioxane (25 ml) and water (15 ml). Lithium hydroxide (0.18 g; 7.52 mmol) was added and the reaction mixture was stirred for 12 h at room temperature. Water (150 ml) and tert-butyl methyl ether (150 ml) was added. The aqueous phase was diluted with an aqueous solution of sodium hydrogensulfate (10 %) until pH 2.5 and extracted with tert-butyl methyl ether (3 x 100 ml). The combined organic phases were dried over magnesium sulfate and evaporated in vacuo. The residue was recrystallized from heptane (20 ml) to afford 0.6 g of (2E)-5-tert-butoxycarbonylamino-3,5-dimethylhex-2-enoic acid.
¹H-NMR (CDCl₃) δ 1.29 (s, 6H); 1.44 (s, 9H); 2.23 (s, 3H); 2.62 (s, 2H); 4.45 (s, 1H); 5.66 (s, 1H).

The remaining synthesis proceeded similarly as in Example 1 to afford the title compound. (2E)-5-tert-Butoxycarbonylamino-3,5-dimethylhex-2-enoic acid was used in the last coupling step instead of (2E)-5-tert-butoxycarbonylamino-5-methylhex-2-enoic acid.
¹H-NMR (CDCl₃) (selected peaks for major rotamer) δ 1.37 (s, 6H); 2.09 (s, 2H); 2.50 (s, 3H); 2.78 (s, 3H); 4.67 (dd, 1H); 5.05 (dd, 1H); 5.89 (s, 1H).
ESMS: m/z 541.7 (M+H)⁺
HPLC (A1): rₜ: 32.40 min

### Example 3

(2E)-5-Methyl-5-(methylamino)hex-2-enoic acid N-((1R)-1-((2R)-2-benzyl-3-oxopiperazine-1-carbonyl)-2-(2-naphthyl)ethyl)-N-methylamide.

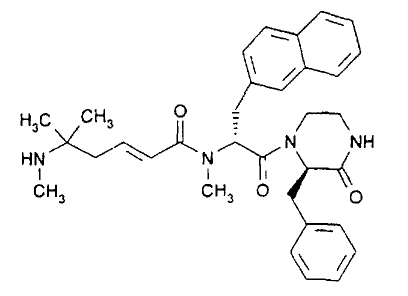

(2E)-5-(N-(tert Butoxycarbonyl)-N-methylamino)-5-methylhex-2-enoic acid.

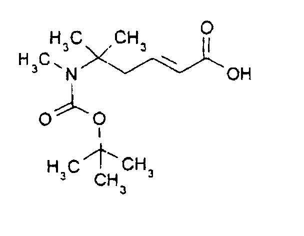

(2E)-5-(tert-Butyloxycarbonylamino)-5-methylhex-2-enoic acid (5.00 g ; 20.6 mmol) was dissolved in tetrahydrofuran (70 ml). Methyl iodide (10.3 ml; 164 mmol) was added and the solution was cooled to 0° C. Sodium hydride (60% in oil)( 2.07 g; 61.6 mmol) was added in portions and the solution was stirred at room temperature for four days. Ethyl acetate (70 ml) and water (60 ml) were added dropwise and the solvent was removed in vacuo. The crude product was dissolved in water (40 ml) and diethyl ether (40 ml). The organic phase was washed with a saturated aqueous solution of sodium hydrogencarbonate (30 ml). The aqueous phases were mixed and 5% aqueous citric acid was added to pH 3. The aqueous phase was extracted with ethyl acetate (4 x 50 ml). The combined organic extracts were washed with water (2 x 40 ml), an aqueous solution of sodium thiosulfate (5%; 40 ml), water (40 ml), dried over MgSO₄ and the solvent was removed in vacuo. The residue was dissolved in ethyl acetate (45 ml) and washed with an aqueous solution of sodium hydrogensulfate (10%; 3 x 30 ml), dried over magnesium sulfate and concentrated in vacuo to give 4.00 g of (2E)-5-(N-(tert-butoxycarbonyl)-N-methylamino)-5-methylhex-2-enoic acid.
¹H-NMR (CDCl₃) δ 1.38 (s, 6H), 1.45 (s, 9H ); 2.80 (d, 2H); 2.85 (s, 3H); 5.88 (d, 1H); 7.01 (q, 1H).

The remaining synthesis proceeded similarly as in Example 1 substituting (2E)-5-(tert-butyloxycarbonylamino)-5-methylhex-2-enoic acid with (2E)-5-(N-(tert- butoxycarbonyl)-N-methylamino)-5-methylhex-2-enoic acid in the last coupling step to afford the title compound.
¹H-NMR (CDCl₃) (selected peaks for major rotamer) δ 1.38 (s, 3H); 1.44 (s, 3H); 2.69 (s, 3H); 2.88 (s, 3H); 4.44 (dd, 1H); 4.52 (dd, 1H); 6.34 (d, 1H).
ESMS: m/z 541.9 (M+H)⁺
HPLC(A1): rₜ: 31.37 min

### Example 4

(2E)-5-Methyl-5-methylaminohex-2-enoic acid N-((1R)-1-((2R,5R)-2-benzyl-5-methyl-3-oxopiperazine-1-carbonyl)-2-(2-naphthyl)ethyl)-N-methylamide.

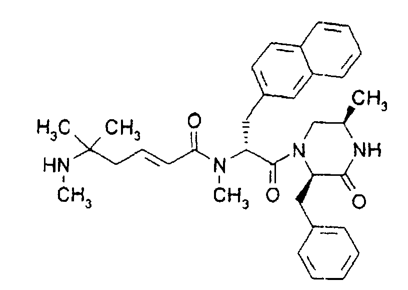

This compound was prepared using the procedure in Example 1 and 3. L-Boc-alaninal was used instead of Boc-glycinal.
¹H-NMR (CDCl₃): (selected peaks for major rotamer) δ 1.21 (d, 3H); 1.32 (s, 3H); 1.40 (s, 3H); 2.63 (s, 3H); 2.88 (s, 3H); 4.42 (dd, 1H); 4.60 (dd, 1H); 6.26 (d, 1H).
ESMS: m/z 555.9 (M+H)⁺
HPLC(A1): rₜ: 32.30 min.

### Example 5

### (2E)-5-Amino-5-methylhex-2-enoic acid N-((1R)-2-((2R)-2-benzyl-4-methyl-3-oxopiperazin-1-yl)-1-((2-naphthyl)methyl)-2-oxoethyl)-N-methylamide

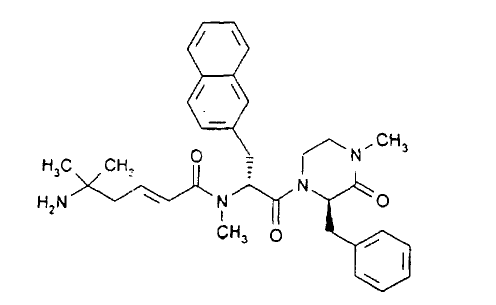

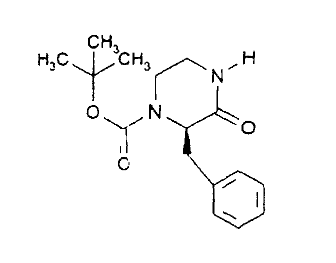

### (3R)-4-tert-Butyloxycarbonyl-3-benzylpiperazin-2-one

(3R)-3-benzylpiperazin-2-one (from Examle 1) (1.3 g) was dissolved in THF (20 ml) and aqueous sodium hydroxide (1 M, 7 ml) was added. Boc-anhydride (1.8 g) was dissolved in THF (10 ml) and added dropwise. The mixture was stirred overnight. THF was removed in vacuo. Water (10 ml) was added and the aqueous phase was extracted with ethyl acetate (2 x 50 ml). The combined organic phases were dried (magnesium sulphate) and the solvent was removed in vacuo and the residue was chromatographed on Silica using ethyl acetate as eluent to afford 750 mg of 3R-4-tert-butyloxycarbonyl-3-benzylpiperazin-2-one.
¹H-NMR (CDCl₃) (major rotamer): δ 1.31 (s, 9H); 2.85 (t, 1H); 3.10 (m, 2H; 3.25 (m, 2H); 3.42 (t, 2H); 4.80 (m, 1H); 6.40 (s, 1H); 7.12-7.35 (5 arom.H).

### (3R)-4-tert-Butyloxycarbonyl-1-methyl-3-benzylpiperazin-2-one.

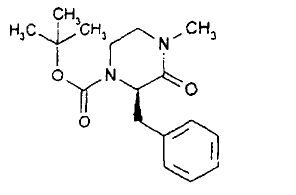

(3R)-4-tert-Butyloxycarbonyl-3-benzylpiperazin-2-one (see Example 1) (400 mg; 1.4 mmol) was added to a mixture of KOH (300 mg; 5.5 mmol) and DMSO (3 ml). Methyl iodide (400 mg 2.7 mmol) was added and the mixture was stirred for 1h. Methylene chloride (20 ml) was added and the organic phase was washed with water (5 x 10 ml). The solvent was removed in vacuo to afford 220 mg of (3R)-4-tert-butyloxycarbonyl-1-methyl-3-benzylpiperazin-2-one.
¹H-NMR (CDCl₃) δ 1.21 (s, 9H); 2.97 (s, 3H); 4.13 (dd,1H).

The remaining steps were performed similarly as in example 1 to afford the title compound.
¹H-NMR (CDCl₃) (selected peaks for major rotamer) δ 1.15 (s, 6H); 2.30 (d, 2H); 2.54 (s, 3H); 2.74 (s, 3H); 4.05 (dd, 1H); 5.72 (dd, 1H); 6.19 (d, 1H).
ESMS: m/z 541.9 (M+H)⁺ HPLC (A1): rₜ: 27.70 min

### Example 6

### (2E)-5-Methyl-5-aminohex-2-enoic acid N-((1R)-1-((2R,5R)-2-benzyl-5-methyl-3-oxopiperazine-1-carbonyl)-2-(2-naphthyl)ethyl)-N-methylamide.

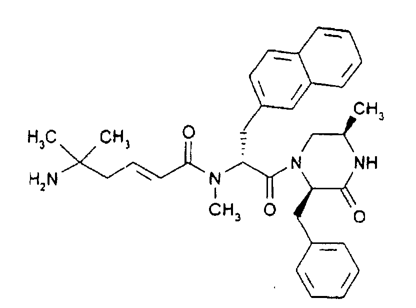

The title compound was prepared using the procedure in Example 1 and 4.
¹H-NMR (CDCl₃): δ 1.09 (d, 3H); 1.31 (s, 6H); 2.74 (s, 3H); 5.12 (dd, 1H); 6.18 (d, 1H).
ESMS: m/z 541.7 (M+H)⁺
HPLC (A1 ): rₜ: 32.12 min.

### Example 7

### (2E)-4-(1-Aminocyclobutyl)but-2-enoic acid N-methyl-N-((1R)-2-(2-naphthyl)-1-((2R)-3-oxo-2-((2-thienyl)methyl)piperazine-1-carbonyl)ethyl)amide

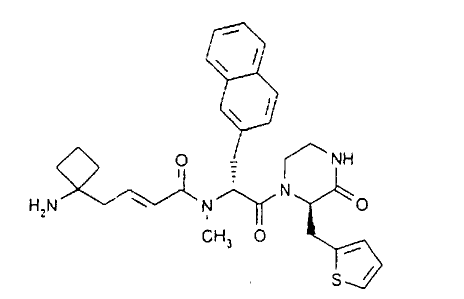

The title compound was prepared using the procedure in Schoen et al. J. Med. Chem., 1994, vol. 37, p. 897-906 substituting isobutylene with methylene cyclobutane and D-phenylalanine with D-thienylalanine.
¹H-NMR (CDCl₃): δ 3.01(s, 3H); 4.76 (dd, 1H); 5.15 (t, 1H); 6.34 (d, 1H)
ESMS: m/z 546.0 (M+H)⁺
HPLC (A1): rₜ: 31.05 min.

### Example 8

### (2E)-5-Amino-5-methylhex-2-enoic acid N-(( 1R)-2-((2R)-2-benzyl-3-oxopiperazin-1-yl)-1-((biphenyl-4-yl)methyl)-2-oxoethyl)-N-methylamide.

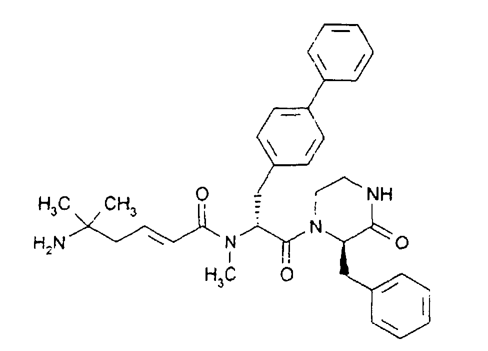

The title compound was prepared using the procedure in Example 1 using 4,4'-biphenylalanine instead of 2-napthylalanine.
¹H-NMR (CDCl₃): δ 1.18 (s, 6H); 2.77 (s, 1H); 5.31 (dd, 1H); 5.72 (dd, 1H); 6.21 (dd, 1H).
ESMS: m/z 554.1(M+H)⁺ HPLC (A1): rₜ: 34.62

### Example 9

### (2E)-4-(1-Aminocyclobutyl)but-2-enoic acid N-((1R)-2-((2R,5R)-2-benzyl-5-methyl-3-oxopiperazin-1-yl)-1-((2-naphthyl)methyl)-2-oxoethyl)-N-methylamide

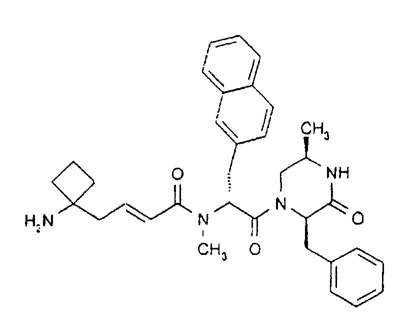

The title compound was prepared as in Schoen et al. J. Med. Chem., 1994, vol. 37, p. 897-906 substituting isobutylene with methylene cyclobutane and Boc-D-alaninal instead of Boc-glycinal.
¹H-NMR (CDCl₃): δ (selected peaks) 1.11 (d, 3H); 2.66 (s, 3H); 4.97 (dd, 1H); 5.85 (dd, 1H); 6.24 (d, 1H); 6.86 (m, 1H)
ESMS: m/z 554.0 (M+H)⁺
HPLC (A1): rₜ: 32.3 min.

### Example 10

### (2E)-5-Methyl-5-methylamino-hex-2-enoic acid N-methyl-N-((1R)-1-(2-naphthyl)methyl-2-oxo-2-((2R)-3-oxo-2-((2-thienyl)methyl)piperazin-1-yl)ethyl)amide

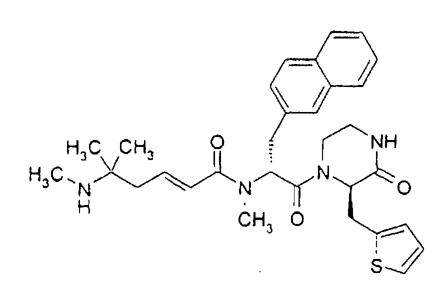

The title compound was prepared similarly as in Example 1 substituting D-phenylalanine with D-thienylalanine.
¹H-NMR (CDCl₃): δ 1.38 (s, 3H); 1.41 (s, 3H); 2.65 (s, 3H); 2.91 (s, 3H); 4.47 (dd, 1H);
ESMS: m/z 547.0 (M+H)⁺
HPLC (A1): rₜ: 31.15 min.

### Example 11

### (2E)-5-Amino-5-methylhex-2-enoic acid N-((1 R)-2-((2R)-2-benzyl-4-methane-sulfonyl-piperazin-1-yl)-1-(naphth-2-ylmethyl)-2-oxoethyl)-N-methylamide.

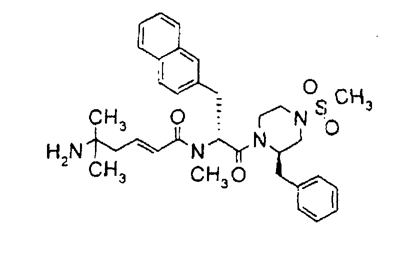

### (3R)-3,4-Dibenzylpiperazin-2-one.

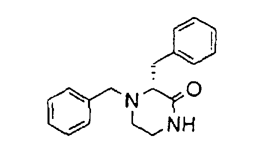

(3R)-3-Benzylpiperazin-2-one (4.1 g; 21.6 mmol, prepared as described in Example 1) was dissolved in methanol (40 ml). Benzaldehyde (2.3 g; 21.6 mmol) was added followed by 4 Å molsieves (2 g) and the mixture was stirred under an atmosphere of nitrogen for ½ h. Acetic acid (1.2 ml) was added and sodium cyanoborohydride (2.0 g; 32.4 mmol) was added portion wise during 20 minutes. The reaction mixture was stirred overnight and the filtered. From the filtrate the solvent was evaporated and the residue was dissolved into ethyl acetate (15 ml) and purified on silica gel using ethyl acetate as eluent. This afforded 2.6 g of (3R)-3,4-dibenzylpiperazin-2-one.
¹H-NMR (CDCl₃): δ 2.50 (m, 1H); 2.95 (m, 1H); 3.08 (m, 1H); 3.15-3.45 (m, 4H); 3.48 (d, 1H); 3.96 (d, 1H); 6.18 (s, 1H); 7.1-7.3 (m, 10H).

### (2R)-1,2-Dibenzylpiperazine.

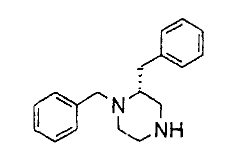

A solution of (3R)-3,4-dibenzylpiperazin-2-one (2.5 g; 9.0 mmol) in dry tetrahydrofuran (30 ml) was placed under an atmosphere of nitrogen and stirred on an ice-bath. Lithium aluminiumhydride (5.0 ml; 1.0 M in tetrahydrofuran) was added dropwise during 15 minutes. The mixture was then heated at reflux temperature for 2 h and then allowed top cool. Another portion of lithium aluminiumhydride (4.0 ml; 1.0 M in tetrahydrofuran) was added and the mixture was refluxed for 1 h. The mixture was allowed to cool and then quenched with water and a 4 N sodium hydroxide solution. Ethyl acetate (50 ml) was added and the mixture was filtered. The solvent was evaporated in vacuo to give 2.0 g of (2R)-1,2-dibenzylpiperazine.
¹H-NMR (CDCl₃): δ 2.20 (m, 1H); 2.55-2.82 (m, 7H); 3.15 (dd, 1H); 3.42 (d, 1H); 4.10(d, 1H); 7.1-7.4 (m, 10H).

### (2R)-1,2-Dibenzyl-4-methanesulfonylpiperazine.

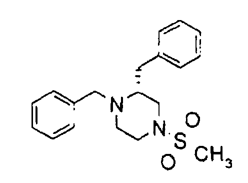

To a stirred solution of (2R)-1,2-dibenzylpiperazine (2.0 g; 7.5 mmol) in dichloromethane (30 ml), methanesulfonyl chloride (0.86 g; 7.5 mmol) was added and the mixture was stirred at ambient temperature for 1 h. Potassium carbonate (1.1 g) was added and stirring was continued for another hour. Dichloromethane (20 ml) and water (10 ml) were added and the phases were separated. The organic phase was washed with water (10 ml) and dried over magnesium sulfate. The solvent was evaporated in vacuo to give 2.2 g of (2R)-1,2-dibenzyl-4-methanesulfonylpiperazine.
¹H-NMR (CDCl₃): δ 2.58 (m, 1H); 2.72 (s, 3H); 2.85-3.10 (m, 6H); 3.27 (m, 1H); 3.45 (m, 1H); 3.72 (d, 1H); 3.95 (m, 1H); 7.1-7.4 (m, 10H).

### (3R)-3-Benzyl-1-methanesulfonylpiperazine hydrochloride.

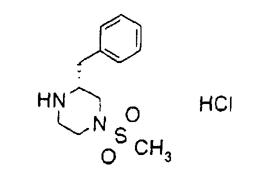

(2R)-1,2-Dibenzyl-4-methanesulfonylpiperazine (2.2 g; 6.4 mmol) was dissolved in dry 1,2-dichloroethane (20 ml) under an atmosphere of nitrogen. The mixture was placed on an ice-bath and a solution of α-chloroethyl chloroformate (1.0 g; 7.04 mmol) in dry 1,2-dichloroethane (10 ml) was added keeping the temperature between 0 and 5°C. When addition is complete stirring is continued for 15 minutes on the ice-bath. The reaction mixture is heated slowly to 90°C on an oil-bath and heated at reflux temperature for 1 h. The major part of the solvent was allowed to evaporate and the residue was allowed to cool on an ice-bath. Methanol (20 ml) was added and the mixture was heated at reflux temperature for 1 h. The mixture was allowed to cool to ambient temperature, diethyl ether (30 ml) was slowly added and the mixture was stirred for 30 minutes. The solid was isolated by filtration, washed with diethyl ether, and dried to give 0.78 g of (3R)-3-benzyl-1-methanesulfonylpiperazine hydrochloride.
M.p. 204-208°C.
¹H-NMR (DMSO-d₆): δ 2.87-3.25 (m, 8H); 3.35-3.65 (m, 4H); 7.25-7.40 (m, 5H).

### N-[(1R)-2-((2R)-2-Benzyl-4-methanesulfonyipiperazin-1-yl)-1-(naphth-2-ylmethyl)-2-oxoethyl]-N-methylcarbamic acid tert-butyl ester.

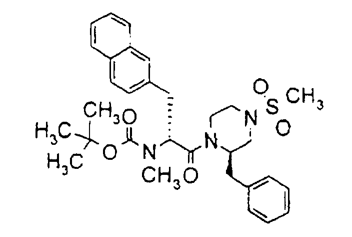

(2R)-(tert-Butoxycarbonylmethylamino)-3-(2-naphthyl)propionic acid (0.86 g; 2.6 mmol) was dissolved into dichloromethane (25 ml) and placed under an atmosphere of nitrogen. (3R)-3-Benzyl-1-methanesulfonylpiperazine hydrochloride (0.75 g; 2.6 mmol), N-methylmorpholine (0.26 g; 2.6 mmol), 1-hydroxy-7-azabenzotriazole (0.39 g; 2.9 mmol) and N-(3-dimethylaminopropyl)-N'-ethylcarbodiimide hydrochloride (0.55; 2.9 mmol) were added and the mixture was stirred overnight at ambient temperature. Dichloromethane (25 ml) was added and the mixture was washed with water (3 x 15 ml) and dried over magnesium sulfate. The solvent was evaporated in vacuo to give 1.3 g of an oily residue which was chromatographed on silica gel using a mixture of heptane and ethyl acetate (1:1) as eluent. This afforded 0.65 g of N-[(1R)-2-((2R)-2-benzyl-4-methanesulfonylpiperazin-1-yl)-1-(naphth-2-ylmethyl)-2-oxo-ethyl]-N-methylcarbamic acid tert-butyl ester as a solid.
M.p. 76-80°C.

### (2R)-1-((2R)-2-Benzyl-4-methanesulfonyl-piperazin-1-yl)-2-methylamino-3-(naphth-2-yl)-propan-1-one.

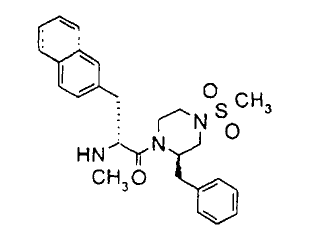

N-[( 1 R)-2-((2 R)-2-Benzyl-4-methanesulfonylpiperazin-1-yl)-(1R)-1-(naphth-2-ylmethyl)-2-oxo-ethyl]-N-methylcarbamic acid tert-butyl ester (0.63 g; 1.1 mmol) was dissolved into dichloromethane (10 ml) with stirring and placed under an atmosphere of nitrogen. Trifluoroacetic acid (10 ml) was added and the mixture was stirred for 15 minutes. The reaction mixture was diluted with dichloromethane (75 ml) and placed on an ice-bath. Saturated aqueous sodium bicarbonate solution was added and then an aqueous potassium carbonate solution until pH 10. The phases were separated and the aqueous phase was extracted with dichloromethane (50 ml). The combined organic extracts were dried over magnesium sulfate and the solvent was evaporated in vacuo. This afforded 0.51 g of (2R)-1-((2R)-2-benzyl-4-methanesulfonyl-piperazin-1-yl)-2-methylamino-3-(naphth-2-yl)propan-1-one as a solid.
M.p. 165-168°C.

### ((3E)-4-(N-((1R)-2-((2R)-2-Benzyl-4-methanesulfonyl-piperazin-1-yl)-1-(naphth-2-yl-methyl)-2-oxo-ethyl)-N-methylcarbamoyl)-1,1-dimethyl-but-3-enyl)carbamic acid tert-butyl ester.

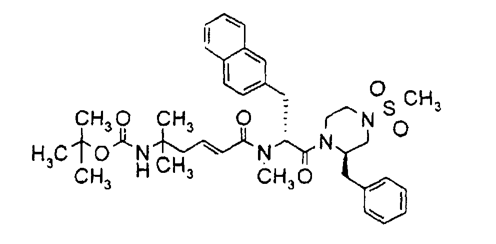

(2E)-5-(tert-Butyloxycarbonylamino)-5-methylhex-2-enoic acid (0.49 g; 2.0 mmol) was dissolved into dichloromethane (10 ml) and placed under an atmosphere of nitrogen. 1-Hydroxy-7-azabenzotriazole (2.0 mmol) and N-(3-dimethylaminopropyl)-N'-ethylcarbodiimide hydrochloride (2.0 mmol) were added and the mixture was stirred for 15 minutes. A solution of 1-((2R)-2-benzyl-4-methanesulfonyl-piperazin-1-yl)-(2R)-2-methylamino-3-naphth-2-yl-propan-1-one (0.51 g; 1.1 mmol) in dichloromethane (10 ml) was added and the mixture was stirred overnight. Dichloromethane (50 ml) was added and the mixture was washed with water (2 x 50 ml). The combined aqueous phases were extracted with dichloromethane (50 ml). The combined organic extracts were dried over magnesium sulfate and the solvent was evaporated in vacuo. This afforded 1.0 g residue which was chromatographed on silica gel using a mixture of heptane and ethyl acetate (3:7) as eluent. This afforded 0.55 g of ((3E)-4-(N-((1R)-2-((2R)-2-benzyl-4-methanesulfonyl-piperazin-1-yl)-1-(naphth-2-yl-methyl)-2-oxoethyl)-N-methylcarbamoyl)-1,1-dimethylbut-3-enyl)carbamic acid tert-butyl ester.
TLC; Rf = 0.33 (heptane/ethyl acetate = 3:7).

((3E)-4-(N-((1R)-2-((2R)-2-Benzyl-4-methanesulfonyl-piperazin-1-yl)-1-(naphth-2-ylmethyl)-2-oxoethyl)-N-methyl-carbamoyl)-1,1-dimethylbut-3-enyl)carbamic acid tert-butyl ester (0.54 g; 0.78 mmol) was dissolved in dry dichloromethane (10 ml) with stirring and trifluoroacetic acid (10 ml) was added. The mixture was stirred for 8 min. at ambient temperature and then diluted with dichloromethane (50 ml). The mixture was placed on an ice-bath and saturated aqueous sodium bicarbonate solution was added and then an aqueous potassium carbonate solution until pH 10. The phases were separated and the aqueous phase was extracted with dichloromethane (20 ml). The combined organic extracts were dried over magnesium sulfate and the solvent was evaporated in vacuo. This afforded 0.33 g of (2E)-5-amino-5-methylhex-2-enoic acid N-((1R)-2-((2R)-2-benzyl-4-methanesulfonyl-piperazin-1-yl)-1-(naphth-2-ylmethyl)-2-oxoethyl)-N-methylamide.
¹H-NMR (CDCl₃): δ 1.17 (s, 3H); 1.20 (s, 3H); 1.8-3.65 (complex m); 4.10 (d, 1H); 4.55 + 4.88 (d+m, 1H); 5.82 + 6.03 (t+dd, 1H); 6.28 (dd, 1H); 6.81 + 6.95 (dt+dt, 1H); 7.15-7.80 (m, 12H).

## Claims

1. A compound of general formula I 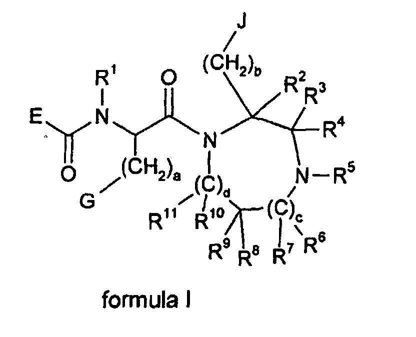 wherein
R¹ is: hydrogen; or C₁₋₆-alkyl optionally substituted with a carbocyclic or heterocyclic aryl group which is chosen among phenyl, naphthyl, pyridyl, 1-H-tetrazol-5-yl, thiazolyl, imidazolyl, indolyl, pyrimidinyl, thiadiazolyl, pyrazolyl, oxazolyl, isoxazolyl, oxadiazolyl, thienyl, quinolinyl, pyrazinyl and isothiazolyl, and which is optionally substituted with one or more C₁₋₆-alkyl, C₁₋₆-alkoxy, halogen or amino groups;
a and b are independently 1 or 2;
c and d are independently 0, 1, or 2;
c + d is 0, 1, or 2;
R², R³, R⁴, R⁶, R⁷, R⁸, R⁹, R¹⁰, and R¹¹ are independently selected from:
hydrogen;
carbocyclic or heterocyclic aryl which is chosen among phenyl, naphthyl, pyridyl, 1-H-tetrazol-5-yl, thiazolyl, imidazolyl, indolyl, pyrimidinyl, thiadiazolyl, pyrazolyl, oxazolyl, isoxazolyl, oxadiazolyl, thienyl, quinolinyl, pyrazinyl and isothiazolyl, and which is optionally substituted with halogen, amino, C₃₋₆-cycloalkyl, hydroxy, -COOR²² or -CONR²³R²⁴; and
C₁₋₆-alkyl optionally substituted with: halogen; amino; C₃₋₆-cycloalkyl; hydroxy; carbocyclic or heterocyclic aryl which is chosen among phenyl, naphthyl, pyridyl, 1-H-tetrazol-5-yl, thiazolyl, imidazolyl, indolyl, pyrimidinyl, thiadiazolyl, pyrazolyl, oxazolyl, isoxazolyl, oxadiazolyl, thienyl, quinolinyl, pyrazinyl and isothiazolyl, and which is optionally substituted with one or more C₁₋₆-alkyl, C₁₋₆-alkoxy, halogen or amino groups; -COOR²²; or -CONR²³R²⁴;
R³ and R⁴ can be taken together to form =O or =S;
R⁸ and R⁹ can be taken together to form =O or =S;
R⁵ is hydrogen, 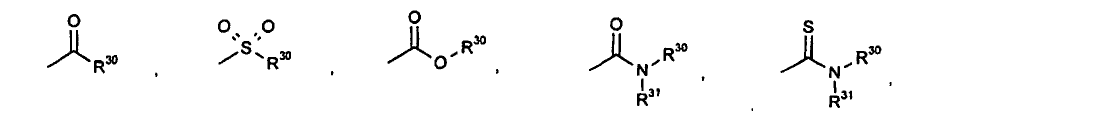 or C₁₋₆-alkyl optionally substituted with:
carbocyclic or heterocyclic aryl which is chosen among phenyl, naphthyl, pyridyl, 1-H-tetrazol-5-yl, thiazolyl, imidazolyl, indolyl, pyrimidinyl, thiadiazolyl, pyrazolyl, oxazolyl, isoxazolyl, oxadiazolyl, thienyl, quinolinyl, pyrazinyl and isothiazolyl, and which is optionally substituted with one or more C₁₋₆-alkyl, C₁₋₆-alkoxy, halogen or amino groups; hydroxy; C₃₋₆-cycloalkyl; amino; -COOR²⁵; -CONR²⁶R²⁷;
-NR'R"; 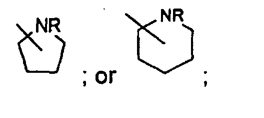 R, R' and R" are independently hydrogen or C₁₋₆-alkyl;
R³⁰ and R³¹ are C₁₋₆-alkyl optionally substituted with: carbocyclic or heterocyclic aryl which is chosen among phenyl, naphthyl, pyridyl, 1-H-tetrazol-5-yl, thiazolyl, imidazolyl, indolyl, pyrimidinyl, thiadiazolyl, pyrazolyl, oxazolyl, isoxazolyl, oxadiazolyl, thienyl, quinolinyl, pyrazinyl and isothiazolyl, and which is optionally substituted with one or more C₁₋₆-alkyl, C₁₋₆-alkoxy, halogen or amino groups; hydroxy; C₃₋₆-cycloalkyl; or amino;
R²², R²³, R²⁴, R²⁵, R²⁶, and R²⁷ are independently hydrogen or C₁₋₆-alkyl;
when R³ and R⁴ are taken together to form =O or =S, then E is 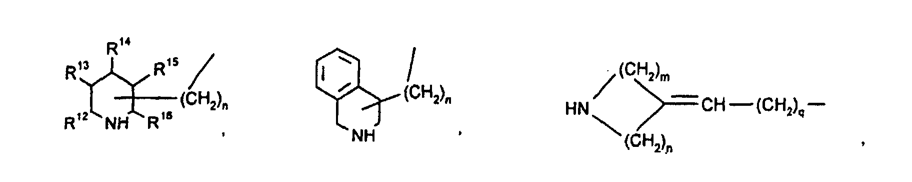 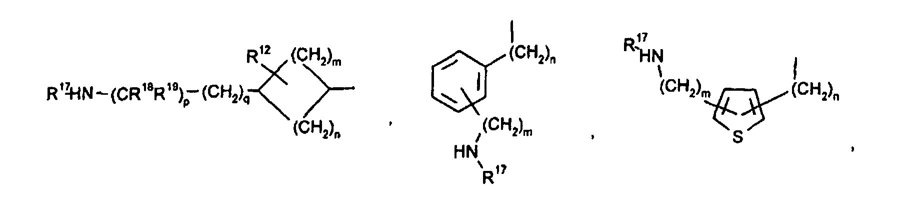 or
R¹⁷-NH―(CR¹⁸R¹⁹)ₚ―(CH₂)ₘ-M―(CHR²⁰)ₒ―(CH₂)ₙ― ;
when R³ or R⁴ is: hydrogen; carbocyclic or heterocyclic aryl which is chosen among phenyl, naphthyl, pyridyl, 1-H-tetrazol-5-yl, thiazolyl, imidazolyl, indolyl, pyrimidinyl, thiadiazolyl, pyrazolyl, oxazolyl, isoxazolyl, oxadiazolyl, thienyl, quinolinyl, pyrazinyl and isothiazolyl, and which is optionally substituted with halogen, amino, C₃₋₆-cycloalkyl, hydroxy, -COOR²² or -CONR²³R²⁴; or C₁₋₆-alkyl optionally substituted with: halogen; amino; C₃₋₆-cycloalkyl; hydroxy; carbocyclic or heterocyclic aryl which is chosen among phenyl, naphthyl, pyridyl, 1-H-tetrazol-5-yl, thiazolyl, imidazolyl, indolyl, pyrimidinyl, thiadiazolyl, pyrazolyl, oxazolyl, isoxazolyl, oxadiazolyl, thienyl, quinolinyl, pyrazinyl and isothiazolyl, and which is optionally substituted with one or more C₁₋₆-alkyl, C₁₋₆-alkoxy, halogen or amino groups; -COOR²²; or -CONR²³R²⁴;
then E is 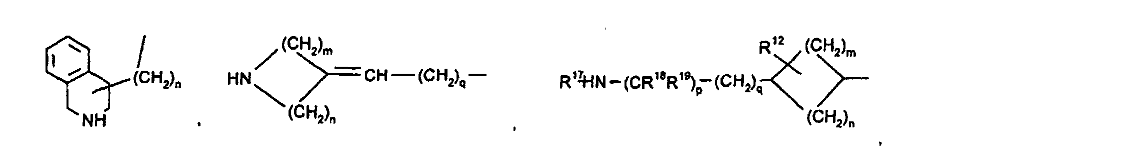 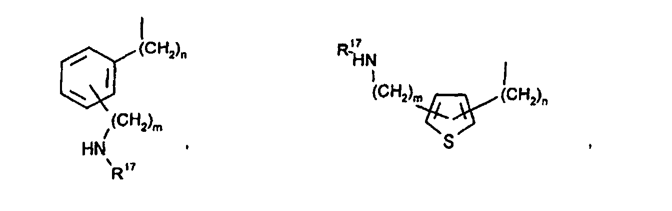 or
R¹⁷-NH―(CR¹⁸R¹⁹)ₚ―(CH₂)ₘ-Q―(CHR²⁰)ₒ―(CH₂)ₙ― ;
R¹², R¹³, R¹⁴, R¹⁵, R¹⁶, R¹⁷, R¹⁸, R¹⁹ and R²⁰ are independently selected from:
hydrogen; and
C₁₋₆-alkyl optionally substituted with: halogen; amino; hydroxyl; or carbocyclic or heterocyclic aryl which is chosen among phenyl, naphthyl, pyridyl, 1-H-tetrazol-5-yl, thiazolyl, imidazolyl, indolyl, pyrimidinyl, thiadiazolyl, pyrazolyl, oxazolyl, isoxazolyl, oxadiazolyl, thienyl, quinolinyl, pyrazinyl and isothiazolyl, and which is optionally substituted with one or more C₁₋₆-alkyl, C₁₋₆-alkoxy, halogen or amino groups;
any two of R¹⁷, R¹⁸ and R¹⁹ can independently be joined together to form an C₁₋₆-alkylene bridge;
n, m and q are independently 0, 1, 2 or 3;
o and p are independently 0 or 1;
M is -CH=CR²¹-, -O-, -S- or a valence bond;
Q is -CH=CR²¹-, -O- or -S-;
R²¹ is selected from:
hydrogen; and
C₁₋₆-alkyl optionally substituted with: halogen; amino; hydroxyl; or carbocyclic or heterocyclic aryl which is chosen among phenyl, naphthyl, pyridyl, 1-H-tetrazol-5-yl, thiazolyl, imidazolyl, indolyl, pyrimidinyl, thiadiazolyl, pyrazolyl, oxazolyl, isoxazolyl, oxadiazolyl, thienyl, quinolinyl, pyrazinyl and isothiazolyl, and which is optionally substituted with one or more C₁₋₆-alkyl, C₁₋₆-alkoxy, halogen or amino groups;
G is hydrogen, 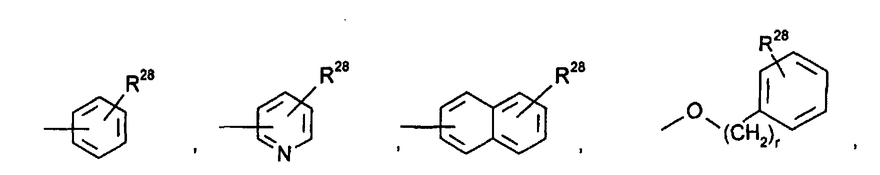 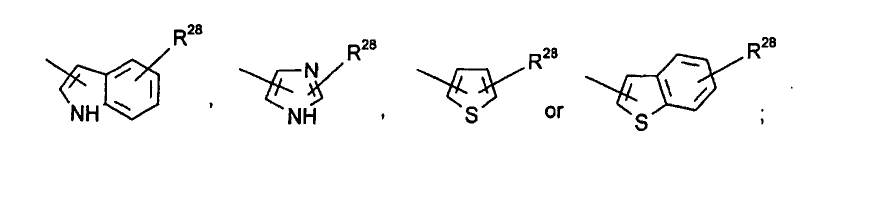 wherein R²⁸ is selected from: hydrogen; halogen; C₁₋₆-alkyl; C₁₋₆-alkoxy; and
carbocyclic or heterocyclic aryl which is chosen among phenyl, naphthyl, pyridyl, 1-H-tetrazol-5-yl, thiazolyl, imidazolyl, indolyl, pyrimidinyl, thiadiazolyl, pyrazolyl, oxazolyl, isoxazolyl, oxadiazolyl, thienyl, quinolinyl, pyrazinyl and isothiazolyl, and which is optionally substituted with one or more C₁₋₆-alkyl, C₁₋₆-alkoxy, halogen or amino groups;
r is 0, 1 or 2;
J is hydrogen, 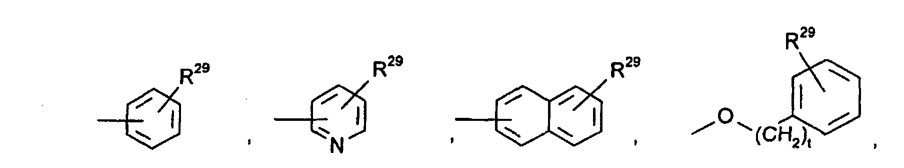 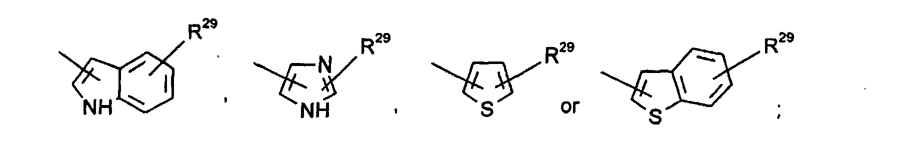 wherein R²⁹ is is selected from: hydrogen; halogen; C₁₋₆-alkyl; C₁₋₆-alkoxy; and
carbocyclic or heterocyclic aryl which is chosen among phenyl, naphthyl, pyridyl, 1-H-tetrazol-5-yl, thiazolyl, imidazolyl, indolyl, pyrimidinyl, thiadiazolyl, pyrazolyl, oxazolyl, isoxazolyl, oxadiazolyl, thienyl, quinolinyl, pyrazinyl and isothiazolyl, and which is optionally substituted with one or more C₁₋₆-alkyl, C₁₋₆-alkoxy, halogen or amino groups;
t is 0, 1 or 2;
or a pharmaceutically acceptable salt thereof.

2. A compound of the general formula II 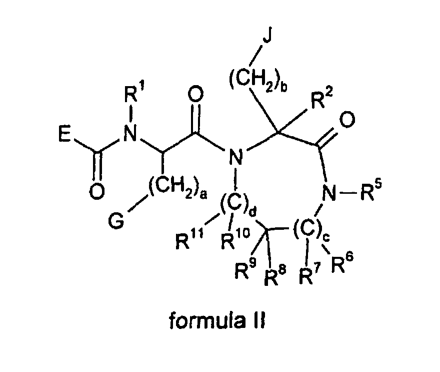 wherein
E is 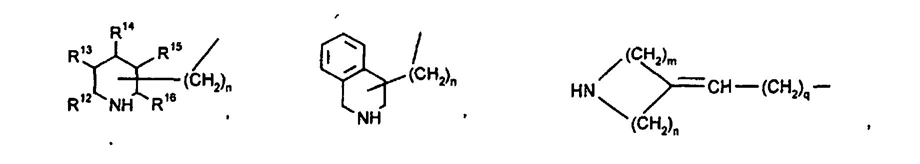 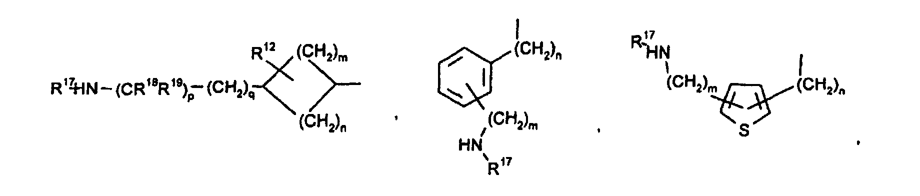 or
R¹⁷-NH―(CR¹⁸R¹⁹)ₚ―(CH₂)ₘ-M―(CHR²⁰)ₒ―CH₂)ₙ― ;
R¹², R¹³, R¹⁴, R¹⁵, R¹⁶, R¹⁷, R¹⁸, R¹⁹ and R²⁰ are independently selected from:
hydrogen; and
C₁₋₆-alkyl optionally substituted with: halogen; amino; hydroxyl; or carbocyclic or heterocyclic aryl which is chosen among phenyl, naphthyl, pyridyl, 1-H-tetrazol-5-yl, thiazolyl, imidazolyl, indolyl, pyrimidinyl, thiadiazolyl, pyrazolyl, oxazolyl, isoxazolyl, oxadiazolyl, thienyl, quinolinyl, pyrazinyl and isothiazolyl, and which is optionally substituted with one or more C₁₋₆-alkyl, C₁₋₆-alkoxy, halogen or amino groups;
any two of R¹⁷, R¹⁸ and R¹⁹ can independently be joined together to form an C₁₋₆-alkylene bridge;
n, m and q are independently 0, 1, 2 or 3;
o and p are independently 0 or 1;
M is -CH=CR²¹-, -O-, -S- or a valence bond;
R²¹ is selected from:
hydrogen; and
C₁₋₆-alkyl optionally substituted with: halogen; amino; hydroxyl; or carbocyclic or heterocyclic aryl which is chosen among phenyl, naphthyl, pyridyl, 1-H-tetrazol-5-yl, thiazolyl, imidazolyl, indolyl, pyrimidinyl, thiadiazolyl, pyrazolyl, oxazolyl, isoxazolyl, oxadiazolyl, thienyl, quinolinyl, pyrazinyl and isothiazolyl, and which is optionally substituted with one or more C₁₋₆-alkyl, C₁₋₆-alkoxy, halogen or amino groups; and
R¹, R², R⁵, R⁶, R⁷, R⁸, R⁹, R¹⁰, R¹¹, G, J, a, b, c and d are as defined in claim 1;
or a pharmaceutically acceptable salt thereof.

3. A compound of the general formula III 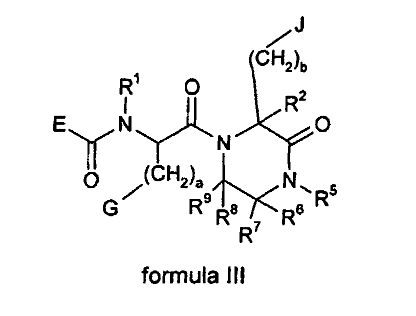 wherein E is 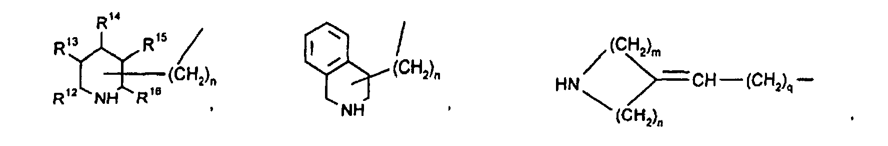 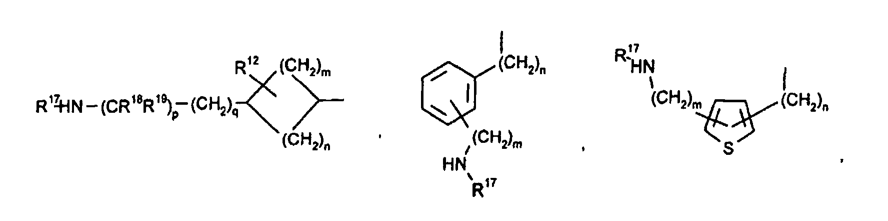 or
R¹⁷-NH―(CR¹⁸R¹⁹)ₚ―(CH₂)ₘ-M―(CHR²⁰)ₒ―(CH₂)ₙ― ;
R¹², R¹³, R¹⁴, R¹⁵, R¹⁶, R¹⁷, R¹⁸, R¹⁹ and R²⁰ are independently selected from:
hydrogen; and
C₁₋₆-alkyl optionally substituted with: halogen; amino; hydroxyl; or carbocyclic or heterocyclic aryl which is chosen among phenyl, naphthyl, pyridyl, 1-H-tetrazol-5-yl, thiazolyl, imidazolyl, indolyl, pyrimidinyl, thiadiazolyl, pyrazolyl, oxazolyl, isoxazolyl, oxadiazolyl, thienyl, quinolinyl, pyrazinyl and isothiazolyl, and which is optionally substituted with one or more C₁₋₆-alkyl, C₁₋₆-alkoxy, halogen or amino groups;
any two of R¹⁷, R¹⁸ and R¹⁹ can independently be joined together to form an C₁₋₆-alkylene bridge;
n, m and q are independently 0, 1, 2 or 3;
o and p are independently 0 or 1;
M is -CH=CR²¹-, -O-, -S- or a valence bond ;
R²¹ is selected from:
hydrogen; and
C₁₋₆-alkyl optionally substituted with: halogen; amino; hydroxyl; or carbocyclic or heterocyclic aryl which is chosen among phenyl, naphthyl, pyridyl, 1-H-tetrazol-5-yl, thiazolyl, imidazolyl, indolyl, pyrimidinyl, thiadiazolyl, pyrazolyl, oxazolyl, isoxazolyl, oxadiazolyl, thienyl, quinolinyl, pyrazinyl and isothiazolyl, and which is optionally substituted with one or more C₁₋₆-alkyl, C₁₋₆-alkoxy, halogen or amino groups;
R⁸ and R⁹ are independently selected from:
hydrogen;
carbocyclic or heterocyclic aryl which is chosen among phenyl, naphthyl, pyridyl, 1-H-tetrazol-5-yl, thiazolyl, imidazolyl, indolyl, pyrimidinyl, thiadiazolyl, pyrazolyl, oxazolyl, isoxazolyl, oxadiazolyl, thienyl, quinolinyl, pyrazinyl and isothiazolyl, and which is optionally substituted with halogen, amino, C₃₋₆-cycloalkyl, hydroxy, -COOR²² or -CONR²³R²⁴; and
C₁₋₆-alkyl optionally substituted with: halogen; amino; C₃₋₆-cycloalkyl; hydroxy; carbocyclic or heterocyclic aryl which is chosen among phenyl, naphthyl, pyridyl, 1-H-tetrazol-5-yl, thiazolyl, imidazolyl, indolyl, pyrimidinyl, thiadiazolyl, pyrazolyl, oxazolyl, isoxazolyl, oxadiazolyl, thienyl, quinolinyl, pyrazinyl and isothiazolyl, and which is optionally substituted with one or more C₁₋₆-alkyl, C₁₋₆-alkoxy, halogen or amino groups; -COOR²²; or -CONR²³R²⁴; and
G, J, R¹, R², R⁵, R⁶, R⁷, R²², R²³, R²⁴, a and b are as defined in claim 1;
or a pharmaceutically acceptable salt thereof.

4. A compound according to claim 2 or 3, wherein R⁵ is hydrogen or C₁₋₆-alkyl optionally substituted with:
carbocyclic or heterocyclic aryl which is chosen among phenyl, naphthyl, pyridyl, 1-H-tetrazol-5-yl, thiazolyl, imidazolyl, indolyl, pyrimidinyl, thiadiazolyl, pyrazolyl, oxazolyl, isoxazolyl, oxadiazolyl, thienyl, quinolinyl, pyrazinyl and isothiazolyl, and which is optionally substituted with one or more C₁₋₆-alkyl, C₁₋₆-alkoxy, halogen or amino groups; hydroxy; C₃₋₆-cycloalkyl; amino; -COOR²⁵; -CONR²⁶R²⁷;
-NR'R"; 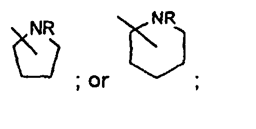 R, R' and R" are independently hydrogen or C₁₋₆-alkyl;
R²⁵, R²⁶ and R²⁷ are independently hydrogen or C₁₋₆-alkyl; and
R¹, R², R³, R⁴, R⁶, R⁷, R⁸, R⁹, R¹⁰, R¹¹, E, G, J, a, b, c and d are as defined in any of the preceding claims;
or a pharmaceutically acceptable salt thereof.

5. A compound according to any one of claims 2-4, wherein
E is 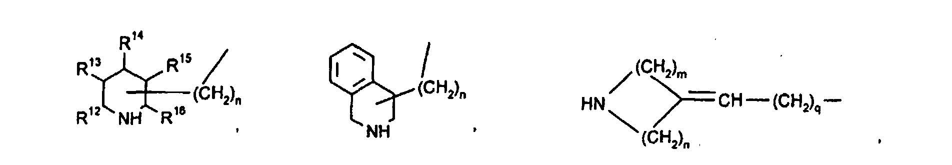 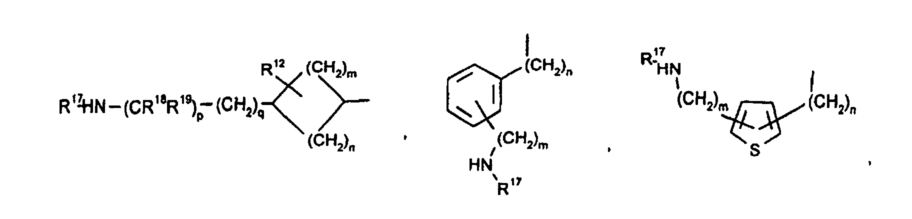 or
R¹⁷-NH―(CR¹⁸R¹⁹)ₚ―(CH₂)ₘ-M―(CHR²⁰)ₒ―(CH₂)ₙ― ;
R¹², R¹³, R¹⁴, R¹⁵, R¹⁶, R¹⁷, R¹⁸, R¹⁹ and R²⁰ are independently hydrogen or methyl;
n, m and q are independently 0, 1, 2 or 3;
o and p are independently 0 or 1;
M is -CH=CR²¹-, -O- or a valence bond ;
R²¹ is hydrogen, methyl or benzyl; and
R¹, R², R³, R⁴, R⁵, R⁶, R⁷, R⁸, R⁹, R¹⁰, R¹¹, G, J, a, b, c and d are as defined in any of the preceding claims;
or a pharmaceutically acceptable salt thereof.

6. A compound according to claim 1, wherein
R³ and R⁴ are independently selected from:
hydrogen;
carbocyclic or heterocyclic aryl which is chosen among phenyl, naphthyl, pyridyl, 1-H-tetrazol-5-yl, thiazolyl, imidazolyl, indolyl, pyrimidinyl, thiadiazolyl, pyrazolyl, oxazolyl, isoxazolyl, oxadiazolyl, thienyl, quinolinyl, pyrazinyl and isothiazolyl, and which is optionally substituted with halogen, amino, C₃₋₆-cycloalkyl, hydroxy, -COOR²² or -CONR²³R²⁴; and
C₁₋₆-alkyl optionally substituted with: halogen; amino; C₃₋₆-cycloalkyl; hydroxy; carbocyclic or heterocyclic aryl which is chosen among phenyl, naphthyl, pyridyl, 1-H-tetrazol-5-yl, thiazolyl, imidazolyl, indolyl, pyrimidinyl, thiadiazolyl, pyrazolyl, oxazolyl, isoxazolyl, oxadiazolyl, thienyl, quinolinyl, pyrazinyl and isothiazolyl, and which is optionally substituted with one or more C₁₋₆-alkyl, C₁₋₆-alkoxy, halogen or amino groups; -COOR²²; or -CONR²³R²⁴;
R²², R²³ and R²⁴ are independently hydrogen or C₁₋₆-alkyl;
E is 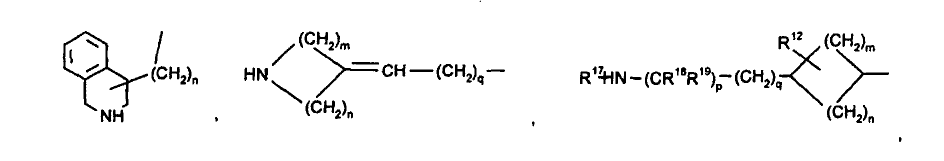 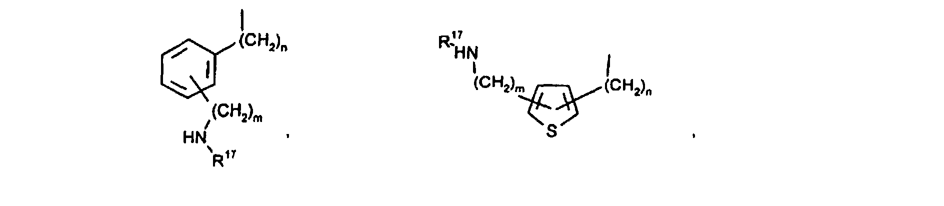 or
R¹⁷-NH―(CR¹⁸R¹⁹)ₚ―(CH₂)ₘ-Q―(CHR²⁰)ₒ―(CH₂)ₙ― ;
R¹², R¹⁷, R¹⁸, R¹⁹ and R²⁰ are independently selected from:
hydrogen; and
C₁₋₆-alkyl optionally substituted with: halogen; amino; hydroxyl; or carbocyclic or heterocyclic aryl which is chosen among phenyl, naphthyl, pyridyl, 1-H-tetrazol-5-yl, thiazolyl, imidazolyl, indolyl, pyrimidinyl, thiadiazolyl, pyrazolyl, oxazolyl, isoxazolyl, oxadiazolyl, thienyl, quinolinyl, pyrazinyl and isothiazolyl, and which is optionally substituted with one or more C₁₋₆-alkyl, C₁₋₆-alkoxy, halogen or amino groups;
any two of R¹⁷, R¹⁸ and R¹⁹ can independently be joined together to form an C₁₋₆-alkylene bridge;
n, m and q are independently 0, 1, 2 or 3;
o and p are independently 0 or 1;
Q is -CH=CR²¹-, -O- or -S-;
R²¹ is selected from:
hydrogen; and
C₁₋₆-alkyl optionally substituted with: halogen; amino; hydroxyl; or carbocyclic or heterocyclic aryl which is chosen among phenyl, naphthyl, pyridyl, 1-H-tetrazol-5-yl, thiazolyl, imidazolyl, indolyl, pyrimidinyl, thiadiazolyl, pyrazolyl, oxazolyl, isoxazolyl, oxadiazolyl, thienyl, quinolinyl, pyrazinyl and isothiazolyl, and
which is optionally substituted with one or more C₁₋₆-alkyl, C₁₋₆-alkoxy, halogen or amino groups; and
R¹, R², R⁵, R⁶, R⁷, R⁸, R⁹, R¹⁰, R¹¹, G, J, a, b, c and d are as defined in claim 1;
or a pharmaceutically acceptable salt thereof.

7. A compound of the general formula IV 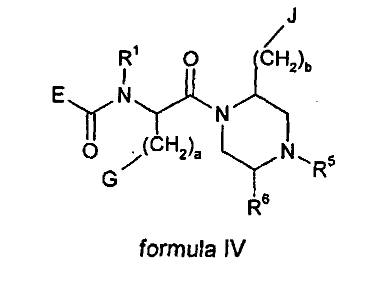 wherein E is 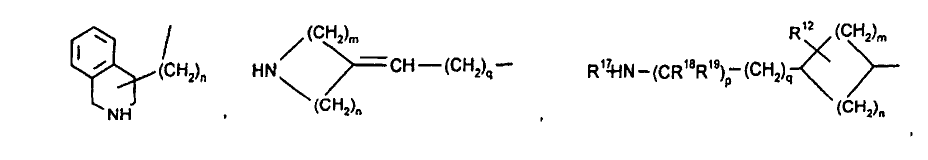 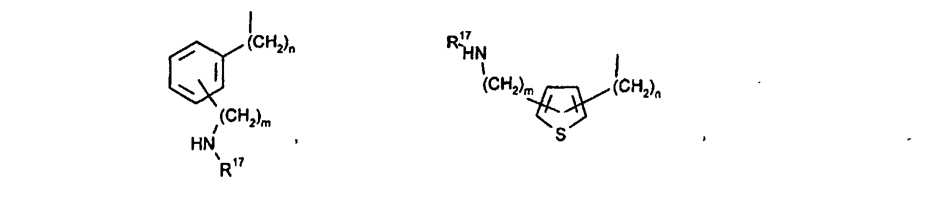 or
R¹⁷-NH-(CR¹⁸R¹⁹)ₚ―(CH₂)ₘ-Q―(CHR²⁰)ₒ―(CH₂)ₙ― ;
R¹², R¹⁷, R¹⁸, R¹⁹ and R²⁰ are independently selected from:
hydrogen; and
C₁₋₆-alkyl optionally substituted with: halogen; amino; hydroxyl; or carbocyclic or heterocyclic aryl which is chosen among phenyl, naphthyl, pyridyl, 1-H-tetrazol-5-yl, thiazolyl, imidazolyl, indolyl, pyrimidinyl, thiadiazolyl, pyrazolyl, oxazolyl, isoxazolyl, oxadiazolyl, thienyl, quinolinyl, pyrazinyl and isothiazolyl, and which is optionally substituted with one or more C₁₋₆-alkyl, C₁₋₈-alkoxy, halogen or amino groups;
any two of R¹⁷, R¹⁸ and R¹⁹ can independently be joined together to form an C₁₋₆-alkylene bridge;
n, m and q are independently 0, 1, 2 or 3;
o and p are independently 0 or 1;
Q is -CH=CR²¹-, -O- or -S-;
R²¹ is selected from:
hydrogen; and
C₁₋₆-alkyl optionally substituted with: halogen; amino; hydroxyl; or carbocyclic or heterocyclic aryl which is chosen among phenyl, naphthyl, pyridyl, 1-H-tetrazol-5-yl, thiazolyl, imidazolyl, indolyl, pyrimidinyl, thiadiazolyl, pyrazolyl, oxazolyl, isoxazolyl, oxadiazolyl, thienyl, quinolinyl, pyrazinyl and isothiazolyl, and which is optionally substituted with one or more C₁₋₆-alkyl, C₁₋₆-alkoxy, halogen or amino groups; and
R¹, R⁵, R⁶, G, J, a and b are as defined in claim 1;
or a pharmaceutically acceptable salt thereof.

8. A compound of the general formula V 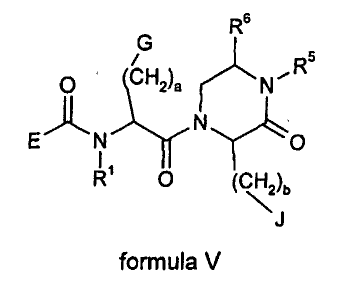 wherein E is 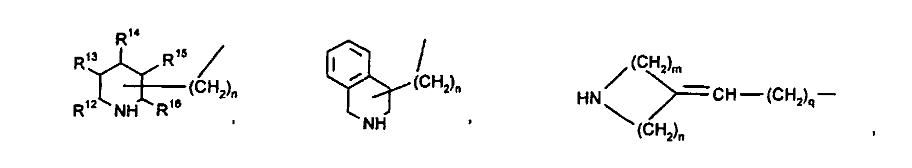 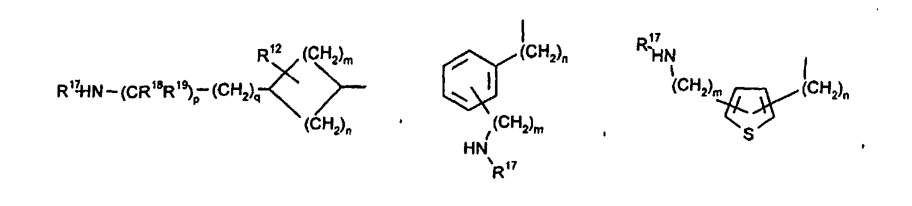 or
R¹⁷-NH―(CR¹⁸R¹⁹)ₚ―(CH₂)ₘ-M―(CHR²⁰)ₒ―(CH₂)ₙ― ;
R¹², R¹³, R¹⁴, R¹⁵, R¹⁶, R¹⁷, R¹⁸, R¹⁹ and R²⁰ are independently selected from:
hydrogen; and
C₁₋₆-alkyl optionally substituted with: halogen; amino; hydroxyl; or carbocyclic or heterocyclic aryl which is chosen among phenyl, naphthyl, pyridyl, 1-H-tetrazol-5-yl, thiazolyl, imidazolyl, indolyl, pyrimidinyl, thiadiazolyl, pyrazolyl, oxazolyl, isoxazolyl, oxadiazolyl, thienyl, quinolinyl, pyrazinyl and isothiazolyl, and which is optionally substituted with one or more C₁₋₆-alkyl, C₁₋₆-alkoxy, halogen or amino groups;
any two of R¹⁷, R¹⁸ and R¹⁹ can independently be joined together to form an C₁₋₆-alkylene bridge;
n, m and q are independently 0, 1, 2 or 3;
o and p are independently 0 or 1;
M is -CH=CR²¹-, -O-, -S- or a valence bond ;
R²¹ is selected from:
hydrogen; and
C₁₋₆-alkyl optionally substituted with: halogen; amino; hydroxyl; or carbocyclic or heterocyclic aryl which is chosen among phenyl, naphthyl, pyridyl, 1-H-tetrazol-5-yl, thiazolyl, imidazolyl, indolyl, pyrimidinyl, thiadiazolyl, pyrazolyl, oxazolyl, isoxazolyl, oxadiazolyl, thienyl, quinolinyl, pyrazinyl and isothiazolyl, and which is optionally substituted with one or more C₁₋₆-alkyl, C₁₋₆-alkoxy, halogen or amino groups;
R⁵ is hydrogen or C₁₋₆-alkyl optionally substituted with:
carbocyclic or heterocyclic aryl which is chosen among phenyl, naphthyl, pyridyl, 1-H-tetrazol-5-yl, thiazolyl, imidazolyl, indolyl, pyrimidinyl, thiadiazolyl, pyrazolyl, oxazolyl, isoxazolyl, oxadiazolyl, thienyl, quinolinyl, pyrazinyl and isothiazolyl, and which is optionally substituted with one or more C₁₋₆-alkyl, C₁₋₆-alkoxy, halogen or amino groups; hydroxy; C₃₋₈-cycloalkyl; amino; -COOR²⁵; -CONR²⁶R²⁷;
-NR'R"; 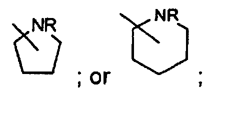 R, R' and R" are independently hydrogen or C₁₋₆-alkyl;
R²⁵, R²⁶, and R²⁷ are independently hydrogen or C₁₋₆alkyl; and
G, J, a, b, R¹ and R⁶ are as defined in claim 1;
or a pharmaceutically acceptable salt thereof.

9. A compound according to any one of claims 1-8, wherein
R¹ is hydrogen or methyl;
or a pharmaceutically acceptable salt thereof.

10. A compound according to any one of the preceding claims, selected from the group consisting of:
(2E)-5-amino-5-methylhex-2-enoic acid N-((1R)-1-((2R)-2-benzyl-3-oxopiperazine-1-carbonyl)-2-(2-naphthyl)ethyl)-N-methylamide,
(2E)-5-amino-5-methylhex-2-enoic acid N-((1R)-1-((2R)-2-benzyl-4-(cyclopropylmethyl)-3-oxopiperazine-1-carbonyl)-2-(2-naphthyl)ethyl)-N-methylamide,
(2E)-5-amino-5-methylhex-2-enoic acid N-((1R)-1-((2R,5R)-2-benzyl-5-(hydroxymethyl)-3-oxopiperazine-1-carbonyl)-2-(2-naphthyl)ethyl)-N-methylamide,
(2E)-5-amino-5-methylhex-2-enoic acid N-((1R)-1 -((2R)-2-benzyl-3-oxo-1,4-diazepane-1-carbonyl)-2-(2-naphthyl)ethyl)-N-methylamide,
piperidine-4-carboxylic acid N-methyl-N-((1R)-2-(2-naphthyl)-1-((2R)-2-((2-napthyl)methyl)-3-oxopiperazine-1-carbonyl)ethyl)amide,
(2E)-5-amino-5-methylhex-2-enoic acid N-((1R)-1-((2R)-2-benzyl-4-(dimethylcarbamoylmethyl)-3-oxopiperazine-1-carbonyl)-2-(2-naphthyl)ethyl)-N-methylamide,
(2E)-5-methyl-5-(methylamino)hex-2-enoic acid N-((1R)-1-((2R)-2-benzyl-3-oxopiperazine-1-carbonyl)-2-(2-naphthyl)ethyl)-N-methylamide,
(2E)-5-amino-5-methylhex-2-enoic acid N-((1 R)-1-((2R,6S)-2-benzyl-6-methyl-3-oxopiperazine-1-carbonyl)-2-(2-naphthyl)ethyl)-N-methylamide,
(2E)-5-amino-5-methylhex-2-enoic acid N-((1R)-1-((2R)-2-benzyl-3-oxopiperazine-1-carbonyl)-2-(biphenyl-4-yl)ethyl)-N-methylamide,
(2E)-5-Amino-5-methylhex-2-enoic acid N-((1R)-1 -((2R)-2-benzyl-4-methanesulfonylpiperazine-1-carbonyl)-2-(2-naphthyl)ethyl)-N-methylamide,
(2E)-5-amino-5-methylhex-2-enoic acid N-((1R)-1-((2R)-2-benzyl-4-methanesulfonylpiperazine-1-carbonyl)-2-benzyloxyethyl)-N-methylamide,
(2E)-5-Amino-3,5-dlmethylhex-2-enoic acid N-((1R)-1-((2R)-2-benzyl-3-oxopiperazine-1-carbonyl)-2-(2-naphthyl)ethyl)-N-methylamide,
(2E)-5-Methyl-5-methylaminohex-2-enoic acid N-((1R)-1-((2R,5R)-2-benzyl-5-methyl-3-oxopiperazine-1-carbonyl)-2-(2-naphthyl)ethyl)-N-methylamide,
(2E)-5-Amino-5-methylhex-2-enoic acid N-((1R)-2-((2R)-2-benzyl-4-methyl-3-oxopiperazin-1-yl)-1-((2-naphthyl)methyl)-2-oxoethyl)-N-methylamide,
(2E)-5-Methyl-5-aminohex-2-enoic acid N-((1 R)-1-((2R,5R)-2-benzyl-5-methyl-3-oxo piperazine-1 -carbonyl)-2-(2-naphthyl)ethyl)-N-methylamide,
(2E)-4-(1-Amlnocyclobutyl)but-2-enoic acid N-methyl-N-((1 R)-2-(2-naphthyl)-1-((2R)-3-oxo-2-((2-thienyl)methyl)piperazine-1-carbonyl)ethyl)amide,
(2E)-4-(1-Aminocyclobutyl)but-2-enoic acid N-((1 R)-2-((2R,5R)-2-benzyl-5-methyl-3-oxopiperazin-1-yl)-1-((2-naphthyl)methyl)-2-oxoethyl)-N-methylamide,
(2E)-5-Methyl-5-methylamino-hex-2-enoic acid N-methyl-N-((1R)-1-(2-naphthyl)methyl-2-oxo-2-((2R)-3-oxo-2-((2-thienyl)methyl)piperazin-1-yl)ethyl)amide and
(2E)-5-Amino-5-methyl-hex-2-enoic acid N-((1R)-2-((2R)-2-benzyl-4-methanesulfonylpiperazin-1-yl)-1-(naphth-2-ylmethyl)-2-oxo-ethyl)-N-methylamide;
or a pharmaceutically acceptable salt thereof.

11. A pharmaceutical composition comprising, as an active ingredient, a compound according to any one of claims 1-10 or a pharmaceutically acceptable salt thereof together with a pharmaceutically acceptable carrier or diluent.

12. The composition according to claim 11 in unit dosage form, comprising from about 10 to about 200 mg of a compound according to any one of claims 1-10 or a pharmaceutically acceptable salt thereof.

13. A pharmaceutical composition for stimulating the release of growth hormone from the pituitary of a human or animal, the composition comprising, as an active ingredient, a compound according to any one of claims 1-10 or a pharmaceutically acceptable salt thereof together with a pharmaceutically acceptable carrier or diluent.

14. A pharmaceutical composition for administration to animals to increase their rate and extent of growth, to increase their milk or wool production, or for the treatment of ailments, the composition comprising, as an active ingredient, a compound according to any one of claims 1-10 or a pharmaceutically acceptable salt thereof together with a pharmaceutically acceptable carrier or diluent.

15. A pharmaceutical composition according to any one of claims 11-14 for oral, nasal, transdermal, pulmonary or parenteral administration.

16. Use of a compound according to any one of claims 1-10 or a pharmaceutically acceptable salt thereof for the preparation of a medicament.

17. Use of a compound according to any one of claims 1-10 or a pharmaceutically acceptable salt thereof for the preparation of a medicament for stimulating the release of growth hormone from the pituitary of a human or an animal.

18. Use of a compound according to any one of claims 1-10 or a pharmaceutically acceptable salt thereof for the preparation of a medicament for administration to animals to increase their rate and extent of growth, to increase their milk or wool production, or for the treatment of ailments.

19. Use of a compound according to any one of claims 1-10 or a pharmaceutically acceptable salt thereof for the preparation of a medicament for: stimulation of growth hormone release in the elderly; prevention of catabolic side effects of glucocorticoids; prevention and treatment of osteoporosis; stimulation of the immune system; acceleration of wound healing; accelerating bone fracture repair; treatment of growth retardation; treatment of renal failure or insufficiency resulting from growth retardation; treatment of physiological short stature, including treatment of growth hormone deficient children and treatment of short stature associated with chronic illness; treatment of obesity and growth retardation associated with obesity; treatment of growth retardation associated with the Prader-Willi syndrome and with Turner's syndrome; accelerating the recovery of, and reducing hospitalization of, burn patients; treatment of intrauterine growth retardation, skeletal dysplasia, hypercortisolism and Cushing's syndrome; induction of pulsatile growth hormone release; replacement of growth hormone in stressed patients, treatment of osteochondrodysplasias, Noonan's syndrome, schizophrenia, depressions, Alzheimer's disease, delayed wound healing and psychosocial deprivation; treatment of pulmonary dysfunction and ventilator dependency; attenuation of protein catabolic responses after major surgery; reduction of cachexia and protein loss due to chronic illness such as cancer or AIDS; treatment of hyperinsulinemia, including nesidioblastosis; adjuvant treatment for ovulation induction; stimulation of thymic development and prevention of the age-related decline of thymic function; treatment of immunosuppressed patients; improvement of muscle strength and mobility; maintenance of skin thickness; metabolic homeostasis; renal homeostasis in the frail elderly; stimulation of osteoblasts; bone remodelling and cartilage growth; stimulation of the immune system in companion animals and treatment of disorder of aging in companion animals; growth promotion in livestock; or stimulation of wool growth in sheep.

## Patentansprüche

1. Verbindung der allgemeinen Formel I 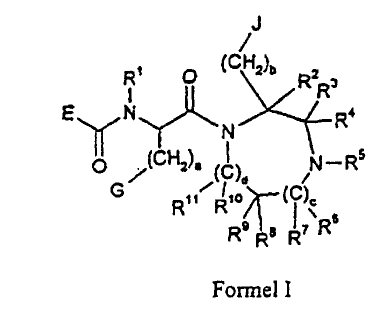 worin
R¹ ist: Wasserstoff; oder C₁₋₆-Alkyl, wahlweise substituiert mit einer carbocyklischen oder heterocyklischen Arylgruppe, welche ausgewählt ist unter Phenyl, Naphthyl, Pyridyl, 1-H-Tetrazol-5-yl, Thiazolyl, Imidazolyl, Indolyl, Pyrimidinyl, Thiadiazolyl, Pyrazolyl, Oxazolyl, Isoxazolyl, Oxadiazolyl, Thienyl, Chinolinyl, Pyrazinyl und Isothiazolyl, und welche wahlweise substituiert ist mit einer oder mehreren C₁₋₆-Alkyl-, C₁₋₆-Alkoxy-, Halogen- oder Aminogruppen;
a und b unabhängig 1 oder 2 sind;
c und d unabhängig 0, 1 oder 2 sind;
c + d 0, 1 oder 2 ist;
R², R³, R⁴, R⁶, R⁷, R⁸, R⁹, R¹⁰ und R¹¹ unabhängig ausgewählt sind aus Wasserstoff;
carbocyklischem oder heterocyklischem Aryl, welches ausgewählt ist unter Phenyl, Naphthyl, Pyridyl, 1-H-Tetrazol-5-yl, Thiazolyl, Imidazolyl, Indolyl, Pyrimidinyl, Thiadiazolyl, Pyrazolyl, Oxazolyl, Isoxazolyl, Oxadiazolyl, Thienyl, Chinolinyl, Pyrazinyl und Isothiazolyl, und welches wahlweise substituiert ist mit Halogen, Amino, C₃₋₆-Cykloalkyl, Hydroxy, -COOR²² oder -CONR²³R²⁴; und
C₁₋₆-Alkyl, wahlweise substituiert mit: Halogen; Amino; C₃₋₆-Cykloalkyl; Hydroxy; carbocyklischem oder heterocyklischem Aryl, welches ausgewählt ist unter Phenyl, Naphthyl, Pyridyl, 1-H-Tetrazol-5-yl, Thiazolyl, Imidazolyl, Indolyl, Pyrimidinyl, Thiadiazolyl, Pyrazolyl, Oxazolyl, Isoxazolyl, Oxadiazolyl, Thienyl, Chinolinyl, Pyrazinyl und Isothiazolyl, und welches wahlweise substituiert ist mit einer oder mehreren C₁₋₆-Alkyl-, C₁₋₆-Alkoxy-, Halogen- oder Aminogruppen; -COOR²²; oder -CONR²³R²⁴;
R³ und R⁴ zusammengenommen werden können, um =O oder =S zu bilden;
R⁸ und R⁹ zusammengenommen werden können, um =O oder =S zu bilden;
R⁵ Wasserstoff ist, 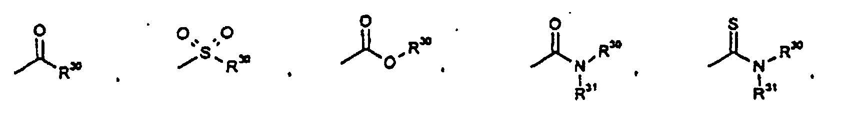 oder C₁₋₆-Alkyl, wahlweise substituiert mit:
carbocyklischem oder heterocyklischem Aryl, welches ausgewählt ist unter Phenyl, Naphthyl, Pyridyl, 1-H-Tetrazol-5-yl, Thiazolyl, Imidazolyl, Indolyl, Pyrimidinyl, Thiadiazolyl, Pyrazolyl, Oxazolyl, Isoxazolyl, Oxadiazolyl, Thienyl, Chinolinyl, Pyrazinyl und Isothiazolyl, und welches wahlweise substituiert ist mit einer oder mehreren C₁₋₆-Alkyl-, C₁₋₆-Alkoxy-, Halogen- oder Aminogruppen; Hydroxy; C₃₋₆-Cykloalkyl; Amino; -COOR²⁵; -CONR²⁶R²⁷;
-NR'R"; 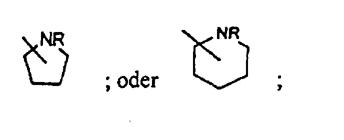 R, R' und R" unabhängig Wasserstoff oder C₁₋₆-Alkyl sind;
R³⁰ und R³¹ C₁₋₆-Alkyl sind, wahlweise substituiert mit: carbocyklischem oder heterocyklischem Aryl, welches ausgewählt ist unter Phenyl, Naphthyl, Pyridyl, 1-H-Tetrazol-5-yl, Thiazolyl, Imidazolyl, Indolyl, Pyrimidinyl, Thiadiazolyl, Pyrazolyl, Oxazolyl, Isoxazolyl, Oxadiazolyl, Thienyl, Chinolinyl, Pyrazinyl und Isothiazolyl, und welches wahlweise substituiert ist mit einer oder mehreren C₁₋₆-Alkyl-, C₁₋₆-Alkoxy-, Halogen- oder Aminogruppen; Hydroxy; C₃₋₆-Cykloalkyl; oder Amino;
R²², R²³, R²⁴, R²⁵, R²⁶ und R²⁷ unabhängig Wasserstoff oder C₁₋₆-Alkyl sind;
wenn R³ und R⁴ zusammengenommen werden, um =O oder =S zu bilden, E dann ist 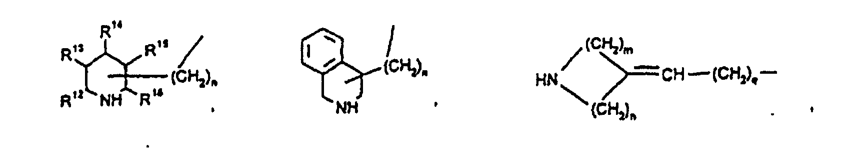 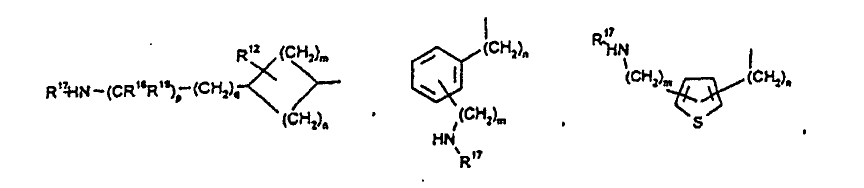 oder
R¹⁷-NH―(CR¹⁸R¹⁹)ₚ―(CH₂)ₘ-M―(CHR²⁰)ₒ―(CH₂)ₙ― ;
wenn R³ oder R⁴ ist: Wasserstoff; carbocyklisches oder heterocyklisches Aryl, welches ausgewählt ist unter Phenyl, Naphthyl, Pyridyl, 1-H-Tetrazol-5-yl, Thiazolyl, Imidazolyl, Indolyl, Pyrimidinyl, Thiadiazolyl, Pyrazolyl, Oxazolyl, Isoxazolyl, Oxadiazolyl, Thienyl, Chinolinyl, Pyrazinyl und Isothiazolyl, und welches wahlweise substituiert ist mit Halogen; Amino; C₃₋₆-Cykloalkyl; Hydroxy; -COOR²² oder -CONR²³R²⁴; oder C₁₋₆-Alkyl, wahlweise substituiert mit: Halogen; Amino; C₃₋₆-Cykloalkyl; Hydroxy; carbocyklischem oder heterocyklischem Aryl, welches ausgewählt ist unter Phenyl, Naphthyl, Pyridyl, 1-H-Tetrazol-5-yl, Thiazolyl, Imidazolyl, Indolyl, Pyrimidinyl, Thiadiazolyl, Pyrazolyl, Oxazolyl, Isoxazolyl, Oxadiazolyl, Thienyl, Chinolinyl, Pyrazinyl und Isothiazolyl, und welches wahlweise substituiert ist mit einer oder mehreren C₁₋₆-Alkyl-, C₁₋₆-Alkoxy-, Halogen- oder Aminogruppen; -COOR²²; oder -CONR²³R²⁴;
E dann ist 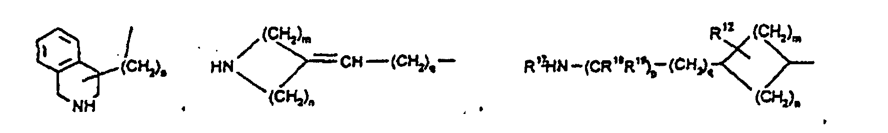 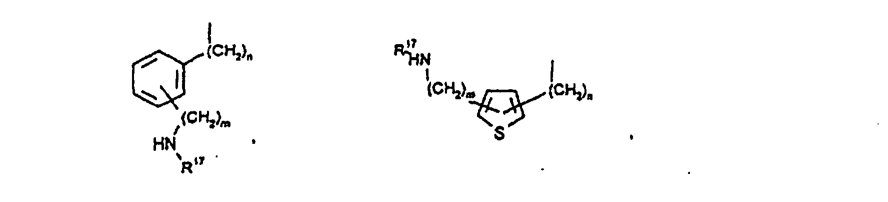 oder
R¹⁷-NH―(CR¹⁸R¹⁹)ₚ―(CH₂)ₘ-Q―(CHR²⁰)ₒ―(CH₂)ₙ― ;
R¹², R¹³, R¹⁴, R¹⁵, R¹⁶, R¹⁷, R¹⁸, R¹⁹ und R²⁰ unabhängig voneinander ausgewählt sind aus:
Wasserstoff; und
C₁₋₆-Alkyl, wahlweise substituiert mit: Halogen; Amino; Hydroxyl; oder carbocyklischem oder heterocyklischem Aryl, welches ausgewählt ist unter Phenyl, Naphthyl, Pyridyl, 1-H-Tetrazol-5-yl, Thiazolyl, Imidazolyl, Indolyl, Pyrimidinyl, Thiadiazolyl, Pyrazolyl, Oxazolyl, Isoxazolyl, Oxadiazolyl, Thienyl, Chinolinyl, Pyrazinyl und Isothiazolyl, und welches wahlweise substituiert ist mit einer oder mehreren C₁₋₆-Alkyl-, C₁₋₆-Alkoxy-, Halogen- oder Aminogruppen;
beliebige zwei von R¹⁷, R¹⁸ und R¹⁹ unabhängig verbunden sein können, um eine C₁₋₆-Alkylenbrücke zu bilden;
n, m und q unabhängig 0, 1, 2 oder 3 sind;
o und p unabhängig 0 oder 1 sind;
M ist -CH=CH²¹-, -O-, -S- oder eine Valenzbindung;
Q ist -CH=CR²¹-, -O- oder -S-;
R²¹ ausgewählt ist aus:
Wasserstoff; und
C₁₋₆-Alkyl, wahlweise substituiert mit: Halogen; Amino; Hydroxyl; oder carbocyklischem oder heterocyklischem Aryl, welches ausgewählt ist unter Phenyl, Naphthyl, Pyridyl, 1-H-Tetrazol-5-yl, Thiazolyl, Imidazolyl, Indolyl, Pyrimidinyl, Thiadiazolyl, Pyrazolyl, Oxazolyl, Isoxazolyl, Oxadiazolyl, Thienyl, Chinolinyl, Pyrazinyl und Isothiazolyl, und welches wahlweise substituiert ist mit einer oder mehreren C₁₋₆-Alkyl-, C₁₋₆-Alkoxy-, Halogen- oder Aminogruppen;
G Wasserstoff ist. 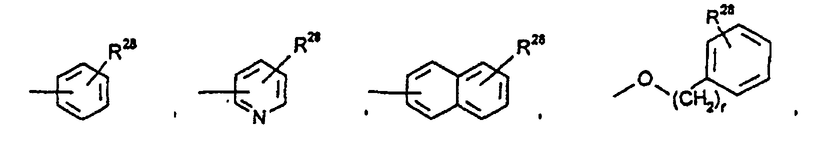 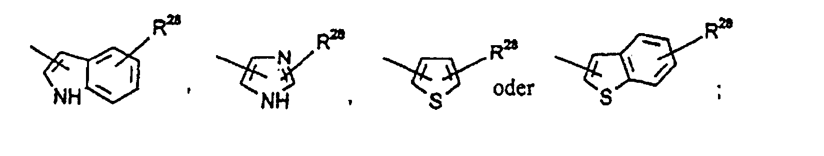 worin R²⁸ ausgewählt ist aus: Wasserstoff; Halogen; C₁₋₆-Alkyl; C₁₋₆-Alkoxy; und carbocyklischem oder heterocyklischem Aryl, welches ausgewählt ist unter Phenyl, Naphthyl, Pyridyl, 1-H-Tetrazol-5-yl, Thiazolyl, Imidazolyl, Indolyl, Pyrimidinyl, Thiadiazolyl, Pyrazolyl, Oxazolyl, Isoxazolyl, Oxadiazolyl, Thienyl, Chinolinyl, Pyrazinyl und Isothiazolyl, und welches wahlweise substituiert ist mit einer oder mehreren C₁₋₆-Alkyl-, C₁₋₆-Alkoxy-, Halogen- oder Aminogruppen;
r 0, 1 oder 2 ist;
J Wasserstoff ist, 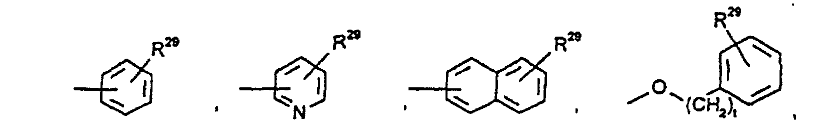 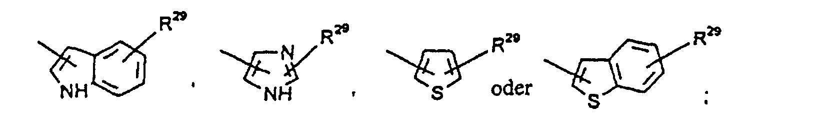 worin R²⁹ ausgewählt ist aus: Wasserstoff; Halogen; C₁₋₆-Alkyl; C₁₋₆-Alkoxy; und carbocyklischem oder heterocyklischem Aryl, welches ausgewählt ist unter Phenyl, Naphthyl, Pyridyl, 1-H-Tetrazol-5-yl, Thiazolyl, Imidazolyl, Indolyl, Pyrimidinyl, Thiadiazolyl, Pyrazolyl, Oxazolyl, Isoxazolyl, Oxadiazolyl, Thienyl, Chinolinyl, Pyrazinyl und Isothiazolyl, und welches wahlweise substituiert ist mit einer oder mehreren C₁₋₆-Alkyl-, C₁₋₆-Alkoxy-, Halogen- oder Aminogruppen;
t 0, 1 oder 2 ist;
oder ein pharmazeutisch geeignetes Salz davon.

2. Verbindung der allgemeinen Formel II 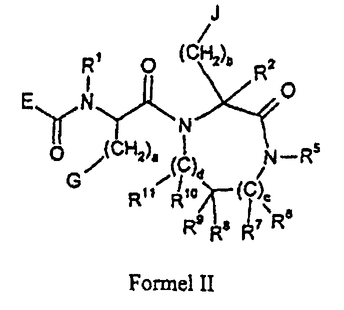 worin
E ist 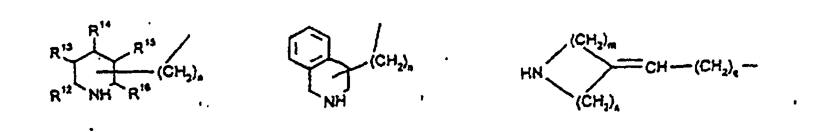 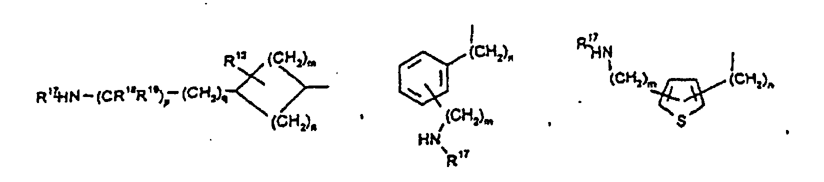 oder
R¹⁷-NH―(CR¹⁸R¹⁸)ₚ―(CH₂)ₘ-M―(CHR²⁰)ₒ―(CH₂)ₙ― ;
R¹², R¹³, R¹⁴, R¹⁵, R¹⁶, R¹⁷, R¹⁸, R¹⁹ und R²⁰ unabhängig ausgewählt sind aus:
Wasserstoff; und
C₁₋₆-Alkyl, wahlweise substituiert mit: Halogen; Amino; Hydroxyl; oder carbocyklischem oder heterocyklischem Aryl, welches ausgewählt ist unter Phenyl, Naphthyl, Pyridyl, 1-H-Tetrazol-5-yl, Thiazolyl, Imidazolyl, Indolyl, Pyrimidinyl, Thiadiazolyl, Pyrazolyl, Oxazolyl, Isoxazolyl, Oxadiazolyl, Thienyl, Chinolinyl, Pyrazinyl und Isothiazolyl, und welches wahlweise substituiert ist mit einer oder mehreren C₁₋₆-Alkyl-, C₁₋₆-Alkoxy-, Halogen- oder Aminogruppen;
beliebige zwei von R¹⁷, R¹⁸ und R¹⁹ unabhängig miteinander verbunden sein können, um eine C₁₋₆-Alkylenbrücke zu bilden;
n, m und q unabhängig 0, 1, 2 oder 3 sind;
o und p unabhängig 0 oder 1 sind;
M ist -CH=CR²¹-, -O-, -S- oder eine Valenzbindung;
R²¹ ausgewählt ist aus:
Wasserstoff; und
C₁₋₆-Alkyl, wahlweise substituiert mit: Halogen; Amino; Hydroxyl; oder carbocyklischem oder heterocyklischem Aryl, welches ausgewählt ist unter Phenyl, Naphthyl, Pyridyl, 1-H-Tetrazol-5-yl, Thiazolyl, Imidazolyl, Indolyl, Pyrimidinyl, Thiadiazolyl, Pyrazolyl, Oxazolyl, Isoxazolyl, Oxadiazolyl, Thienyl, Chinolinyl, Pyrazinyl und Isothiazolyl, und welches wahlweise substituiert ist mit einer oder mehreren C₁₋₆-Alkyl-, C₁₋₆-Alkoxy-, Halogen- oder Aminogruppen; und
R¹, R², R⁵, R⁶, R⁷, R⁸, R⁹, R¹⁰, R¹¹, G, J, a, b, c und d wie in Anspruch 1 definiert sind;
oder ein pharmazeutisch geeignetes Salz davon.

3. Verbindung der allgemeinen Formel III 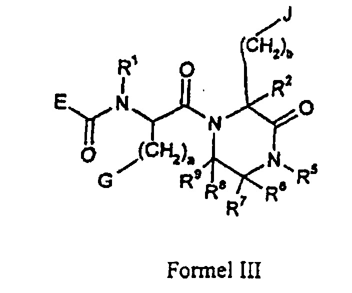 worin E ist 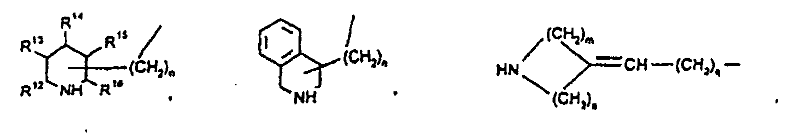 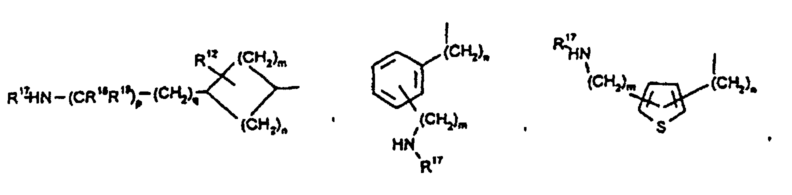 oder
R¹⁷-NH―(CR¹⁸R¹⁸)ₚ―(CH₂)ₘ-M―(CHR²⁰)ₒ―(CH₂)ₙ― ;
R¹², R¹³, R¹⁴, R¹⁵, R¹⁶, R¹⁷, R¹⁸, R¹⁹ und R²⁰ unabhängig ausgewählt sind aus:
Wasserstoff; und
C₁₋₆-Alkyl, wahlweise substituiert mit: Halogen; Amino; Hydroxyl; oder carbocyklischem oder heterocyklischem Aryl, welches ausgewählt ist unter Phenyl, Naphthyl, Pyridyl, 1-H-Tetrazol-5-yl, Thiazolyl, Imidazolyl, Indolyl, Pyrimidinyl, Thiadiazolyl, Pyrazolyl, Oxazolyl, Isoxazolyl, Oxadiazolyl, Thienyl, Chinolinyl, Pyrazinyl und Isothiazolyl, und welches wahlweise substituiert ist mit einer oder mehreren C₁₋₆-Alkyl-, C₁₋₆-Alkoxy-, Halogen- oder Aminogruppen; beliebige zwei von R¹⁷, R¹⁸ und R¹⁹ unabhängig miteinander verbunden sein können, um eine C₁₋₆-Alkylenbrücke zu bilden;
n, m und q unabhängig 0, 1, 2 oder 3 sind;
o und p unabhängig 0 oder 1 sind;
M ist -CH=CR²¹-, -O-, -S- oder eine Valenzbindung;
R²¹ ausgewählt ist aus:
Wasserstoff; und
C₁₋₆-Alkyl, wahlweise substituiert mit: Halogen; Amino; Hydroxyl; oder carbocyklischem oder heterocyklischem Aryl, welches ausgewählt ist unter Phenyl, Naphthyl, Pyridyl, 1-H-Tetrazol-5-yl, Thiazolyl, Imidazolyl, Indolyl, Pyrimidinyl, Thiadiazolyl, Pyrazolyl, Oxazolyl, Isoxazolyl, Oxadiazolyl, Thienyl, Chinolinyl, Pyrazinyl und Isothiazolyl, und welches wahlweise substituiert ist mit einer oder mehreren C₁₋₆-Alkyl-, C₁₋₆-Alkoxy-, Halogen- oder Aminogruppen;
R⁸ und R⁹ unabhängig ausgewählt sind aus:
Wasserstoff;
carbocyklischem oder heterocyklischem Aryl, welches ausgewählt ist unter Phenyl, Naphthyl, Pyridyl, 1-H-Tetrazol-5-yl, Thiazolyl, Imidazolyl, Indolyl, Pyrimidinyl, Thiadiazolyl, Pyrazolyl, Oxazolyl, Isoxazolyl, Oxadiazolyl, Thienyl, Chinolinyl, Pyrazinyl und Isothiazolyl, und welches wahlweise substituiert ist mit Halogen, Amino, C₃₋₆-Cykloalkyl, Hydroxy, -COOR²² oder -CONR²³R²⁴; und C₁₋₆-Alkyl, wahlweise substituiert mit: Halogen; Amino; C₃₋₆-Cykloalkyl; Hydroxy; carbocyklischem oder heterocyklischem Aryl, welches ausgewählt ist unter Phenyl, Naphthyl, Pyridyl, 1-H-Tetrazol-5-yl, Thiazolyl, Imidazolyl, Indolyl, Pyrimidinyl, Thiadiazolyl, Pyrazolyl, Oxazolyl, Isoxazolyl, Oxadiazolyl, Thienyl, Chinolinyl, Pyrazinyl und Isothiazolyl, und welches wahlweise substituiert ist mit einer oder mehreren C₁₋₆-Alkyl-, C₁₋₆-Alkoxy-, Halogen- oder Aminogruppen; -COOR²²; oder -CONR²³R²⁴; und
G, J, R¹, R², R⁵, R⁶, R⁷, R²², R²³, R²⁴, a und b wie in Anspruch 1 definiert sind;
oder ein pharmazeutisch geeignetes Salz davon.

4. Verbindung nach Anspruch 2 oder 3, worin R⁵ Wasserstoff oder C₁₋₆-Alkyl ist, wahlweise substituiert mit:
carbocyklischem oder heterocyklischem Aryl, welches ausgewählt ist unter Phenyl, Naphthyl, Pyridyl, 1-H-Tetrazol-5-yl, Thiazolyl, Imidazolyl, Indolyl, Pyrimidinyl, Thiadiazolyl, Pyrazolyl, Oxazolyl, Isoxazolyl, Oxadiazolyl, Thienyl, Chinolinyl, Pyrazinyl und Isothiazolyl, und welches wahlweise substituiert ist mit einer oder mehreren C₁₋₆-Alkyl-, C₁₋₆-Alkoxy-, Halogen- oder Aminogruppen; Hydroxy; C₃₋₆-Cykloylkyl; Amino; -COOR²⁵; oder -CONR²⁶R²⁷;
-NR'R"; 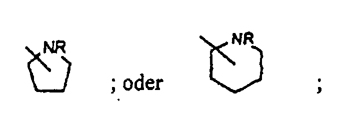 R, R' und R" unabhängig Wasserstoffoder C₁₋₆-Alkyl sind;
R²⁵, R²⁶ und R²⁷ unabhängig Wasserstoffoder C₁₋₆-Alkyl sind; und
R¹, R², R³, R⁴, R⁶, R⁷, R⁸, R⁹, R¹⁰, R¹¹, E, G, J, a, b, c und d wie in einem der vorangegangenen Ansprüche definiert sind;
oder ein pharmazeutisch geeignetes Salz davon.

5. Verbindung nach irgendeinem der Ansprüche 2-4, worin
E ist 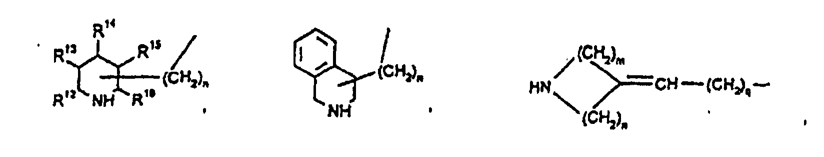 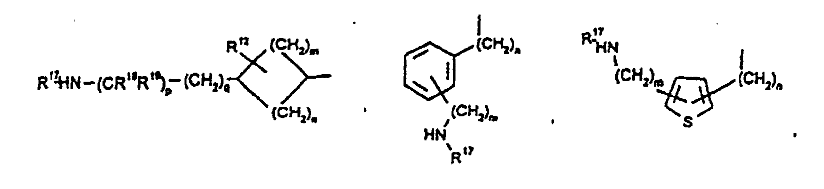 oder
R¹⁷-NH―(CR¹⁸R¹⁹)ₚ―(CH₂)ₘ―M―(CHR²⁰)ₒ―(CH₂)ₙ― ;
R¹², R¹³, R¹⁴, R¹⁵, R¹⁶, R¹⁷, R¹⁸, R¹⁹ und R²⁰ unabhängig Wasserstoff oder Methyl sind;
n, m und q unabhängig 0, 1, 2 oder 3 sind;
o und p unabhängig 0 oder 1 sind;
M ist -CH=CR²¹-, -O- oder eine Valenzbindung;
R²¹ Wasserstoff, Methyl oder Benzyl ist; und
R¹, R², R³, R⁴, R⁵, R⁶, R⁷, R⁸, R⁹, R¹⁰, R¹¹, G, J, a, b, c und d wie in einem der vorangegangenen Ansprüche definiert sind;
oder ein pharmazeutisch geeignetes Salz davon.

6. Verbindung nach Anspruch 1, worin
R³ und R⁴ unabhängig ausgewählt sind aus:
Wasserstoff;
carbocyklischem oder heterocyklischem Aryl, welches ausgewählt ist unter Phenyl, Naphthyl, Pyridyl, 1-H-Tetrazol-5-yl, Thiazolyl, Imidazolyl, Indolyl, Pyrimidinyl, Thiadiazolyl, Pyrazolyl, Oxazolyl, Isoxazolyl, Oxadiazolyl, Thienyl, Chinolinyl, Pyrazinyl und Isothiazolyl, und welches wahlweise substituiert ist mit Halogen, Amino, C₃₋₆-Cykloalkyl, Hydroxy, -COOR²² oder -CONR²³R²⁴; und C₁₋₆-Alkyl, wahlweise substituiert mit: Halogen; Amino; C₃₋₆-Cykloalkyl; Hydroxy; carbocyklischem oder heterocyklischem Aryl, welches ausgewählt ist unter Phenyl, Naphthyl, Pyridyl, 1-H-Tetrazol-5-yl, Thiazolyl, Imidazolyl, Indolyl, Pyrimidinyl, Thiadiazolyl, Pyrazolyl, Oxazolyl, Isoxazolyl, Oxadiazolyl, Thienyl, Chinolinyl, Pyrazinyl und Isothiazolyl, und welches wahlweise substituiert ist mit einer oder mehreren C₁₋₆-Alkyl-, C₁₋₆-Alkoxy-, Halogen- oder Aminogruppen; -COOR²²; oder -CONR²³R²⁴;
R²², R²³ und R²⁴ unabhängig Wasserstoff oder C₁₋₆-Alkyl sind;
E ist 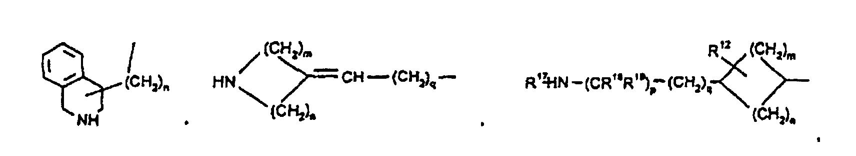 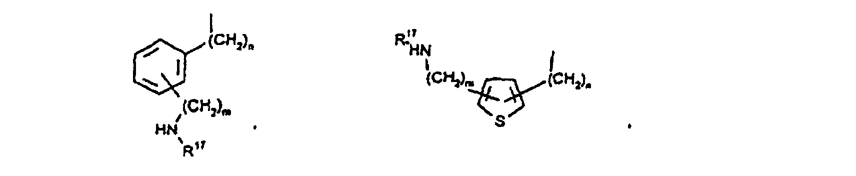 oder
R¹⁷-NH―(CR¹⁸R¹⁹)ₚ―(CH₂)ₘ-Q―(CHR²⁰)ₒ―(CH₂)ₙ―
R¹², R¹⁷, R¹⁸, R¹⁹ und R²⁰ unabhängig ausgewählt sind aus:
Wasserstoff; und
C₁₋₆-Alkyl, wahlweise substituiert mit: Halogen; Amino; Hydroxyl; oder carbocyklischem oder heterocyklischem Aryl, welches ausgewählt ist unter Phenyl, Naphthyl, Pyridyl, 1-H-Tetrazol-5-yl, Thiazolyl, Imidazolyl, Indolyl, Pyrimidinyl, Thiadiazolyl, Pyrazolyl, Oxazolyl, Isoxazolyl, Oxadiazolyl, Thienyl, Chinolinyl, Pyrazinyl und Isothiazolyl, und welches wahlweise substituiert ist mit einer oder mehreren C₁₋₆-Alkyl-, C₁₋₆-Alkoxy-, Halogen- oder Aminogruppen;
beliebige zwei von R¹⁷, R¹⁸ und R¹⁹ unabhängig miteinander verbunden sein können, um eine C₁₋₆-Alkylenbrücke zu bilden;
n, m und q unabhängig 0, 1, 2 oder 3 sind;
o und p unabhängig 0 oder 1 sind;
Q ist -CH=CR²¹-, -O- oder -S-;
R²¹ ausgewählt aus ist:
Wasserstoff; und
C₁₋₆-Alkyl, wahlweise substituiert mit: Halogen; Amino; Hydroxyl; oder carbocyklischem oder heterocyklischem Aryl, welches ausgewählt ist unter Phenyl, Naphthyl, Pyridyl, 1-H-Tetrazol-5-yl, Thiazolyl, Imidazolyl, Indolyl, Pyrimidinyl, Thiadiazolyl, Pyrazolyl, Oxazolyl, Isoxazolyl, Oxadiazolyl, Thienyl, Chinolinyl, Pyrazinyl und Isothiazolyl, und
welches wahlweise substituiert ist mit einer oder mehreren C₁₋₆-Alkyl-, C₁₋₆-Alkoxy-, Halogen- oder Aminogruppen; und
R¹, R², R⁵, R⁶, R⁷, R⁸, R⁹, R¹⁰, R¹¹, G, J, a, b, c und d wie in Anspruch 1 definiert sind;
oder ein pharmazeutisch geeignetes Salz davon.

7. Verbindung der allgemeinen Formel IV 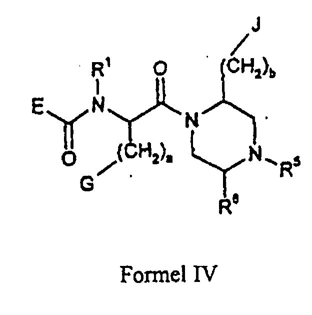 worin E ist 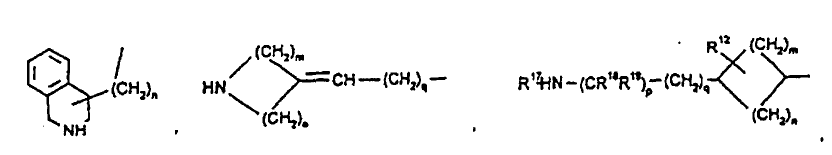 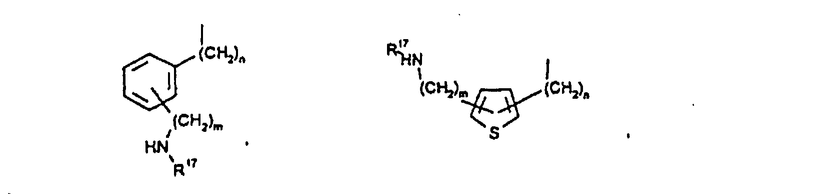 oder
R¹⁷-NH―(CR¹⁸R¹⁹)ₚ―(CH₂)ₘ-Q―(CHR²⁰)ₒ―(CH₂)ₙ― ;
R¹², R¹⁷, R¹⁸, R¹⁹ und R²⁰ unabhängig ausgewählt sind aus:
Wasserstoff; und
C₁₋₆-Alkyl, wahlweise substituiert mit: Halogen; Amino; Hydroxyl; oder carbocyklischem oder heterocyklischem Aryl, welches ausgewählt ist unter Phenyl, Naphthyl, Pyridyl, 1-H-Tetrazol-5-yl, Thiazolyl, Imidazolyl, Indolyl, Pyrimidinyl, Thiadiazolyl, Pyrazolyl, Oxazolyl, Isoxazolyl, Oxadiazolyl, Thienyl, Chinolinyl, Pyrazinyl und Isothiazolyl, und welches wahlweise substituiert ist mit einer oder mehreren C₁₋₆-Alkyl-, C₁₋₆-Alkoxy-, Halogen- oder Aminogruppen; beliebige zwei von R¹⁷, R¹⁸ und R¹⁹ unabhängig miteinander verbunden sein können, um eine C₁₋₆-Alkylenbrücke zu bilden;
n, m und q unabhängig 0, 1, 2 oder 3 sind;
o und p unabhängig 0 oder 1 sind;
Q ist -CH=CR²¹-, -O- oder -S-;
R²¹ ausgewählt ist aus:
Wasserstoff; und
C₁₋₆-Alkyl, wahlweise substituiert mit: Halogen; Amino; Hydroxyl; oder carbocyklischem oder heterocyklischem Aryl, welches ausgewählt ist unter Phenyl, Naphthyl, Pyridyl, 1-H-Tetrazol-5-yl, Thiazolyl, Imidazolyl, Indolyl, Pyrimidinyl, Thiadiazolyl, Pyrazolyl, Oxazolyl, Isoxazolyl, Oxadiazolyl, Thienyl, Chinolinyl, Pyrazinyl und Isothiazolyl, und welches wahlweise substituiert ist mit einer oder mehreren C₁₋₆-Alkyl-, C₁₋₆-Alkoxy-, Halogen- oder Aminogruppen; und
R¹, R⁵, R⁶, G, J, a und b wie in Anspruch 1 definiert sind;
oder ein pharmazeutisch geeignetes Salz davon.

8. Verbindung der allgemeinen Formel V 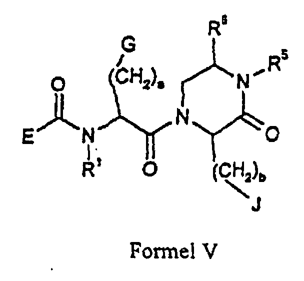 worin E ist 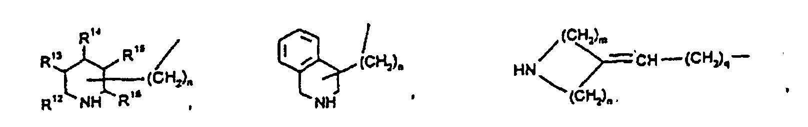 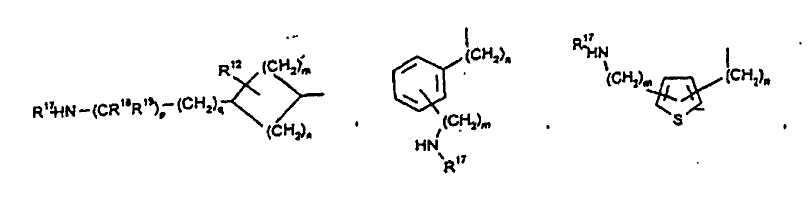 oder
R¹⁷-NH―(CR¹⁸R¹⁹)ₚ―(CH₂)ₘ-M―(CHR²⁰)ₒ―(CH₂)ₙ― ;
R¹², R¹³, R¹⁴, R¹⁵, R¹⁶, R¹⁷, R¹⁸, R¹⁹ und R²⁰ unabhängig ausgewählt sind aus:
Wasserstoff; und
C₁₋₆-Alkyl, wahlweise substituiert mit: Halogen; Amino; Hydroxyl; oder carbocyklischem oder heterocyklischem Aryl, welches ausgewählt ist unter Phenyl, Naphthyl, Pyridyl, 1-H-Tetrazol-5-yl, Thiazolyl, Imidazolyl, Indolyl, Pyrimidinyl, Thiadiazolyl, Pyrazolyl, Oxazolyl, Isoxazolyl, Oxadiazolyl, Thienyl, Chinolinyl, Pyrazinyl und Isothiazolyl, und welches wahlweise substituiert ist mit einer oder mehreren C₁₋₆-Alkyl-, C₁₋₆-Alkoxy-, Halogen- oder Aminogruppen; beliebige zwei von R¹⁷, R¹⁸ und R¹⁹ unabhängig miteinander verbunden sein können, um eine C₁₋₆-Alkylenbrücke zu bilden;
n, m und q unabhängig 0, 1, 2 oder 3 sind;
o und p unabhängig 0 oder 1 sind;
M ist -CH=CR²¹-, -O- oder -S- oder eine Valenzbindung;
R²¹ ausgewählt ist aus:
Wasserstoff; und
C₁₋₆-Alkyl, wahlweise substituiert mit: Halogen; Amino; Hydroxyl; oder carbocyklischem oder heterocyklischem Aryl, welches ausgewählt ist unter Phenyl, Naphthyl, Pyridyl, 1-H-Tetrazol-5-yl, Thiazolyl, Imidazolyl, Indolyl, Pyrimidinyl, Thiadiazolyl, Pyrazolyl, Oxazolyl, Isoxazolyl, Oxadiazolyl, Thienyl, Chinolinyl, Pyrazinyl und Isothiazolyl, und welches wahlweise substituiert ist mit einer oder mehreren C₁₋₆-Alkyl-, C₁₋₆-Alkoxy-, Halogen- oder Aminogruppen;
R⁵ Wasserstoff oder C₁₋₆-Alkyl ist, wahlweise substituiert mit:
carbocyklischem oder heterocyklischem Aryl, welches ausgewählt ist unter Phenyl, Naphthyl, Pyridyl, 1-H-Tetrazol-5-yl, Thiazolyl, Imidazolyl, Indolyl, Pyrimidinyl, Thiadiazolyl, Pyrazolyl, Oxazolyl, Isoxazolyl, Oxadiazolyl, Thienyl, Chinotinyl, Pyrazinyl und Isothiazolyl, und welches wahlweise substituiert ist mit einer oder mehreren C₁₋₆-Alkyl-, C₁₋₆-Alkoxy-, Halogen- oder Aminogruppen; Hydroxy; C₃₋₆-Cykloalkyl; Amino; -COOR²⁵; -CONR²⁶R²⁷;
-NR'R"; 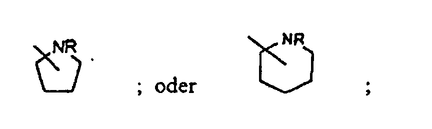 R, R' und R" unabhängig Wasserstoff oder C₁₋₆-Alkyl sind;
R²⁵, R²⁶ und R²⁷ unabhängig Wasserstoff oder C₁₋₆-Alkyl sind; und
G, J, a, b, R¹ und R⁶ wie in Anspruch 1 definiert sind;
oder ein pharmazeutisch geeignetes Salz davon.

9. Verbindung nach einem der Ansprüche 1-8, worin
R¹ Wasserstoff oder Methyl ist;
oder ein pharmazeutisch geeignetes Salz davon.

10. Verbindung nach einem der vorangehenden Ansprüche, ausgewählt aus der Gruppe bestehend aus:
(2E)-5-Amino-5-methylhex-2-ensäure-N-((1R)-1-((2R)-2-benzyl-3-oxopiperazin-1-carbonyl)-2-(2-naphthyl)ethyl)-N-methylamid,
(2E)-5-Amino-5-methylhex-2-ensäure-N-((1R)-1-((2R)-2-benzyl-4-(cyclopropylmethyl)-3-oxopiperazin-1-carbonyl)-2-(2-naphthyl)ethyl)-N-methylamid,
(2E)-5-Amino-5-methylhex-2-ensäure-N-((1R)-1-((2R,5R)-2-benzyl-5-(hydroxymethyl)-3-oxopiperazin-1-carbonyl)-2-(2-naphthyl)ethyl)-N-methylamid,
(2E)-5-Amino-5-methylhex-2-ensäure-N-((1R)-1-((2R)-2-benzyl-3-oxo-1,4-diazepan-1-carbonyl)-2-(2-naphthyl)ethyl)-N-methylamid,
Piperidin-4-carbonsäure-N-methyl-N-((1R)-2-(2-naphthyl)-1-1((2R)-2-((2-naphthyl)methyl)-3-oxopiperazin-1-carbonyl)ethyl)amid,
(2E)-5-Amino-5-methylhex-2-ensäure-N-((1R)-1-((2R)-2-benzyl-4-(dimethylcarbamoylmethyl)-3-oxopiperazin-1-carbonyl)-2-(2-naphthyl)ethyl)-N-methylamid,
(2E)-5-Methyl-5-(methylamino)hex-2-ensäure-N-((1R)-1-((2R)-2-benzyl-3-oxopiperazin-1-carbonyl)-2-(2-naphthyl)ethyl)-N-methylamid,
(2E)-5-Amino-5-methylhex-2-ensäure-N-((1R)-1-((2R,6S)-2-benzyl-6-methyl-3-oxopiperazin-1-carbonyl)-2-(2-naphthyl)ethyl)-N-methylamid,
(2E)-5-Amino-5-methylhex-2-ensäure-N-((1R)-1-((2R)-2-benzyl-3-oxopiperazin-1-carbonyl)-2-(biphenyl-4-yl)ethyl)-N-methylamid,
(2E)-5-Amino-5-methylhex-2-ensäure-N-(( 1R)-1-((2R)-2-benzyl-4-methansulfonylpiperazin-1-carbonyl)-2-(2-naphthyl)ethyl)-N-methylamid,
(2E)-5-Amino-5-methylhex-2-ensäure-N-((1R)-1-((2R)-2-benzyl-4-methansulfonylpiperazin-1-carbonyl)-2-benzyloxyethyl)-N-methylamid,
(2E)-5-Amino-3,5-dimethylhex-2-ensäure-N-((1R)-1-((2R)-2-benzyl-3-oxopiperazin-1-carbonyl)-2-(2-naphthyl)ethyl)-N-methylamid,
(2E)-5-Methyl-5-methylaminohex-2-ensäure-N-((1R)-1-((2R,5R)-2-benzyl-5-methyl-3-oxopiperazin-1-carbonyl)-2-(2-naphthyl)ethyl)-N-methylamid,
(2E)-5-Amino-5-methylhex-2-ensäure-N-((1R)-2-((2R)-2-benzyl-4-methyl-3-oxopiperazin-1-yl)-1-((2-naphthyl)methyl)-2-oxoethyl)-N-methylamid,
(2E)-5-Methyl-5-aminohex-2-ensäure-N-((1R)-1-((2R,5R)-2-benzyl-5-methyl-3-oxopiperazin-1-carbonyl)-2-(2-naphthyl)ethyl)-N-methylamid,
(2E)-4-(1-Aminocyclobutyl)but-2-ensäure-N-methyl-N-((1R)-2-(2-naphthyl)-1-((2R)-3-oxo-2-((2-thienyl)methyl)piperazin-1-carbonyl)ethyl)amid,
(2E)-4-(1-Aminocyclobutyl)but-2-ensäure-N-((1R)-2-((2R,5R)-2-benzyl-5-methyl-3-oxopiperazin-1-yl)-1-((2-naphthyl)methyl)-2-oxoethyl)-N-methylamid,
(2E)-5-Methyl-5-methylamino-hex-2-ensäure-N-methyl-N-((1R)-1-(2-naphthyl)methyl-2-oxo-2-((2R)-3-oxo-2-((2-thienyl)methyl)piperazin-1-yl)ethyl)amid und
(2E)-5-Amino-5-methyl-hex-2-ensäure-N-((1R)-2-((2R)-2-benzyl-4-methansulfonylpiperazin-1-yl)-1-(naphth-2-ylmethyl)-2-oxo-ethyl)-N-methylamid;
oder ein pharmazeutisch geeignetes Salz davon.

11. Pharmazeutische Zusammensetzung umfassend, als einen aktiven Bestandteil, eine Verbindung gemäß irgendeinem der Ansprüche 1-10 oder ein pharmazeutisch geeignetes Salz davon, zusammen mit einem pharmazeutisch geeigneten Träger oder Verdünnungsmittel.

12. Verbindung gemäß Anspruch 11 in Einheit-Dosierungsform, umfassend von ca. 10 bis ca. 200 mg einer Verbindung gemäß einem der Ansprüche 1-10 oder einem pharmazeutisch geeigneten Salz davon.

13. Pharmazeutische Zusammensetzung zur Stimulierung der Freisetzung von Wachstumshormon von der Hypophyse eines Menschen oder Tieres, die Verbindung umfasst, als einen aktiven Bestandteil, eine Verbindung gemäß irgendeinem der Ansprüche 1-10 oder ein pharmazeutisch geeignetes Salz davon, zusammen mit einem pharmazeutisch geeigneten Träger oder Verdünnungsmittel.

14. Pharmazeutische Zusammensetzung zur Verabreichung an Tiere, um ihre/ihr Wachstumsrate und -ausmaß zu erhöhen, um ihre Milch- oder Wollproduktion zu erhöhen oder zur Behandlung von Beschwerden, die Zusammensetzung umfasst, als einen aktiven Bestandteil, eine Verbindung gemäß irgendeinem der Ansprüche 1-10 oder ein pharmazeutisch geeignetes Salz davon, zusammen mit einem pharmazeutisch geeigneten Träger oder Verdünnungsmittel.

15. Pharmazeutische Zusammensetzung gemäß irgendeinem der Ansprüche 11-14 zur oralen, nasalen, transdermalen, pulmonalen oder parenteralen Verabreichung.

16. Verwendung einer Verbindung gemäß irgendeinem der Ansprüche 1-10 oder eines pharmazeutisch geeigneten Salzes davon zur Zubereitung eines Medikaments.

17. Verwendung einer Verbindung gemäß irgendeinem der Ansprüche 1-10 oder eines pharmazeutisch geeigneten Salzes davon zur Zubereitung eines Medikaments zur Stimulierung der Freisetzung von Wachstumshormon von der Hypophyse eines Menschen oder eines Tieres.

18. Verwendung einer Verbindung gemäß irgendeinem der Ansprüche 1-10 oder eines pharmazeutisch geeigneten Salzes davon zur Zubereitung eines Medikaments zur Verabreichung an Tiere, um ihre/ihr Wachstumsrate und -ausmaß zu erhöhen, um ihre Milch- oder Wollproduktion zu erhöhen oder zur Behandlung von Beschwerden.

19. Verwendung einer Verbindung gemäß irgendeinem der Ansprüche 1-10 oder eines pharmazeutisch geeigneten Salzes davon zur Zubereitung eines Medikaments zur: Stimulation von Wachstumshormon-Freisetzung bei den Älteren; Schutz vor katabolischen Nebenwirkungen von Glucocorticoiden; Prävention und Behandlung von Osteoporose; Stimulation des Immunsystems; Beschleunigung der Wundheilung; beschleunigte Knochenfrakturreparatur; Behandlung von Wachstumsverzögerung; Behandlung von Nierenversagen oder -insuffizienz, resultierend aus einer Wachstumsverzögerung; Behandlung von physiologisch kleiner Statur, einschließlich Behandlung von Wachstumshormon-defizienten Kindern und Behandlung von kleiner Statur, verbunden mit chronischer Krankheit; Behandlung von Fettsucht und Wachstumsverzögerung verbunden mit Fettsucht; Behandlung von Wachstumsverzögerung, verbunden mit dem Prader-Willi-Syndrom und mit dem Turner-Syndrom; Beschleunigung der Genesung und Reduzierung des Krankenhausaufenthaltes von Verbrennungspatienten; Behandlung von Wachstumsstörung innerhalb der Gebärmutter, Skelett-Fehlbildung, Hyperkortisolismus (hypercortisolism) und Cushing's-Syndrom; Induzierung von pulsierender Wachstumshormon-Freisetzung; Ersatz von Wachstumshormon bei Stresspatienten, Behandlung von Osteochondrodysplasie, Noonan's Syndrom, Schizophrenie, Depressionen, Alzheimer-Krankheit, verzögerte Wundheilung und psychosoziale Deprivation; Behandlung von pulmonare Disfunktion und Ventilationsabhängigkeit; Abschwächung von Protein-katabolischen Antworten (protein catabolic response) nach großen Operationen; Reduzierung von Kräfteverfall und Proteinverlust durch chronische Krankheiten, wie Krebs oder AIDS; Behandlung von Hyperinsulinämie, einschließlich Nesidioblastose; Zusatzbehandlung zur Eisprung-Induktion, Stimulierung der Thymusentwicklung und Prävention vor altersbedingter Abnahme der Thymusfunktion; Behandlung von immunsuppressiven Patienten; Verbesserung der Muskelstärke und -mobilität; Erhaltung der Hautdicke; metabolische Homöostasis; Nierenhomöostasis bei gebrechlichen Älteren; Stimulation von Knochenbildung; Knochenumbildung und Knorpelwachstum; Stimulation des Immunsystems in Begleittieren (companion animals) und Behandlung von Alterskrankheiten in Begleittieren (companion animals); Wachstumsförderung beim Vieh; oder Stimulation des Wollwachstums beim Schaf.

## Revendications

1. Composé de formule générale I : 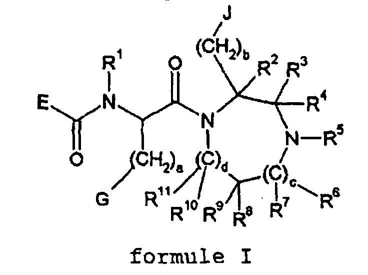 dans laquelle
R¹ est : l'hydrogène ; ou un radical alkyle en C₁ à C₆ éventuellement substitué par un groupe aryle carbocyclique ou hétérocyclique qui est choisi parmi phényle, naphtyle, pyridyle, 1-H-tétrazol-5-yle, thiazolyle, imidazolyle, indolyle, pyrimidinyle, thiadiazolyle, pyrazolyle, oxazolyle, isoxazolyle, oxadiazolyle, thiényle, quinolinyle, pyrazinyle et isothiazolyle, et qui est éventuellement substitué par un ou plusieurs groupes alkyle en C₁ à C₆, alcoxy en C₁ à C₆, halogéno ou amine ;
a et b valent indépendamment 1 ou 2 ;
c et d valent indépendamment 0, 1 ou 2 ;
c + d vaut 0, 1 ou 2 ;
R², R³, R⁴, R⁶, R⁷, R⁸, R⁹, R¹⁰ et R¹¹ sont indépendamment choisis parmi :
l'hydrogène ;
les radicaux aryle carbocycliques ou hétérocycliques qui sont choisis parmi phényle, naphtyle, pyridyle, 1-H-tétrazol-5-yle, thiazolyle, imidazolyle, indolyle, pyrimidinyle, thiadiazolyle, pyrazolyle, oxazolyle, isoxazolyle, oxadiazolyle, thiényle, quinolinyle, pyrazinyle et isothiazolyle, et qui sont éventuellement substitués par un halogène ou un radical amino, cycloalkyle en C₃ à C₆, hydroxy, -COOR²² ou -CONR²³R²⁴ ; et
les radicaux alkyle en C₁ à C₆ éventuellement substitués par un halogène ou un radical amino ; cycloalkyle en C₃ à C₆ ; hydroxy ; aryle carbocyclique ou hétérocyclique qui est choisi parmi phényle, naphtyle, pyridyle, 1-H-tétrazol-5-yle, thiazolyle, imidazolyle, indolyle, pyrimidinyle, thiadiazolyle, pyrazolyle, oxazolyle, isoxazolyle, oxadiazolyle, thiényle, quinolinyle, pyrazinyle et isothiazolyle, et qui est éventuellement substitué par un ou plusieurs groupes alkyle en C₁ à C₆, alcoxy en C₁ à C₆, halogéno ou amino ; -COOR²² ; ou -CONR²³R²⁴ ;
R³ et R⁴ peuvent être pris ensemble pour former =O ou =S ;
R⁸ et R⁹ peuvent être pris ensemble pour former =O ou =S ;
R⁵ est l'hydrogène, 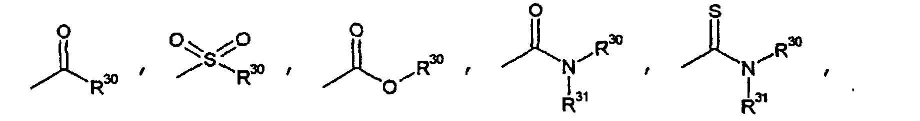 ou un radical alkyle en C₁ à C₆ éventuellement substitué par :
un radical aryle carbocyclique ou hétérocyclique qui est choisi parmi phényle, naphtyle, pyridyle, 1-H-tétrazol-5-yle, thiazolyle, imidazolyle, indolyle, pyrimidinyle, thiadiazolyle, pyrazolyle, oxazolyle, isoxazolylé, oxadiazolyle, thiényle, quinolinyle, pyrazinyle et isothiazolyle, et qui est éventuellement substitué par un ou plusieurs groupes alkyle en C₁ à C₆, alcoxy en C₁ à C₆, halogéno ou amino ; hydroxy ; cycloalkyle en C₃ à C₆ ; amino ; -COOR²⁵ ; -CONR²⁶R²⁷ ;
-NR'R" ; 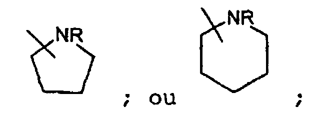 R, R' et R" sont indépendamment l'hydrogène ou un radical alkyle en C₁ à C₆ ;
R³⁰ et R³¹ sont des radicaux alkyle en C₁ à C₆ éventuellement substitués par : un radical aryle carbocyclique ou hétérocyclique qui est choisi parmi phényle, naphtyle, pyridyle, 1-H-tétrazol-5-yle, thiazolyle, imidazolyle, indolyle, pyrimidinyle, thiadiazolyle, pyrazolyle, oxazolyle, isoxazolyle, oxadiazolyle, thiényle, quinolinyle, pyrazinyle et isothiazolyle, et qui est éventuellement substitué par un ou plusieurs groupes alkyle en C₁ à C₆, alcoxy en C₁ à C₆, halogéno ou amino ; hydroxy ; cycloalkyle en C₃ à C₆ ; ou amino ;
R²², R²³, R²⁴, R²⁵, R²⁶ et R²⁷ sont indépendamment l'hydrogène ou un radical alkyle en C₁ à C₆ ;
quand R³ et R⁴ sont pris ensemble pour former =O ou =S,
alors E est 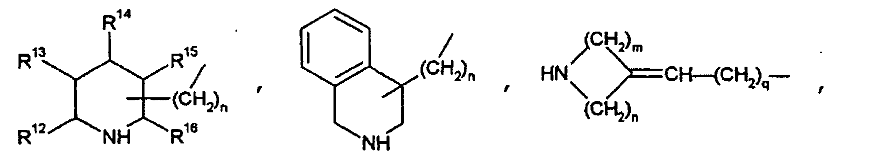 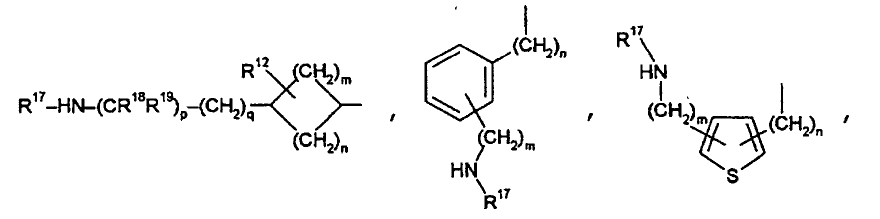 ou
R¹⁷-NH-(CR¹⁸R¹⁹)ₚ-(CH₂)ₘ-M-(CHR²⁰)ₒ-(CH₂)ₙ- ;
quand R³ ou R⁴ est : l'hydrogène ou un radical aryle carbocyclique ou hétérocyclique qui est choisi parmi phényle, naphtyle, pyridyle, 1-H-tétrazol-5-yle, thiazolyle, imidazolyle, indolyle, pyrimidinyle, thiadiazolyle, pyrazolyle, oxazolyle, isoxazolyle, oxadiazolyle, thiényle, quinolinyle, pyrazinyle et isothiazolyle, et qui est éventuellement substitué par un halogène ou un radical amino, cycloalkyle en C₃ à C₆, hydroxy, -COOR²² ou -CONR²³R²⁴ ; ou alkyle en C₁ à C₆ éventuellement substitué par : un halogène ou un radical amino ; cycloalkyle en C₃ à C₆ ; hydroxy, aryle carbocyclique ou hétérocyclique qui est choisi parmi phényle, naphtyle, pyridyle, 1-H-tétrazol-5-yle, thiazolyle, imidazolyle, indolyle, pyrimidinyle, thiadiazolyle, pyrazoyle, oxazolyle, isoxazolyle, oxadiazolyle, thiényle, quinolinyle, pyrazinyle et isothiazolyle, et qui est éventuellement substitué par un ou plusieurs groupes alkyle en C₁ à C₆, alcoxy en C₁ à C₆, halogéno ou amino ; -COOR²² ; ou -CONR²³R²⁴ ;
alors E est 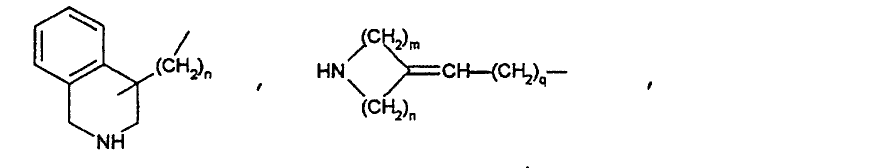 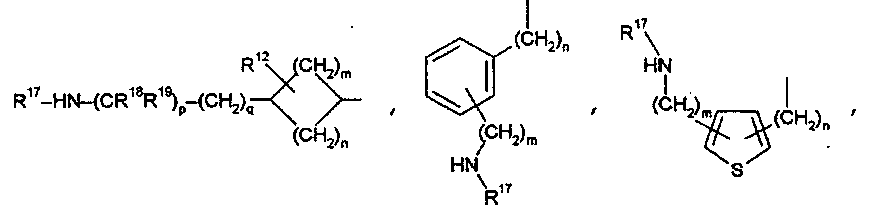 ou
R¹⁷-NH-(CR¹⁸R¹⁹)ₚ-(CH₂)ₘ-M-(CHR²⁰)ₒ-(CH₂)ₙ- ;
R¹², R¹³, R¹⁴, R¹⁵, R¹⁶, R¹⁷, R¹⁸, R¹⁹ et R²⁰ sont indépendamment choisis parmi :
l'hydrogène ; et
les radicaux alkyle en C₁ à C₆ éventuellement substitués par : un halogène ; ou un radical amino ; hydroxyle ; ou aryle carbocyclique ou hétérocyclique qui est choisi parmi phényle, naphtyle, pyridyle, 1-H-tétrazol-5-yle, thiazolyle, imidazolyle, indolyle, pyrimidinyle, thiadiazolyle, pyrazolyle, oxazolyle, isoxazolyle, oxadiazolyle, thiényle, quinolinyle, pyrazinyle et isothiazolyle, et qui est éventuellement substitué par un ou plusieurs groupes alkyle en C₁ à C₆, alcoxy en C₁ à C₆, halogéno ou amino ;
deux quelconques de R¹⁷, R¹⁸ et R¹⁹ peuvent indépendamment se réunir ensemble pour former un pont alkylène en C₁ à C₆ ;
n, m et q valent indépendamment 0, 1, 2 ou 3 ;
o et p valent indépendamment 0 ou 1 ;
M est -CH=CR²¹, -O-, -S- ou une liaison de valence ;
Q est -CH=CR²¹, -O- ou -S- ;
R²¹ est choisi parmi :
l'hydrogène ; et
les radicaux alkyle en C₁ à C₆ éventuellement substitués par : un halogène ; ou un radical amino ; hydroxyle ; ou aryle carbocyclique ou hétérocyclique qui est choisi parmi phényle, naphtyle, pyridyle, 1-H-tétrazol-5-yle, thiazolyle, imidazolyle, indolyle, pyrimidinyle, thiadiazolyle, pyrazolyle, oxazolyle, isoxazolyle, oxadiazolyle, thiényle, quinolinyle, pyrazinyle et isothiazolyle, et qui est éventuellement substitué par un ou plusieurs groupes alkyle en C₁ à C₆, alcoxy en C₁ à C₆, halogéno ou amino ;
G est l'hydrogène, 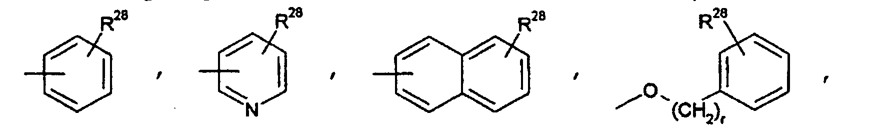 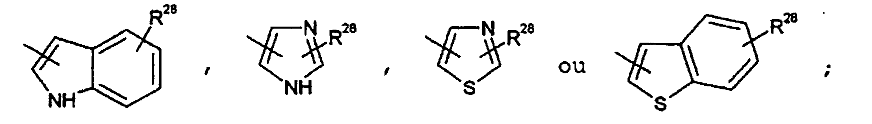 où R²⁸ est choisi parmi ; l'hydrogène ; les halogènes ; et les radicaux alkyle en C₁ à C₆ ; alcoxy en C₁ à C₆ ; et aryle carbocycliques ou hétérocycliques qui sont choisis parmi phényle, naphtyle, pyridyle, 1-H-tétrazol-5-yle, thiazolyle, imidazolyle, indolyle, pyrimidinyle, thiadiazolyle, pyrazolyle, oxazolyle, isoxazolyle, oxadiazolyle, thiényle, quinolinyle, pyrazinyle et isothiazolyle, et qui sont éventuellement substitués par un ou plusieurs groupes alkyle en C₁ à C₆, alcoxy en C₁ à C₆, halogéno ou amino ;
r vaut 0, 1 ou 2 ;
J est l'hydrogène, 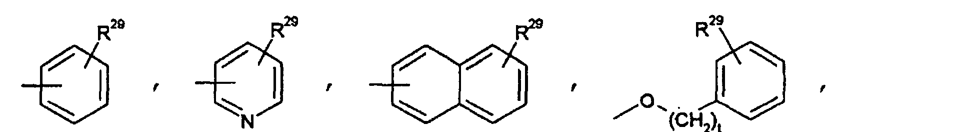 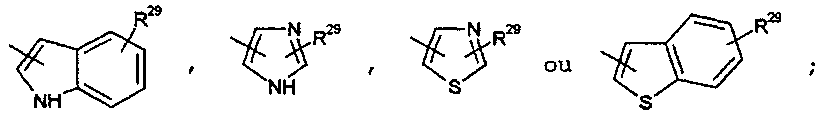 où R²⁹ est choisi parmi ; l'hydrogène ; les halogènes ; et les radicaux alkyle en C₁ à C₆ ; alcoxy en C₁ à C₆ ; et aryle carbocycliques ou hétérocycliques qui sont choisis parmi phényle, naphtyle, pyridyle, 1-H-tétrazol-5-yle, thiazolyle, imidazolyle, indolyle, pyrimidinyle, thiadiazolyle, pyrazolyle, oxazolyle, isoxazolyle, oxadiazolyle, thiényle, quinolinyle, pyrazinyle et isothiazolyle, et qui sont éventuellement substitués par un ou plusieurs groupes alkyle en C₁ à C₆, alcoxy en C₁ à C₆, halogéno ou amino ;
t vaut 0, 1 ou 2 ;
ou un sel acceptable en pharmacie d'un tel composé.

2. Composé de formule générale II : 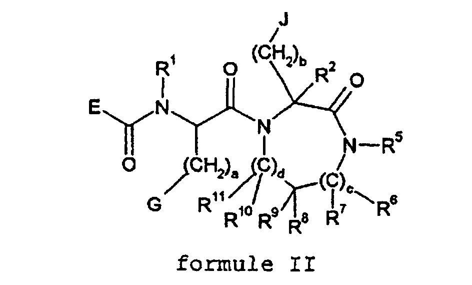 dans laquelle
E est 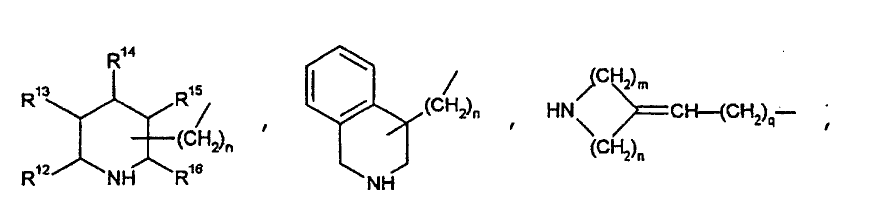 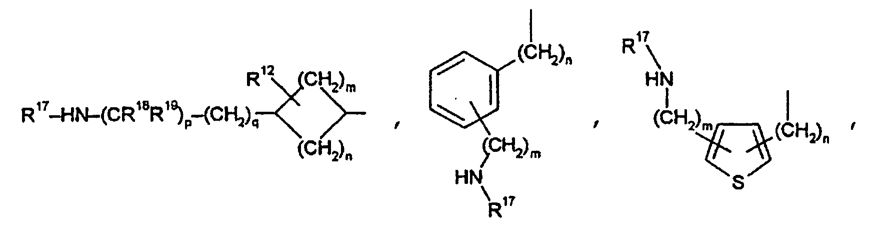 ou
R¹⁷-NH-(CR¹⁸R¹⁹)ₚ-(CH₂)ₘ-M-(CHR²⁰)ₒ-(CH₂)ₙ- ;
R¹², R¹³, R¹⁴, R¹⁵, R¹⁶, R¹⁷, R¹⁸, R¹⁹ et R²⁰ sont indépendamment choisis parmi :
l'hydrogène ; et
les radicaux alkyle en C₁ à C₆ éventuellement substitués par : un halogène ; ou un radical amino ; hydroxyle ; ou aryle carbocyclique ou hétérocyclique qui est choisi parmi phényle, naphtyle, pyridyle, 1-H-tétrazol-5-yle, thiazolyle, imidazolyle, indolyle, pyrimidinyle, thiadiazolyle, pyrazolyle, oxazolyle, isoxazolyle, oxadiazolyle, thiényle, quinolinyle, pyrazinyle et isothiazolyle, et qui est éventuellement substitué par un
ou plusieurs groupes alkyle en C₁ à C₆, alcoxy en C₁ à C₆, halogéno ou amino ;
deux quelconques de R¹⁷, R¹⁸ et R¹⁹ peuvent indépendamment se réunir ensemble pour former un pont alkylène en C₁ à C₆ ;
n, m et q valent indépendamment 0, 1, 2 ou 3 ;
o et p valent indépendamment 0 ou 1 ;
M est -CH=CR²¹, -O-, -S- ou une liaison de valence ;
R²¹ est choisi parmi :
l'hydrogène ; et
les radicaux alkyle en C₁ à C₆ éventuellement substitués par : un halogène ; ou un radical amino ; hydroxyle ; ou aryle carbocyclique ou hétérocyclique qui est choisi parmi phényle, naphtyle, pyridyle, 1-H-tétrazol-5-yle, thiazolyle, imidazolyle, indolyle, pyrimidinyle, thiadiazolyle, pyrazolyle, oxazolyle, isoxazolyle, oxadiazolyle, thiényle, quinolinyle, pyrazinyle et isothiazolyle, et qui est éventuellement substitué par un ou plusieurs groupes alkyle en C₁ à C₆, alcoxy en C₁ à C₆, halogéno ou amino ; et
R¹, R², R⁵, R⁶, R⁷, R⁸, R⁹, R¹⁰, R¹¹, G, J, a, b, c et d sont tels que définis dans la revendication 1 ;
ou un sel acceptable en pharmacie d'un tel composé.

3. Composé de formule générale III : 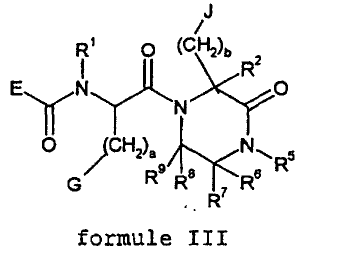 dans laquelle
E est 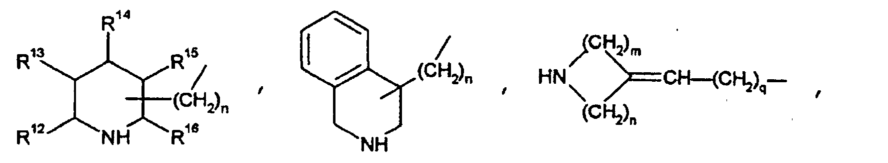 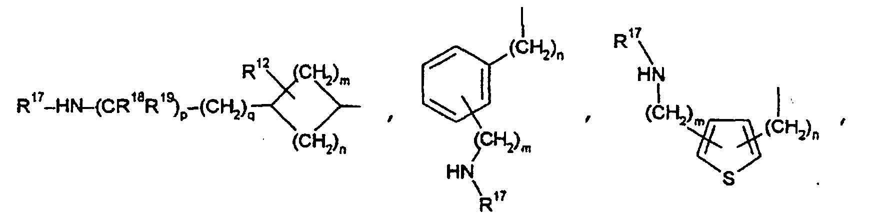 ou
R¹⁷-NH-(CR¹⁸R¹⁹)ₚ-(CH₂)ₘ-M-(CHR²⁰)ₒ-(CH₂)ₙ- ;
R¹², R¹³, R¹⁴, R¹⁵, R¹⁶, R¹⁷, R¹⁸, R¹⁹ et R²⁰ sont indépendamment choisis parmi :
l'hydrogène ; et
les radicaux alkyle en C₁ à C₆ éventuellement substitués par : un halogène ; ou un radical amino ; hydroxyle ; ou aryle carbocyclique ou hétérocyclique qui est choisi parmi phényle, naphtyle, pyridyle, 1-H-tétrazol-5-yle, thiazolyle, imidazolyle, indolyle, pyrimidinyle, thiadiazolyle, pyrazolyle, oxazolyle, isoxazolyle, oxadiazolyle, thiényle, quinolinyle, pyrazinyle et isothiazolyle, et qui est éventuellement substitué par un ou plusieurs groupes alkyle en C₁ à C₆, alcoxy en C₁ à C₆, halogéno ou amino ;
deux quelconques de R¹⁷, R¹⁸ et R¹⁹ peuvent indépendamment se réunir ensemble pour former un pont alkylène en C₁ à C₆ ;
n, m et q valent indépendamment 0, 1, 2 ou 3 ;
o et p valent indépendamment 0 ou 1 ;
M est -CH=CR²¹, -O-, -S- ou une liaison de valence ;
R²¹ est choisi parmi :
l'hydrogène ; et
les radicaux alkyle en C₁ à C₆ éventuellement substitués par : un halogène ; ou un radical amino ; hydroxyle ; ou aryle carbocyclique ou hétérocyclique qui est choisi parmi phényle, naphtyle, pyridyle, 1-H-tétrazol-5-yle, thiazolyle, imidazolyle, indolyle, pyrimidinyle, thiadiazolyle, pyrazolyle, oxazolyle, isoxazolyle, oxadiazolyle, thiényle, quinolinyle, pyrazinyle et isothiazolyle, et qui est éventuellement substitué par un ou plusieurs groupes alkyle en C₁ à C₆, alcoxy en C₁ à C₆, halogéno ou amino ;
R⁸ et R⁹ sont indépendamment choisis parmi :
l'hydrogène ;
les radicaux aryle carbocycliques ou hétérocycliques qui sont choisis parmi phényle, naphtyle, pyridyle, 1-H-tétrazol-5-yle, thiazolyle, imidazolyle, indolyle, pyrimidinyle, thiadiazolyle, pyrazolyle, oxazolyle, isoxazolyle, oxadiazolyle, thiényle, quinolinyle, pyrazinyle et isothiazolyle, et qui sont éventuellement substitués par un halogène ou un radical amino, cycloalkyle en C₃ à C₆, hydroxy, -COOR²² ou -CONR²³R²⁴ ; et
les radicaux alkyle en C₁ à C₆ éventuellement substitués par : un halogène ou un radical amino ; cycloalkyle en C₃ à C₆ ; hydroxy ; aryle carbocyclique ou hétérocyclique qui est choisi parmi phényle, naphtyle, pyridyle, 1-H-tétrazol-5-yle, thiazolyle, imidazolyle, indolyle, pyrimidinyle, thiadiazolyle, pyrazolyle, oxazolyle, isoxazolyle, oxadiazolyle, thiényle, quinolinyle, pyrazinyle et isothiazolyle, et qui est éventuellement substitué par un ou plusieurs groupes alkyle en C₁ à C₆, alcoxy en C₁ à C₆, halogéno ou amino ; -COOR²² ; ou -CONR²³R²⁴ ; et
G, J, R¹, R², R⁵, R⁶, R⁷, R²², R²³, R²⁴, a et b sont tels que définis dans la revendication 1 ;
ou un sel acceptable en pharmacie d'un tel composé.

4. Composé selon la revendication 2 ou 3, dans lequel R⁵ est l'hydrogène ou un radical alkyle en C₁ à C₆ éventuellement substitué par :
un radical aryle carbocyclique ou hétérocyclique qui est choisi parmi phényle, naphtyle, pyridyle, 1-H-tétrazol-5-yle, thiazolyle, imidazolyle, indolyle, pyrimidinyle, thiadiazolyle, pyrazolyle, oxazolyle, isoxazolyle, oxadiazolyle, thiényle, quinolinyle, pyrazinyle et isothiazolyle, et qui est éventuellement substitué par un ou plusieurs groupes alkyle en C₁ à C₆, alcoxy en C₁ à C₆, halogéno ou amino ; hydroxy ; cycloalkyle en C₃ à C₆ ; amino ; -COOR²⁵ ; -CONR²⁶R²⁷ ;
-NR'R" ; 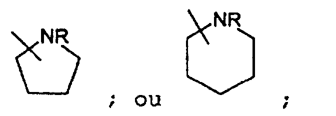 R, R' et R" sont indépendamment l'hydrogène ou un radical alkyle en C₁ à C₆ ;
R²⁵, R²⁶ et R²⁷ sont indépendamment l'hydrogène ou un radical alkyle en C₁ à C₆ ; et
R¹, R², R³, R⁴, R⁶, R⁷, R⁸, R⁹, R¹⁰, R¹¹, E, G, J, a, b, c et d sont tels que définis dans l'une quelconque des revendications précédentes ;
ou un sel acceptable en pharmacie d'un tel composé.

5. Composé selon l'une quelconque des revendications 2 à 4, dans lequel
E est 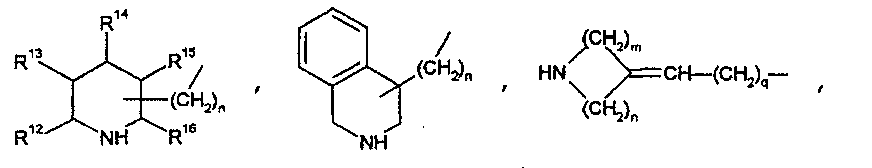 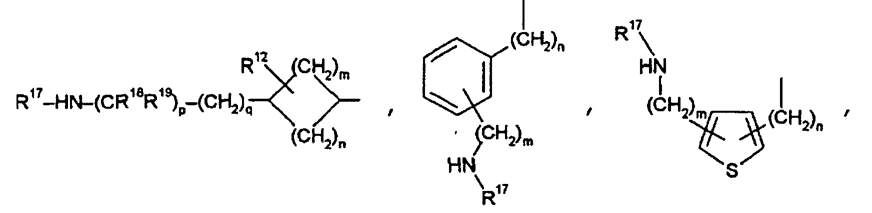 ou
R¹⁷-NH-(CR¹⁸R¹⁹)ₚ-(CH₂)ₘ-M-(CHR²⁰)ₒ-(CH₂)ₙ- ;
R¹², R¹³, R¹⁴, R¹⁵, R¹⁶, R¹⁷, R¹⁸, R¹⁹ et R²⁰ sont indépendamment l'hydrogène ou le radical méthyle ;
n, m et q valent indépendamment 0, 1, 2 ou 3 ;
o et p valent indépendamment 0 ou 1 ;
M est -CH=CR²¹-, -O- ou une liaison de valence ;
R²¹ est l'hydrogène ou le radical méthyle ou benzyle ; et
R¹, R², R³, R⁴, R⁵, R⁶, R⁷, R⁸, R⁹, R¹⁰, R¹¹, G, J, a, b, c et d sont tels que définis dans l'une quelconque des revendications précédentes ;
ou un sel acceptable en pharmacie d'un tel composé.

6. Composé selon la revendication 1, dans lequel R³ et R⁴ sont indépendamment choisis parmi
l'hydrogène ;
les radicaux aryle carbocycliques ou hétérocycliques qui sont choisis parmi phényle, naphtyle, pyridyle, 1-H-tétrazol-5-yle, thiazolyle, imidazolyle, indolyle, pyrimidinyle, thiadiazolyle, pyrazolyle, oxazolyle, isoxazolyle, oxadiazolyle, thiényle, quinolinyle, pyrazinyle et isothiazolyle, et qui sont éventuellement substitués par un halogène ou un radical amino, cycloalkyle en C₃ à C₆, hydroxy, -COOR²² ou -CONR²³R²⁴ ; et
les radicaux alkyle en C₁ à C₆ éventuellement substitués par : un halogène ou un radical amino ; cycloalkyle en C₃ à C₆ ; hydroxy ; aryle carbocyclique ou hétérocyclique qui est choisi parmi phényle, naphtyle, pyridyle, 1-H-tétrazol-5-yle, thiazolyle, imidazolyle, indolyle, pyrimidinyle, thiadiazolyle, pyrazolyle, oxazolyle, isoxazolyle, oxadiazolyle, thiényle, quinolinyle, pyrazinyle et isothiazolyle, et qui est éventuellement substitué par un ou plusieurs groupes alkyle en C₁ à C₆, alcoxy en C₁ à C₆, halogéno ou amino ; -COOR²² ; ou -CONR²³R²⁴ ;
R²², R²³ et R²⁴ sont indépendamment l'hydrogène ou un radical alkyle en C₁ à C₆ ;
E est 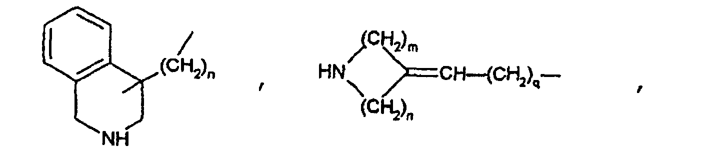 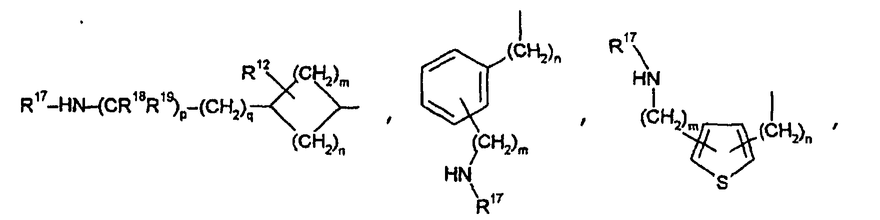 ou
R¹⁷-NH-(CR¹⁸R¹⁹)ₚ-(CH₂)ₘ-M-(CHR²⁰)ₒ-(CH₂)ₙ- ;
R¹², R¹⁷, R¹⁸, R¹⁹ et R²⁰ sont indépendamment choisis parmi :
l'hydrogène ; et
les radicaux alkyle en C₁ à C₆ éventuellement substitués par : un halogène ; ou un radical amino ; hydroxyle ; ou aryle carbocyclique ou hétérocyclique qui est choisi parmi phényle, naphtyle, pyridyle, 1-H-tétrazol-5-yle, thiazolyle, imidazolyle, indolyle, pyrimidinyle, thiadiazolyle, pyrazolyle, oxazolyle, isoxazolyle, oxadiazolyle, thiényle, quinolinyle, pyrazinyle et isothiazolyle, et qui est éventuellement substitué par un ou plusieurs groupes alkyle en C₁ à C₆, alcoxy en C₁ à C₆, halogéno ou amino ;
deux quelconques de R¹⁷, R¹⁸ et R¹⁹ peuvent indépendamment se réunir ensemble pour former un pont alkylène en C₁ à C₆ ;
n, m et q valent indépendamment 0, 1, 2 ou 3 ;
o et p valent indépendamment 0 ou 1 ;
Q est -CH=CR²¹-, -O- ou -S- ;
R²¹ est choisi parmi :
l'hydrogène ; et
les radicaux alkyle en C₁ à C₆ éventuellement substitués par : un halogène ; ou un radical amino ; hydroxyle ; ou aryle carbocyclique ou hétérocyclique qui est choisi parmi phényle, naphtyle, pyridyle, 1-H-tétrazol-5-yle, thiazolyle, imidazolyle, indolyle, pyrimidinyle, thiadiazolyle, pyrazolyle, oxazolyle, isoxazolyle, oxadiazolyle, thiényle, quinolinyle, pyrazinyle et isothiazolyle, et
qui est éventuellement substitué par un ou plusieurs groupes alkyle en C₁ à C₆, alcoxy en C₁ à C₆, halogéno ou amino ; et
R¹, R², R⁵, R⁶, R⁷, R⁸, R⁹, R¹⁰, R¹¹, G, J, a, b, c et d sont tels que définis dans la revendication 1 ;
ou un sel acceptable en pharmacie d'un tel composé.

7. Composé de formule générale IV : 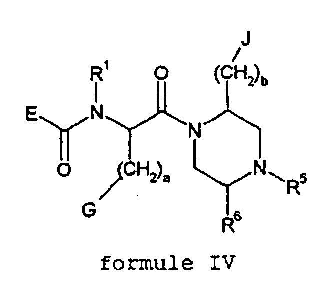 dans laquelle E est 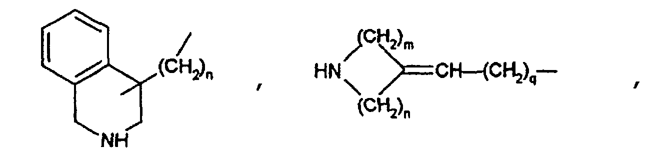 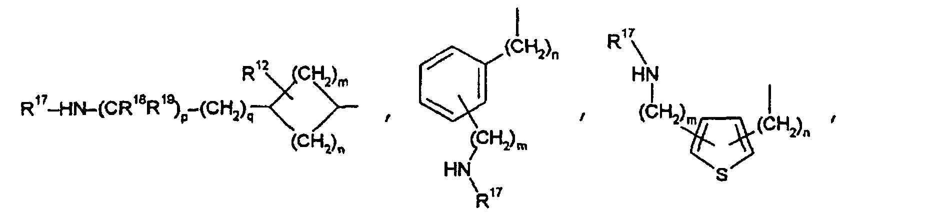 ou
R¹⁷-NH-(CR¹⁸R¹⁹)ₚ-(CH₂)ₘ-M-(CHR²⁰)ₒ-(CH₂)ₙ- ;
R¹², R¹⁷, R¹⁸, R¹⁹ et R²⁰ sont indépendamment choisis parmi :
l'hydrogène ; et
les radicaux alkyle en C₁ à C₆ éventuellement substitués par : un halogène ; ou un radical amino ; hydroxyle ; ou aryle carbocyclique ou hétérocyclique qui est choisi parmi phényle, naphtyle, pyridyle, 1-H-tétrazol-5-yle, thiazolyle, imidazolyle, indolyle, pyrimidinyle, thiadiazolyle, pyrazolyle, oxazolyle, isoxazolyle, oxadiazolyle, thiényle, quinolinyle, pyrazinyle et isothiazolyle, et qui est éventuellement substitué par un
ou plusieurs groupes alkyle en C₁ à C₆, alcoxy en C₁ à C₆, halogéno ou amino ;
deux quelconques de R¹⁷, R¹⁸ et R¹⁹ peuvent indépendamment se réunir ensemble pour former un pont alkylène en C₁ à C₆ ;
n, m et q valent indépendamment 0, 1, 2 ou 3 ;
o et p valent indépendamment 0 ou 1 ;
Q est -CH=CR²¹-, -O- ou -S- ;
R²¹ est choisi parmi :
l'hydrogène ; et
les radicaux alkyle en C₁ à C₆ éventuellement substitués par : un halogène ; ou un radical amino ; hydroxyle ; ou aryle carbocyclique ou hétérocyclique qui est choisi parmi phényle, naphtyle, pyridyle, 1-H-tétrazol-5-yle, thiazolyle, imidazolyle, indolyle, pyrimidinyle, thiadiazolyle, pyrazolyle, oxazolyle, isoxazolyle, oxadiazolyle, thiényle, quinolinyle, pyrazinyle et isothiazolyle, et qui est éventuellement substitué par un
ou plusieurs groupes alkyle en C₁ à C₆, alcoxy en C₁ à C₆, halogéno ou amino ; et
R¹, R⁵, R⁶, G, J, a et b sont tels que définis dans la revendication 1 ;
ou un sel acceptable en pharmacie d'un tel composé.

8. Composé de formule générale V : 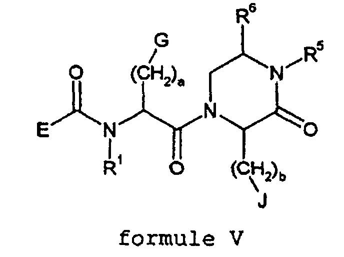 dans laquelle E est 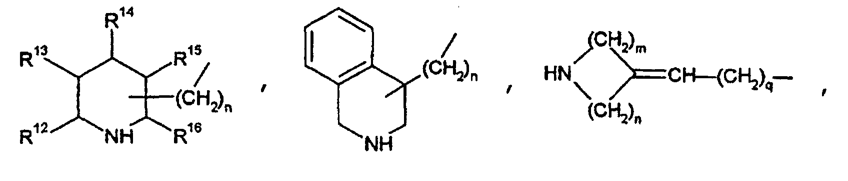 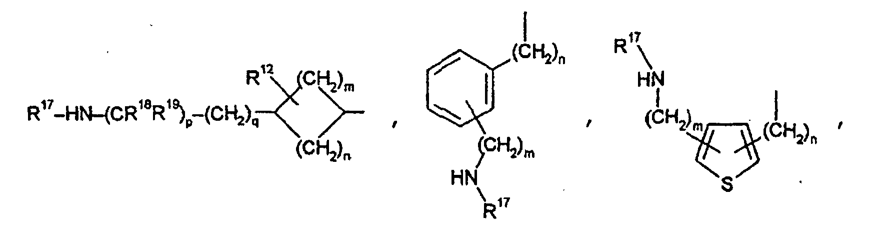 ou
R¹⁷-NH-(CR¹⁸R¹⁹)ₚ-(CH₂)ₘ-M-(CHR²⁰)ₒ-(CH₂)ₙ- ;
R¹², R¹³, R¹⁴, R¹⁵, R¹⁶, R¹⁷, R¹⁸, R¹⁹ et R²⁰ sont indépendamment choisis parmi :
l'hydrogène ; et
les radicaux alkyle en C₁ à C₆ éventuellement substitués par : un halogène ; ou un radical amino ; hydroxyle ; ou aryle carbocyclique ou hétérocyclique qui est choisi parmi phényle, naphtyle, pyridyle, 1-H-tétrazol-5-yle, thiazolyle, imidazolyle, indolyle, pyrimidinyle, thiadiazolyle, pyrazolyle, oxazolyle, isoxazolyle, oxadiazolyle, thiényle, quinolinyle, pyrazinyle et isothiazolyle, et qui est éventuellement substitué par un
ou plusieurs groupes alkyle en C₁ à C_{6,} alcoxy en C₁ à C₆, halogéno ou amino ;
deux quelconques de R¹⁷, R¹⁸ et R¹⁹ peuvent indépendamment se réunir ensemble pour former un pont alkylène en C₁ à C₆ ;
n, m et q valent indépendamment 0, 1, 2 ou 3 ;
o et p valent indépendamment 0 ou 1 ;
M est -CH=CR²¹, -O-, -S- ou une liaison de valence ;
R²¹ est choisi parmi :
l'hydrogène ; et
les radicaux alkyle en C₁ à C₆ éventuellement substitués par : un halogène ; ou un radical amino ; hydroxyle ; ou aryle carbocyclique ou hétérocyclique qui est choisi parmi phényle, naphtyle, pyridyle, 1-H-tétrazol-5-yle, thiazolyle, imidazolyle, indolyle, pyrimidinyle, thiadiazolyle, pyrazolyle, oxazolyle, isoxazolyle, oxadiazolyle, thiényle, quinolinyle, pyrazinyle et isothiazolyle, et qui est éventuellement substitué par un ou plusieurs groupes alkyle en C₁ à C₆, alcoxy en C₁ à C₆, halogéno ou amino ;
R⁵ est l'hydrogène ou un radical alkyle en C₁ à C₆ éventuellement substitué par :
un radical aryle carbocyclique ou hétérocyclique qui est choisi parmi phényle, naphtyle, pyridyle, 1-H-tétrazol-5-yle, thiazolyle, imidazolyle, indolyle, pyrimidinyle, thiadiazolyle, pyrazolyle, oxazolyle, isoxazolyle, oxadiazolyle, thiényle, quinolinyle, pyrazinyle et isothiazolyle, et qui est éventuellement substitué par un ou plusieurs groupes alkyle en C₁ à C₆, alcoxy en C₁ à C₆, halogéno ou amino ; hydroxy ; cycloalkyle en C₃ à C₆ ; amino ; -COOR²⁵ ; -CONR²⁶R²⁷ ;
-NR'R" ; 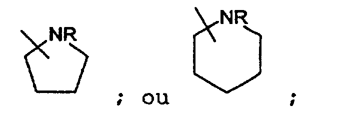 R, R' et R" sont indépendamment l'hydrogène ou un radical alkyle en C₁ à C₆ ;
R²⁵, R²⁶ et R²⁷ sont indépendamment l'hydrogène ou un radical alkyle en C₁ à C₆ ; et
R, J, a, b, R¹ et R⁶ sont tels que définis dans la revendication 1 ;
ou un sel acceptable en pharmacie d'un tel composé.

9. Composé selon l'une quelconque des revendications 1 à 8, dans lequel
R¹ est l'hydrogène ou le radical méthyle ;
ou un sel acceptable en pharmacie d'un tel composé.

10. Composé selon l'une quelconque des revendications précédentes, choisi dans l'ensemble comprenant les suivants :
N-((1R)-1-((2R)-2-benzyl-3-oxopipérazine-1-carbonyl)-2-(2-naphtyl)éthyl)-N-méthylamide d'acide (2E)-5-amino-5-méthylhex-2-énoïque,
N-((1R)-1-((2R)-2-benzyl-4-(cyclopropylméthyl)-3-oxopipérazine-1-carbonyl)-2-(2-naphtyl)éthyl)-N-méthylamide d'acide (2E)-5-amino-5-méthylhex-2-énoïque,
N-((1R)-1-((2R)-2-benzyl-5-(hydroxyméthyl)-3-oxopipérazine-1-carbonyl)-2-(2-naphtyl)éthyl)-N-méthylamide d'acide (2E)-5-amino-5-méthylhex-2-énoïque,
N-((1R)-1-((2R)-2-benzyl-3-oxo-1,4-diazépane-1-carbonyl)-2-(2-naphtyl)éthyl)-N-méthylamide d'acide (2E)-5-amino-5-méthylhex-2-énoïque,
N-méthyl-N-((1R)-2-(2-naphtyl)-1-((2R)-2-((2-naphtyl)méthyl)-3-oxopipérazine-1-carbonyl)éthyl)amide d'acide pipéridine-4-carboxylique,
N-((1R)-1-((2R)-2-benzyl-4-(diméthylcarbamoylméthyl)-3-oxopipérazine-1-carbonyl)-2-(2-naphtyl)éthyl)-N-méthylamide d'acide (2E)-5-amino-5-méthylhex-2-énoïque,
N-((1R)-1-((2R)-2-benzyl-3-oxopipérazine-1-carbonyl)-2-(2-naphtyl)éthyl)-N-méthylamide d'acide (2E)-5-méthyl-5-(méthylamino)hex-2-énoïque,
N-((1R)-2-((2R,6S)-2-benzyl-6-méthyl-3-oxopipérazine-1-carbonyl)-2-(2-naphtyl)éthyl)-N-méthylamide d'acide (2E)-5-amino-5-méthylhex-2-énoïque,
N-((1R)-1-((2R)-2-benzyl-3-oxopipérazine-1-carbonyl)-2-(biphényl-4-yl)éthyl)-N-méthylamide d'acide (2E)-5-amino-5-méthylhex-2-énoïque,
N- ((1R)-1-((2R) -2-benzyl-4-méthanesulfonylpipérazine-1-carbonyl)-2-(2-naphtyl)éthyl)-N-méthylamide d'acide (2E)-5-amino-5-méthylhex-2-énoïque,
N-((1R)-1-((2R)-2-benzyl-4-méthanesulfonylpipérazine-1-carbonyl)-2-benzyloxyéthyl)-N-méthylamide d'acide (2E)-5-amino-5-méthylhex-2-énoïque,
N-((1R)-1-(2R)-2-benzyl-3-oxopipérazine-1-carbonyl)-2-(2-naphtyl)éthyl)-N-méthylamide d'acide (2E)-5-amino-3,5-diméthylhex-2-énoïque,
N-((1R)-1-((2R,5R)-2-benzyl-5-méthyl-3-oxopipérazine-1-carbonyl)-2-(2-naphtyl)éthyl)-N-méthylamide d'acide (2E)-5-méthyl-5-méthylaminohex-2-énoïque,
N-((1R)-2-((2R)-2-benzyl-4-méthyl-3-oxopipérazin-1-yl)-1-((2-naphtyl)méthyl)-2-oxoéthyl)-N-méthylamide d'acide (2E)-5-amino-5-méthylhex-2-énoïque,
N-((1R)-1-((2R,5R)-2-benzyl-5-méthyl-3-oxopipérazine-1-carbonyl)-2-(2-naphtyl)éthyl)-N-méthylamide d'acide (2E)-5-méthyl-5-aminohex-2-énoïque,
N-méthyl-N-((1R)-2-(2-naphtyl)-1-((2R)-3-oxo-2-((2-thiényl)méthyl)pipérazine-1-carbonyl)éthyl)amide d'acide (2E)-4-(1-aminocyclobutyl)but-2-énoïque,
N-((1R)-2-((2R,5R)-2-benzyl-5-méthyl-3-oxopipérazin-1-yl)-1-((2-naphtyl)méthyl)-2-oxoéthyl)-N-méthylamide d'acide (2E)-4-(1-aminocyclobutyl)but-2-énoïque,
N-méthyl-N-((1R)-1-(2-naphtyl)méthyl-2-oxo-2-((2R)-3-oxo-2-((2-thiényl)méthyl)pipérazin-1-yl)éthyl)amide d'acide (2E)-5-méthyl-5-méthylaminohex-2-énoïque, et
N-((1R)-2-((2R)-2-benzyl-4-méthanesulfonylpipérazin-1-yl)-1-(napht-2-ylméthyl)-2-oxoéthyl)-N-méthylamide d'acide (2E)-5-amino-5-méthylhex-2-énoïque ;
et leurs sels acceptables en pharmacie.

11. Composition pharmaceutique comprenant, à titre d'ingrédient actif, un composé selon l'une quelconque des revendications 1 à 10 ou un sel acceptable en pharmacie d'un tel composé conjointement avec un véhicule ou diluant acceptable en pharmacie.

12. Composition selon la revendication 11 sous forme posologique unitaire, comprenant d'environ 10 à environ 200 mg d'un composé selon l'une quelconque des revendications 1 à 10 ou d'un sel acceptable en pharmacie d'un tel composé.

13. Composition pharmaceutique pour stimuler la libération de somatotrophine par l'hypophyse d'un humain ou d'un animal, la composition comprenant, à titre d'ingrédient actif, un composé selon l'une quelconque des revendications 1 à 10 ou un sel acceptable en pharmacie d'un tel composé conjointement avec un véhicule ou diluant acceptable en pharmacie.

14. Composition pharmaceutique destinée à être administrée à des animaux pour augmenter leur vitesse et leur ampleur de croissance, pour augmenter leur production de lait ou de laine, ou pour traiter des maladies, la composition comprenant, à titre d'ingrédient actif, un composé selon l'une quelconque des revendications 1 à 10 ou un sel acceptable en pharmacie d'un tel composé conjointement avec un véhicule ou diluant acceptable en pharmacie.

15. Composition pharmaceutique selon l'une quelconque des revendications 11 à 14, destinée à être administrée par voie orale, nasale, transdermique, pulmonaire ou parentérale.

16. Utilisation d'un composé selon l'une quelconque des revendications 1 à 10 ou d'un sel acceptable en pharmacie d'un tel composé pour la préparation d'un médicament.

17. Utilisation d'un composé selon l'une quelconque des revendications 1 à 10 ou d'un sel acceptable en pharmacie d'un tel composé pour la préparation d'un médicament destiné à stimuler la libération de gonadotrophine par l'hypophyse d'un humain ou d'un animal.

18. Utilisation d'un composé selon l'une quelconque des revendications 1 à 10 ou d'un sel acceptable en pharmacie d'un tel composé pour la préparation d'un médicament destiné à être administré à des animaux pour augmenter leur vitesse et leur ampleur de croissance, pour augmenter leur production de lait ou de laine, ou pour traiter des maladies.

19. Utilisation d'un composé selon l'une quelconque des revendications 1 à 10 ou d'un sel acceptable en pharmacie d'un tel composé pour la préparation d'un médicament destiné à : stimuler la libération de gonadotrophine chez les sujets âgés ; prévenir les effets secondaires cataboliques des glucocorticoïdes ; prévenir et traiter l'ostéoporose ; stimuler le système immunitaire ; accélérer la cicatrisation ; accélérer la réparation d'une fracture osseuse ; traiter un retard de croissance ; traiter une défaillance ou une insuffisance rénale résultant d'un retard de croissance ; traiter une insuffisance staturale physiologique, y compris traiter les enfants déficients en gonadotrophine et traiter une insuffisance staturale associée à une maladie chronique ; traiter l'obésité et un retard de croissance associé à l'obésité ; traiter un retard de croissance associé au syndrome de Prader-Labhart-Willi et au syndrome de Turner ; accélérer le rétablissement et réduire l'hospitalisation de patients brûlés ; traiter un retard de croissance intra-utérin, une dysplasie du squelette, un hypercorticisme et un syndrome de Cushing ; induire la libération pulsée de gonadotrophine ; remplacer l'hormone de croissance chez des patients stressés, traiter une ostéochondrose dysplasique, un syndrome de Noonan, une schizophrénie, des dépressions, la maladie d'Alzheimer, un retard de cicatrisation et une carence psychique sociale ; traiter un dysfonctionnement pulmonaire et une dépendance au respirateur ; atténuer les réponses cataboliques protéiques après une opération chirurgicale lourde ; réduire une cachexie et une perte protéique dues à une maladie chronique telle que le cancer ou le SIDA ; traiter l'hyperinsulinémie, y compris la nésidioblastose ; réaliser un traitement adjuvant pour l'induction de l'ovulation ; stimuler le développement thymique et prévenir le déclin associé à l'âge de la fonction thymique ; traiter des patients immunodéprimés ; améliorer la force musculaire et la mobilité ; maintenir l'épaisseur de la peau ; une homéostase métabolique ; une homéostase rénale chez les sujets âgés fragiles ; stimuler les ostéoblastes ; permettre un remodelage osseux et une croissance de cartilage ; stimuler le système immunitaire chez les animaux de compagnie et traiter les troubles du vieillissement chez les animaux de compagnie ; favoriser la croissance du bétail ; ou stimuler la pousse de laine chez les moutons.
